(19) Europäisches Patentamt

European Patent Office

Office européen des brevets

(11) EP 0 746 229 B1

(12) **EUROPEAN PATENT SPECIFICATION**

(45) Date of publication and mention
of the grant of the patent:
**10.09.2003 Bulletin 2003/37**

(51) Int Cl.⁷: $A61B\ 5/0468$, $A61B\ 5/0452$

(21) Application number: **95908695.0**

(86) International application number:
**PCT/US95/01072**

(22) Date of filing: **26.01.1995**

(87) International publication number:
**WO 95/020351 (03.08.1995 Gazette 1995/33)**

(54) **APPARATUS FOR MEASURING AND ASSESSING CARDIAC ELECTRICAL STABILITY**

GERÄT ZUR MESSUNG UND AUSWERTUNG DER ELEKTRISCHEN STABILITÄT DES HERZENS

APPAREIL POUR LE MESURAGE ET LA EVALUATION DE LA STABILITE ELECTRIQUE CARDIAQUE

(84) Designated Contracting States:
**DE FR GB IT**

(30) Priority: **26.01.1994 US 187275
14.11.1994 US 339050
14.11.1994 US 339032**

(43) Date of publication of application:
**11.12.1996 Bulletin 1996/50**

(73) Proprietor: **CAMBRIDGE HEART, INC.
Burlington, MA 01803 (US)**

(72) Inventors:
• **ARNOLD, Jeffrey
Wellesley, MA 02181 (US)**
• **ALBRECHT, Paul
Bedford, MA 01730 (US)**
• **LIBRETT, Kevin S.
Boston, MA 02114 (US)**
• **COHEN, Richard J.
Waban, MA 02168 (US)**

(74) Representative: **Deans, Michael John Percy et al
Lloyd Wise, Tregear & Co.,
Commonwealth House,
1-19 New Oxford Street
London WC1A 1LW (GB)**

(56) References cited:
**GB-A- 2 101 335        US-A- 4 732 157
US-A- 4 930 517        US-A- 5 148 812**

• **SMITH J.M. ET AL: 'ELECTRIC ALTERNANS AND CARDIAC ELECTRICAL INSTABILITY' CIRCULATION vol. 77, no. 1, 1988, pages 110 - 121**

EP 0 746 229 B1

**Description**

This invention relates to schemes for measuring and assessing cardiac electrical stability, e.g., by measurement of alternans, a temporal pattern of cycle-to-cycle variability in the morphology of the electrocardiographic (ECG), or by measurement of other physiologic waveforms.

Disturbances of electrical conduction processes in the heart are a major cause of morbidity and mortality. Sudden cardiac death, resulting from disturbances of electrical conduction in the heart, is responsible for approximately 400,000 fatalities per year in the United States alone. The mechanism responsible for the great majority of sudden cardiac deaths is ventricular fibrillation, a disorganized pattern of electrical activity in the ventricles of the heart, which leads to a disorganized pattern of mechanical contraction and results in the cessation of effective pumping action and death. Another disturbance of heart conduction processes, ventricular tachycardia, also reduces the effectiveness of the pumping action of the heart and thereby cause loss of consciousness (syncope) or death. Even in cases where ventricular tachycardia itself does not cause death, ventricular tachycardia can degenerate into ventricular fibrillation, which is lethal.

Effective means are now available to treat patients with electrical instabilities of the heart. For example, the internal programmed cardioverter/defibrillator is effective in preventing sudden cardiac death. This implanted device can terminate ventricular tachycardia and fibrillation by delivering an electric shock to the heart. Also available are antiarrhythmic drugs, which modify the electrical properties of the heart. These drugs may be used to render the heart electrically more stable. However, under certain circumstances, these drugs can also cause the heart to become more susceptible to ventricular tachycardia and fibrillation.

The first step in preventing sudden cardiac death is to identify individuals at risk. A common procedure for identification of risk involves invasive electrophysiologic testing. In this procedure, a catheter with one or more electrodes is advanced through the vasculature until the electrodes are properly positioned in a patient's heart. The electrodes are used to deliver electrical impulses to the heart in a deliberate attempt to initiate ventricular tachycardia. Patients in whom sustained ventricular tachycardia or ventricular fibrillation is induced are deemed to be at enhanced risk of sustaining spontaneous ventricular tachycardia or fibrillation. This invasive procedure is only suitable for stratifying risk in individuals already known to be at high risk. Typically, this procedure is used in individuals who have been successfully resuscitated from an episode of sudden cardiac death. But such invasive electrophysiologic testing would not be preferred for screening large populations of individuals for risk of serious ventricular arrhythmias.

A variety of non-invasive measures have been used to stratify risk, including measurement of the ejection fraction of the heart, measurement of the signal average electrocardiogram, measurement of heart rate variability, and measurement of ambient ventricular ectopic activity on a 24 hour electrocardiogram. These methods generally are not considered sufficiently predictive of risk to justify invasive testing or treatment of an asymptomatic individual. What is desired is a test that is non-invasive and can be reasonably performed in a doctor's office or clinic during routine evaluation of patients for heart disease. It is also desirable that such a test be highly sensitive (i.e., it identifies almost all of the high risk patients) and sufficiently specific (i.e., most positive tests are correct) to justify invasive testing of those patients who test positive.

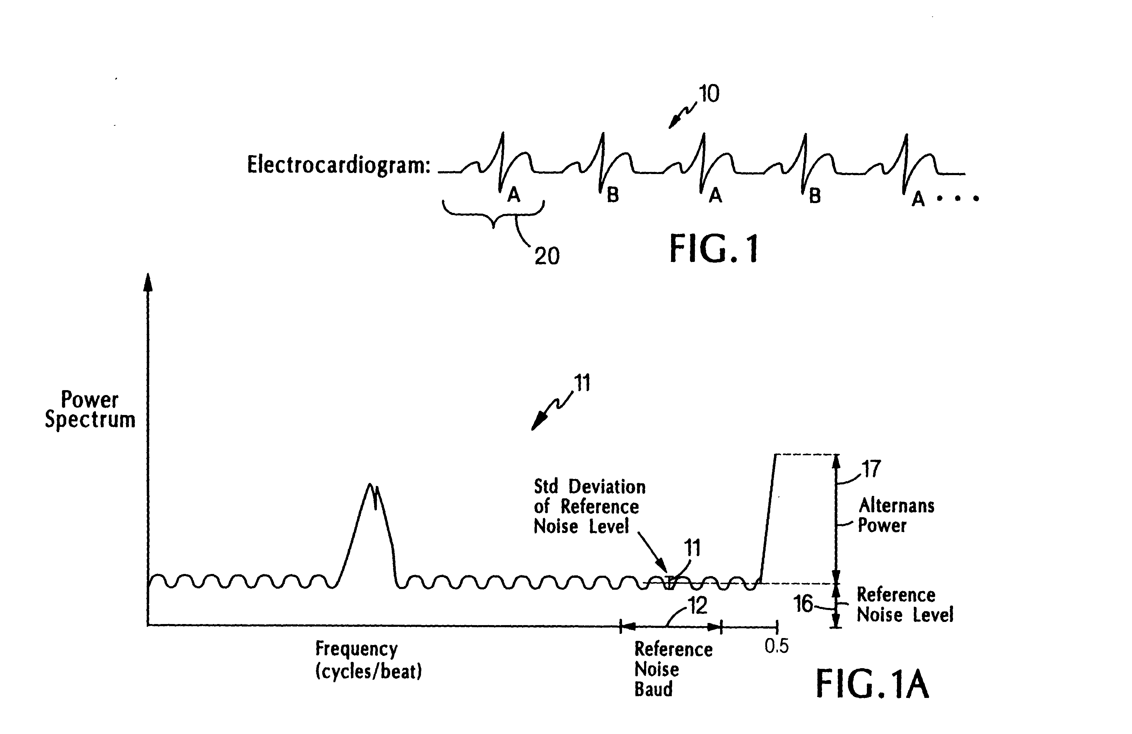

Recently, it has been determined that the measurement of minute levels of alternans is a powerful technique for assessing susceptibility to ventricular arrhythmias. Alternans is a pattern in which certain portions of the electrocardiographic or other physiologic waveforms, such as the ST segment and the T-wave of an electrocardiogram, alternate in shape in successive beats in an ABABAB... pattern (see Fig. 1). The alternans signal occurs at a fundamental frequency equal to half the heart rate and odd harmonics of the fundamental frequency. There have been, over the years, occasional reports of visible alternans in the electrocardiograms of humans. Alternans have also been associated with some rare disease states and with sudden death. Recently, it has been realized that the detection of microvolt levels of alternans during direct electrical pacing of the heart is generally indicative of cardiac electrical instability and predictive of an individual's susceptibility to ventricular arrhythmias.

A device according to the preambles of claims 1 to 3 is known from US-A- 4802491.

What is needed is a scheme to make analysis of alternans a practical clinical tool for the assessment of cardiac electrical stability that can be reasonably used in a doctor's office or clinic.

According to the present invention, an apparatus for measuring an alternans pattern of cycle-to-cycle morphology variations in a sequence of substantially repeating physiological waveforms in at least one signal measured from a patient, comprises:

a means for receiving a physiological signal representative of activity of the heart of a patient comprising one or more transducers adapted for respectively operatively measuring one or more signals from the patient;

a means for digitally processing the physiological signal to determine a level of alternans in the signal, the physiological signal representative of the activity of the heart of the patient is representative of the activity of the heart of the patient with an altered physiological condition as a result of stress to the heart; and

a stressing device.

The stressing device comprises either an exerciser for controllably exercising the patient, or a device adapted to administer a pharmacological agent that controllably alters the heart rate of the patient, or a tilt table adapted to securely hold the patient and to enable selective alteration of the orientation of the patient's body to stress the patient's heart.

Embodiments according to the invention may include one or more of the following features. Preferably, the physiologic condition of the patient is altered so that the patient's heart rate is above a predetermined rate. The physiologic condition of the patient is preferably altered so that the patient's heart rate is between about 90 and about 150 beats per minute. The physiologic condition of the patient is preferably altered by exercising the patient (e.g. by pedalling a bicycle, walking on a treadmill, or stair stepping). Preferably, a signal is produced for controlling the patient's exercise intensity. Preferably, the signal is produced to achieve a desired heart rate in the patient. Signals are preferably received representative of the patient's rate of exercise.

In certain preferred embodiments, a signal is produced for controlling the patient's rate of exercise.

The patient's rate of exercise is preferably selected to be between about twenty-eight percent of the patient's heart rate and about forty-three percent of the patient's heart rate or between about fifty-seven percent of the patient's heart rate and about seventy-two percent of the patient's heart rate. The controlling signal is preferably produced to reduce noise that interfere with determining the level of alternans. The controlling signal is preferably produced to reduce noise having a substantially repeating component that repeats at a frequency of about one half the patient's heart rate or at a submultiple thereof. The controlling signal is preferably produced to reduce noise artifacts generated by one or more sources other than the patient's heart. The controlling signal is preferably produced to reduce noise artifacts generated as a result of exercise of the patient. The controlling signal is preferably produced to reduce noise artifacts generated as a result of respiration of the patient. The controlling signal is preferably produced based on a predetermined exercise protocol specifying target exercise rates.

In certain preferred embodiments, the signal is processed to reduce noise that interferes with determining the level of alternans. The determination of a level of alternans is preferably based on portions of the received signal corresponding to periods when the level of exercise falls within a predetermined range. The determination of a level of alternans is preferably based on portions of the received signal corresponding to periods when the patient's respiratory rate is different from the patient's heart rate and is different from sub-multiples of the patient's heart rate. Preferably, portions of the received signal are selected for the determination of alternans based on the presence of abnormal beats. The portions selected for the determination of alternans is based on a comparison of a weighted predetermined level.

Preferably, a signal representative of the determined level of alternans and a signal representative of the determined heart rate are simultaneously produced.

In certain preferred embodiments, interfering variability in the morphology of the substantially repeating waveforms is compensated for in the received signal.
The interfering variability that is compensated may be intercycle interval variability. The interfering variability that is compensated may be variability generated by the patient's respiratory activity.

In certain preferred embodiments the physiologic condition of the patient is altered by administering to the patient a pharmacological agent that stresses the heart of the patient (e.g., a beta-sympathetic agent, a parasympathetic blocking agent, and a vasodilator).

Also, the physiologic condition of the patient may be altered by applying negative body pressure to the lower body of the patient or by selecting the orientation of the patient's body to be different from a supine position. In certain embodiments, the physiologic condition of the patient is altered spontaneously.

In certain embodiments the measurements of alternans is conducted at the same time as other tests (e.g., when determining whether the patient has coronary artery disease by determining physiologic indices of ischemia in the patient).

The processing step may comprise processing physiologic signals from periods of time before the physiologic condition of the patient is altered.

Physiologic signals may be processed from periods of time after the physiologic condition of the patient is altered.

In certain embodiments, the apparatus may be used to obtain a second signal from the patient; determine a relationship between variability in the first and second received signals; and use the relationship in the determination of a level of alternans.

The invention advantageously enhances the measurement of the pattern of cycle-to-cycle morphology variations by reducing the effect of variability in the interval between adjacent waveforms in said at least one representative signal.

Preferably, the invention provides compensation for interfering variability in the morphology of the substantially repeating waveforms in the received signal.

Preferably, the one or more signals are additionally processed to produce one or more of the following additional diagnostic tests: a multi-lead electrocardiogram, a signal averaged electrocardiogram, a test for the presence of coronary artery disease, an analysis of QRS complex variability, or physiologic system identification.

Preferably, the apparatus includes a stressing device adapted to interact with the patient for altering the physiologic condition of the patient to controllably stress the heart of the patient to enhance the measurement of alternans.

Advantageously, the apparatus includes a recorder coupled to said one or more transducers and adapted to store signals measured by said one or more transducers for a period in excess of one hour; and the processor is adapted to characterise portions of said measured signals corresponding to one or more periods according to one or more pre-selected criteria, and further adapted to obtain, from said one or more stored signals corresponding to said one or more selected periods, a measure of the level of alternans in said at least one representative signal for assessing the condition of the patient's heart.

The processor is advantageously capable of processing the signals to produce one or more of the following additional diagnostic tests: a multi-lead electrocardiogram, a signal averaged electrocardiogram, a test for the presence of coronary artery disease, an analysis of QRS variability, or physiologic system identification.

Schemes embodying combination of the above features are also contemplated. Such combinations offer complementary improvements for the measurement of alternans.

The present invention will be described, by way of example, with reference to the accompanying drawings, in which:

Fig. 1 is a diagrammatic plot of a typical ECG.

Fig. 1A is a diagrammatic power spectrum of the ECG of Fig. 1.

Fig. 2 is a plot of the approximate number of patients at risk for cardiac instability in whom alternans is observed plotted as a function of heart rate.

Fig. 3 is a schematic block diagram of a patient, an apparatus for altering the physical condition of the patient and an apparatus for measuring physiologic signals from the patient.

Fig. 4 is a plot of the frequency locations of alternans, reference noise bands, and pedalling motion artifacts.

Fig. 5 is a plot of a power spectrum of data obtained during atrial pacing of the heart.

Fig. 6 is a plot of a power spectrum of data obtained during uncontrolled exercised.

Fig. 7 is plot of a power spectrum of data obtained during bicycle exercise controlled according to the invention.

Figs. 8 and 8A are respective power spectra plots corresponding to a "perfect" alternans pattern and a pattern in which it is assumed that there is a 5% probability of phase-resetting at each beat.

Fig. 9 is a flow diagram of a process for reducing the effect of abnormal beats on an alternans measurement.

Fig. 10 is a plot typical of the relationship between the amplitude, $V_a$, on one point on a waveform at a specified offset from a fiducial point and the $\Delta RR$ interval, the deviation from the preceding interbeat interval and the mean beat interval.

Fig. 11 is a plot of a power spectrum of data in which there is RR interval variation.

Fig. 12 is a plot of a power spectrum of the same data as Fig. 11 using the method of the invention to compensate for RR interval variation.

Fig. 13 is a plot typical of the relationship between $V_a(i,j)$ and S, the contemporaneous value of the respiration signal.

Figs. 14 and 14A are plots of power spectra in which a harmonic of respiration overlaps with the alternans frequency, before and after correction for respiration artifact, respectively.

Fig. 15 is a flow diagram of a measurement method.

Figs. 16-16D are plots of physiologic signals over time.

Figs. 17-17C are plots of physiologic signals over time.

Fig. 18 is a flow diagram of the steps of a measurement method.

Figs. 19 and 19A are diagrammatic views of components of a multi-segment electrode.

Fig. 20 is a schematic diagram of a circuit for measuring noise created by electrode movement.

Fig. 21 is a schematic diagram of a circuit for recording ECG, respiration, and electrode impedance.

Fig. 22 shows the placement location of electrodes on the body of a patient. Fig. 22A defines the location of the electrodes, shown in Fig. 7, their type, and defines the input signals that are recorded from these electrodes.

Fig. 23 is a diagrammatic view illustrating steps for preprocessing input signals.

Fig. 24 is a table defining signal elements of a b(n) matrix.

Fig. 25 is an ECG potential over a single beat.

Fig. 26 is a matrix containing 32 input signals for an nth beat.

Fig. 27 is a table defining coefficients for combining the input signals to create the output signals.

Fig. 28-28B are plots of measured output signals.

Fig. 29 is a schematic diagram of a heart represented as a dipole source.

Fig. 29A is a schematic diagram of clinical ECG measurements represented as vector projections of a dipole source.

Fig. 30 is a plot of a power spectrum corresponding to the same data as in Fig. 7, in which signals from at least two electrodes were used to reduce noise in the data according to the invention.

Fig. 31 is a flow diagram of steps for the real-time computation of alternans.

Fig 32 is a plot of the probability that the alternans energy is greater than a reference revise level by $2_\mu$V and that K is greater than 3 as a function of RMS noise per frequency bias.

Fig. 33 is a plot of the probability that the alternans energy is greater than $2_\mu$V RMS as a function of RMS noise per frequency bias.

Fig. 34 is a flow diagram of steps for selecting epochs from a measured data signal.

Figs. 35-35D are respective plots over time of the level of alternans, a flag for ectopic beats, the heart rate the level of overall noise, and the level of noise and respiration occurring at 1/4 and 1/6 of the heart rate.

Fig. 36 is the block diagram of a combined exercise stress test and alternans measurement system.

Fig. 37 is a flow diagram of a method for selecting one or more epochs from a measured signal.

Fig. 38 is the block diagram for the Calculate Alternans process of the system in Figure 19.

Fig. 39 is a block diagram of a combined ECG cart and an alternans system.

Fig. 40 is the block diagram for a combined ambulatory (i.e., long term or Holter) ECG analysis and alternans system.

Fig. 41 is a table of results from a clinical study testing the invention's ability to identify ventricular vulnerability.

The following description describes an example of an apparatus according to the present invention, and examples of methods of use of the apparatus

Referring to Figs. 1 and 1A, a typical ECG signal 10 has a power spectrum 11 characterized by a reference band 12 that has a standard deviation 14 and a reference noise level 16. The alternans level 18 is measured from the energy level at the alternans frequency of one half the heart rate. As described in U.S. Patent No. 4,802,491, the power spectrum 11 is determined by sampling a sequence of substantially repeating electrocardiographic waveforms 20, in this case T waves, and processing this sampled data. The alternans is estimated from the amplitude of the power spectrum at the alternans frequency (0.5 cycles per beat), as well as the mean and standard deviation of a reference noise band. The amplitude of the power spectrum at the alternans frequency is compared with the mean and standard deviation of the reference noise band to determine if alternans is present at a statistically significant level.

An alternans pattern of variability in an electrocardiogram with an amplitude even in the microvolt range is clinically significant (in contrast, an ECG typically has a peak amplitude in the millivolt range). An alternans pattern of variability is usually too small to be detected by visual inspection of an electrocardiogram. Alternans is also difficult to measure because it must be detected and quantified in the presence of other temporal patterns of beat-to-beat variability in the electrocardiographic waveform, which are usually larger in magnitude than the level of alternans. For example, skeletal muscle activity, electrode and cable motion, ambient electromagnetic activity in the room, and electrocardiograph amplifiers all introduce noise which cause subtle beat-to-beat variability in the spaces of electrocardiographic waveforms.

I. Measurement of Alternans in Association with Application of Physiologic Stress

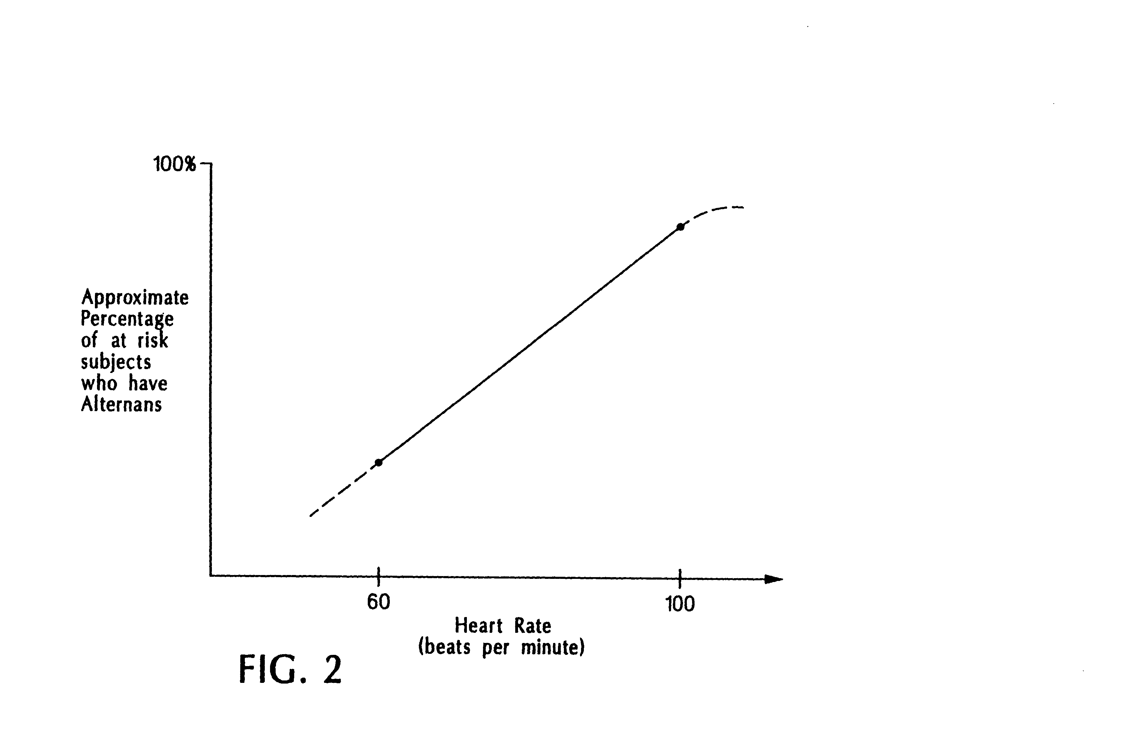

Data we have collected in patients susceptible to ventricular arrhythmias indicate that the incidence of alternans increases with heart rate. As illustrated in Fig. 2, as the heart rate increases from sixty to approximately ninety-five beats per minutes, the incidence of alternans increases in susceptible patients from under forty percent to approximately eighty percent. Based on this data, we believe that a sensitive clinical test to measure susceptibility to ventricular arrhythmias using currently know techniques requires that the heart rate be elevated to around this level.

As shown in Fig. 2, physiologic waveform variability may depend greatly on the repetition rate of the waveform. In the case of an electrocardiographic waveform, the magnitude of alternans in individual animals increases with heart rate when the heart is paced by electrodes placed within the heart. However, recent data we have collected from a population of subjects susceptible to ventricular arrhythmias indicates that the incidence of alternans also increases with heart rate. That is, at normal heart rates many or most patients who are susceptible to ventricular tachycardia do not appear to demonstrate alternans in their electrocardiogram even though they will when electrically paced at higher heart rates. By about 100 beats per minute (BPM) almost all patients susceptible to ventricular arrhythmias will demonstrate alternans. We believe that a sensitive clinical test to measure susceptibility to ventricular arrhythmias using currently known techniques requires that the heart rate be elevated to around this level.

We have realized that the measurement of alternans can be enhanced by altering the physiologic condition of the patient to controllably stress the patient's heart (i.e., by application of one or more selected physiologic stress techniques, e.g., as described below). Data we have collected indicates that application of a physiologic stress may increase the incidence and level of alternans in a population of subjects at risk for ventricular arrhythmias in a manner which is independent of the effect of the physiologic stress on the heart rate. In the clinical study presented below, we found that alternans measured during an exercise protocol designed to raise the heart rate to 100 beats per minute was a more accurate predictor of a patient's susceptibility to ventricular tachycardia or ventricular fibrillation than alternans measured during atrial pacing at 100 beats per minute. This indicates that the physiologic stress of exercise may have an effect independent of the effect of exercise on heart rate on inducing and/or raising the level of alternans. In addition, fewer than fifty percent of patients who had measurable levels of alternans during exercise were found to have measurable levels of alternans at rest. Thus, measurement of alternans in the presence of a physiologic stress appears to make the alternans measurement a more sensitive test for cardiac electrical instability independent of the change in heart rate resulting from the physiologic stress.

Altering the physiologic condition of the patient (e.g., by exercise, by administration of a suitable pharmacologic agent, by applying negative body pressure, or by changing the orientation of the patient's body) is easily achieved in a doctor's office or clinic. Also, using such techniques to control the application of physiologic stress enables a patient's heart rate, or elevation in heart rate, to be used to assess the general level of the physiological stress.

Altering the Physical Condition of a Patient

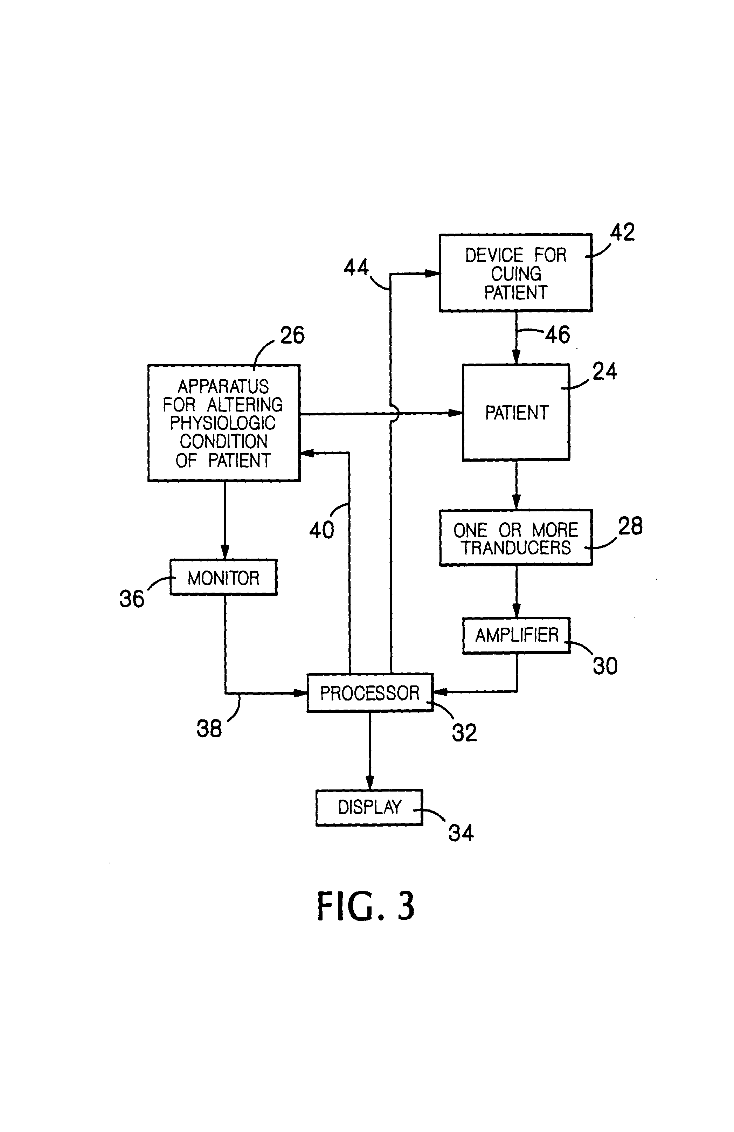

Referring to Fig. 3, a generalized scheme for measuring alternans during alteration of the physical condition of a patient 24 includes an apparatus 26 for altering the physical condition of the patient (e.g., as described below). One or more transducers 28 (e.g., ECG electrodes) are positioned on the surface of the patient to measure signals from the patient, at least one of which being representative of one or more signals from the patient's heart. The measured signals are amplified by an amplifier 30 and are subsequently received by a processor 32. The processor is adapted (e.g., in hardware or software) to obtain a measure of the level of alternans in at least one of the measured signals. This measure is displayed on a display 34 (e.g., a TV monitor or a chart recorder) for viewing by an operator.

A monitor 36 is included to provide to the processor signals 38 representative of the status of apparatus 26 (e.g., the pedal rate if the apparatus is a bicycle). The processor, based on status signals 38, adaptively controls the alteration of the physical condition of the patient by passing control signals to apparatus 26 over feedback loop 40. The processor may also selectively pass signals to a device 42 for cuing the patient over control path 44. Cuing device 42 delivers signals 46 to the patient specifying instructions for voluntary alterations of the patient's physical condition.

A. Exercise

In one example, physiologic stress is non-invasively applied by having the subject exercise, for example, by pedaling on an upright bicycle, pedaling on a supine bicycle, walking on a treadmill, or stepping on stairs. Such method might include a means of measuring heart rate and using such measurement to adjust the level of exercise so that the heart rate is maintained within a desired range. In preferred embodiments of the invention, the patient exercises at a rate controlled so that his heart rate is greater than about 90 BPM, preferably greater than about 100 BPM, and more preferably between about 100 and about 150 BPM.

In one preferred embodiment of the invention, the apparatus of this invention is combined with the apparatus used to conduct physiologic stress tests for other purposes, for example exercise stress tests used to detect the presence of coronary artery disease. Apparatus for the latter usually includes a treadmill or bicycle or other equipment for performing controlled exercise and equipment for recording electrocardiographic signals. This combination of apparatus is desirable furthermore because for many patients in whom one would want to assess physiologic waveform variability, one would also wish to perform an exercise stress test to detect the presence of coronary artery disease.

The level of exercise may be altered to adjust the subject's heart rate in a variety of ways. For example, when a subject is exercising on a bicycle the resistance of the bicycle to pedaling may be altered and/or the subject may be instructed to alter the pedaling speed. For example, when a treadmill is being used the speed of the treadmill (or equivalently the patient's step rate) may be altered, or the incline of the treadmill may be altered. For example, when the physiologic stress is stair stepping the level of exercise may be altered by adjusting the step rate and/or the incline of the treadmill.

B. Pharmacologic Agent

In another example, physiologic stress is non-invasively applied by administration of pharmacological agents known to simulate physiologic stress. Examples of drugs that are used include: parasympathetic blocking agents, such as atropine and glycopyrrulate; a beta-sympathetic agonist, such as isoproterenol, dopamine, and dobutamine; and a vasodilator, such as nitroprusside. We have found that beta-sympathetic agonists may be desired agents because beta-sympathetic stimulation enhances alternans independent of their effect on heart rate. In each case, the method of using the apparatus of the invention may involve measuring heart rate and adjusting the dosage of the drug to maintain heart rate in the desired range. For example, this may be accomplished by intravenously infusing a solution of the drug and then progressively increasing the infusion rate until the heart rate achieves a desired level, e.g., greater than 90 BPM, preferably greater than 100 BPM, and more preferably between 100 and 150 BPM. Alternatively, the data could be collected during a standard pharmacological stress test conducted for other diagnostic purposes, e.g., a stress test used to detect coronary artery disease. Preferably, the only data segments analyzed are those in which the target heart achieves a desired level.

Example: Isoproterenol Protocol

A presently preferred example for measuring alternans in a patient by administering isoproterenol has the following protocol. Assure that standard ECG electrodes and X, Y, Z electrodes are properly positioned on the patient. In the event of adverse reaction to isoproterenol, have on hand the beta-sympathetic drug propranolol hydrochloride for intravenous administration in 1 mg doses to reverse the effects of isoproterenol on the cardiovascular and other systems. Via a peripheral intravenous line, administer isoproterenol at a rate of 0.5 micrograms per minute and gradually increase the infusion rate until the heart rate exceeds 100 beat per minute (range 100-110 beats per minute). Then adjust infusion rate to maintain heart rate in desired range. Monitor patient for evidence of arrhythmias on the electrocardiogram and/or the development of ischemia manifested by chest pain or significant ST segment shifts on the electrocardiogram. Continue recording ECG with Alt-ECG device until a sufficient amount of data has been collected, e.g., as determined by one or more of the techniques described below.

Example: Atropine Protocol

A presently preferred example for measuring alternans in a patient by administering atropine has the following protocol. Place standard ECG electrodes and X, Y, Z electrodes on the patient. In the event of adverse reaction to atropine, have on hand physostigmine salicylate for intravenous administration in a dose of 0.5 to 1.0 mg to reverse the central and peripheral anticholinergic effects of atropine. Also have on hand physostigmine eye drops to reverse any ocular effects of the atropine. Via a peripheral intravenous line, administer an initial does of atropine 0.8 mg IV. Increase the dosage by 0.2 mg to 0.4 mg increments until the heart rate exceeds 100 bpm (range 100-110 bpm), or until a total cumulative does of 2.0 mg of atropine has been administered. Continue recording ECG until a sufficient amount of data has been collected, e.g., as determined by one or more of the techniques described below.

C. Negative Body Pressure

In certain situations, physiologic stress is non-invasively applied by means of applying lower body negative pressure (here negative pressure is taken to mean negative with respect to ambient atmospheric pressure). In one presently preferred example, the lower extremities are placed in a negative pressure chamber, the heart rate is measured by a heart rate monitor, and the level of negative pressure is adjusted to maintain heart rate in the desired range.

D. Change of Body Orientation

In certain other situations, physiologic stress is non-invasively applied by means of change of body position, for example, by recording data while the patient is tilted on a tilt table or by recording data when the patient assumes a sitting or standing position. Physiologic signal recording in patients is most commonly obtained with the patient in the supine position. Substantial physiologic stress can be achieved by changing the orientation of the patient's body in the earth's gravitational field. This is preferably achieved by placing the patient on a tilt table and tilting the patient. Significant stress can also be achieved by having the patient assume a standing position, or even the sitting position - particularly in hemodynamically compromised patients. In this preferred embodiment, the patient's body position is changed to induce physiologic stress, physiologic signals are recorded while the patient is subjected to the stress, and the physiologic signals are analyzed for the presence of alternans.

II. Reducing Interfering Noise Signals:

A. Exercise Artifacts

One problem associated with the measurement of alternans associated with the application of a physiologic stress is that the physiologic stress itself can induce noise that interferes with the alternans measurement. For example, exercise may induce a rhythmic artifact at the alternans frequency (half of the heart rate). It is therefore desirable to choose modes of physiologic stress that induce as little rhythmic artifact as is practical. For example, one would wish to limit upper body movement to reduce motion artifact induced in the chest leads. Thus, bicycle exercise would be expected to be preferred over arm motion exercise.

In a preferred example, the parameters of the physiologic stress are adjusted to minimize the interference with the alternans measurement. In certain preferred embodiments, where exercise is used as the physiologic stress, exercise parameters are adjusted in such a way to minimize the effect of rhythmic noise which interferes with the alternans measurement.

Example: Bicycle Exercise

During bicycle exercise, we have realized that the recorded electrocardiograms contains motion artifact at frequencies corresponding to the pedal rate and its harmonics. The pedal rate is set so that frequency content of the pedaling signal and the frequency content of the alternans signal are separated as much as is practical, consistent with achieving a target heart rate and comfortable pedal rates. The separation in the frequency domain between the pedaling signal's fundamental and second harmonic from the alternans signal's fundamental and third harmonic is improved by setting the pedal rate (the pedal rate and its second harmonic both lie between half the heart rate and three halves of the heart rate) at one-third or two-thirds of the heart rate. This is obtained by solving the equation:

$$PR - HR/2 = 3 \cdot HR/2 - 2 \cdot PR$$

where HR is the heart rate and PR is the pedal rate.

A reference frequency noise band is used to estimate the noise in the alternans measurement. In one presently preferred example, this noise band is calculated, as described in Smith et al ("Electrical alternans and cardiac electrical instability" by J.M Smith et al, Circulation, 77, 1988, 110-121). According to the technique described in Smith et al, the noise band corresponds to the frequency band of 0.43HR to 0.46HR in the power spectrum. To minimise the overlap between the frequency content of the pedalling signal and the reference noise band, after taking into account the aliasing about the Nyquist frequency of HR/2, this noise band corresponds to the unaliased frequency bands of:

$$0.43HR + nHR \text{ to } 0.46HR + nHR,$$

and

$$0.54HR + nHR \text{ to } 0.57HR + nHR$$

where n is the set of non-negative integers. So if PR and 2PR lie between the upper edge of the noise band at 0.57HR and the lower edge of the noise band located at 1.43HR, then

$$0.57HR < PR < 0.715HR$$

with PR optimally being set at 0.642HR. If PR lies between 0.57HR and 1.43HR but 2PR lies between the band edges of 1.57HR and 2.43HR this implies

$$0.785HR < PR < 1.215HR$$

with PR optimally being equal to the HR. If PR is less than the band edge at 0.43HR and 2PR lies above the band edge at 0.57HR this implies that

$$0.285HR < PR < 0.43HR$$

with an optimal frequency of 0.3575HR.

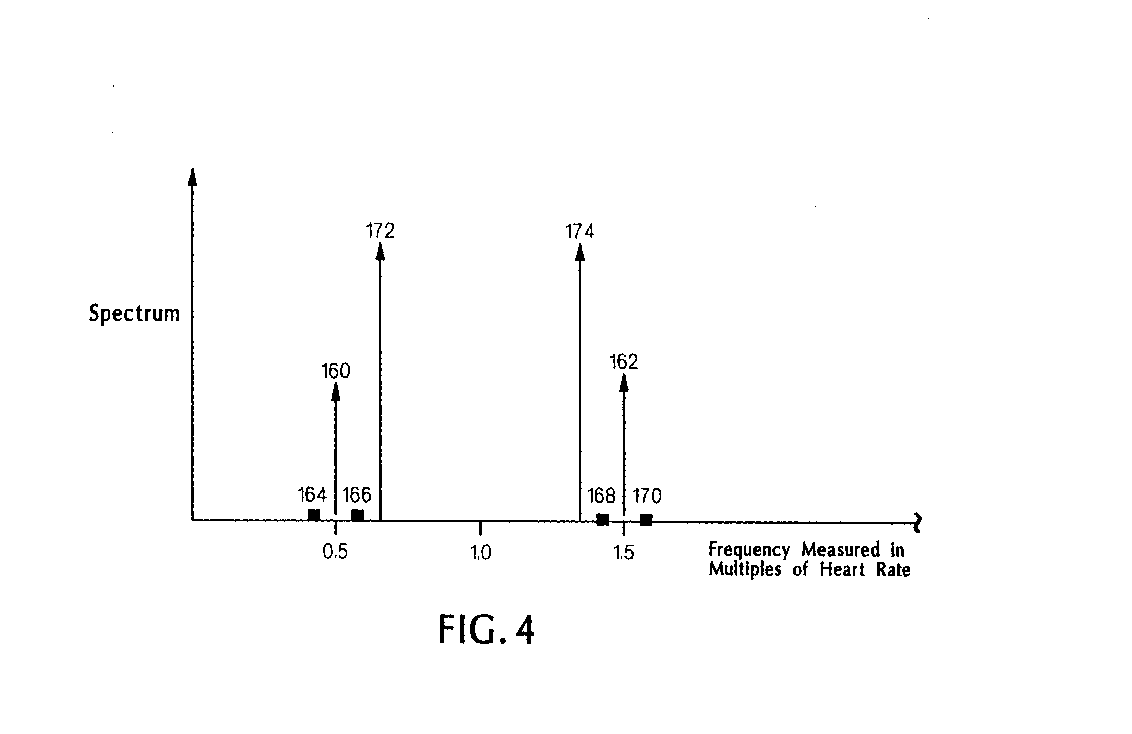

Referring to Fig. 4, the frequency content of the alternans signal fundamental 160, the third harmonic of the alternans signal 162, the reference noise bands 164, 166, 168, 170, the first harmonic of a sample pedal rate 172 and the second harmonic of this sample pedal rate 174. Using such considerations, protocols may be established defining target pedaling rates for target heart rates:

| Target Heart Rate (beats per minute) | Target Pedal Rate (revolutions per minute) |
|---|---|
| 105 - 115 | 65 - 75 |
| 100 - 110 | 63 - 72 |
| 105 - 115 | 33 - 45 |
| 100 - 110 | 31 - 43 |

The subject is instructed to maintain the pedal rate in the desired range (the apparatus may measure the actual pedaling rate and display to the subject the actual pedaling rate and the target), and the resistance to pedaling is adjusted automatically by the apparatus or manually by the operator to maintain the heart rate within the desired range. The protocol in which the target pedaling rate is controlled near one-third of the heart rate is found to be particularly convenient and comfortable for many patients. In certain situations, the operator may choose a different protocol, depending on the ability of the patient.

In one preferred example the pedal rate is controlled by the use of a metronome to cue the patient to pedal at the appropriate rate. For example, the patient wears ear phones that provide alternating audio tones cuing the patient to pedal alternately with the right and left foot. Alternatively, the patient is cued to pedal with the right and left foot by using alternating visual prompts.

In certain preferred embodiments, the apparatus has a tachometer to measure pedal rate as well as heart rate and measured data that does not fall within the specified limits of heart rate and pedal rate are ignored. Means of measuring pedal rate includes an electronic interface with a revolution rate meter on the bicycle, an accelerometer on the patient's leg, and means to analyze the electrocardiogram signal noise induced by pedaling (for example, by analyzing the variability in the PQ segment of the ECG which normally reflects no cardiac electrical activity).

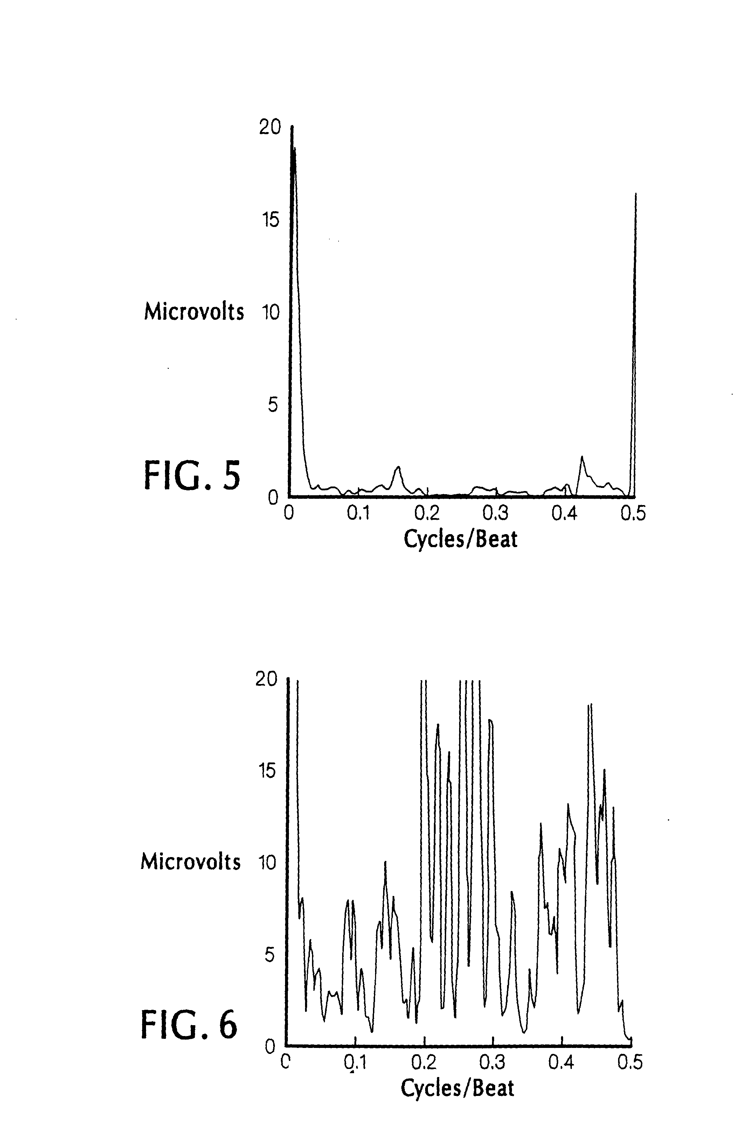

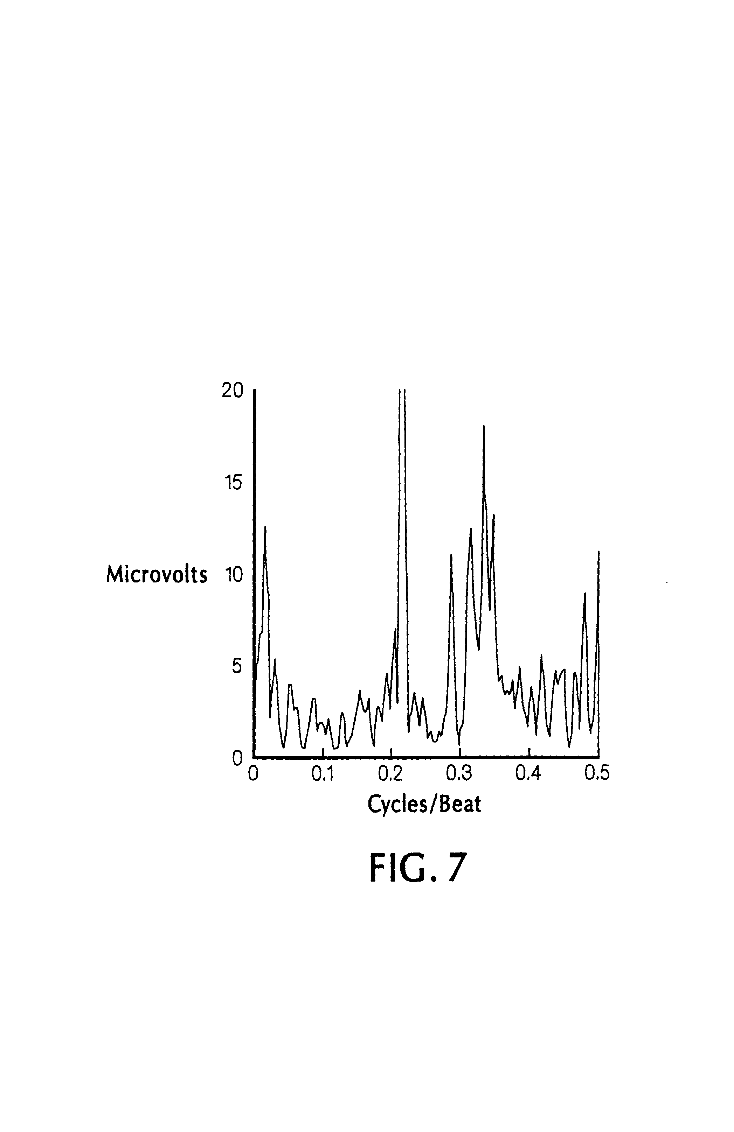

The importance of controlling pedal rate is illustrated in Figs. 5, 6, and 7. These figures show respective plots of power spectra computed from a vector magnitude ECG signal obtained by computing the square root of the sum of the squares of the standard X,Y, and Z lead signals of the vectorcardiogram. The power spectra were computed over the T wave according to the method of U.S. Patent No. 4,802,491 (cited above). Fig. 5 is a plot of a power spectrum obtained by processing data obtained during electrical pacing of the heart. Notice the low level of noise. Fig. 6 is a plot of a power spectrum computed from data obtained during uncontrolled exercise. Notice that the noise level is so high as to make estimation of alternans impossible. Fig. 7 is a plot of a power spectrum computed from data obtained during bicycle exercise where the pedal rate is controlled using a metronome according to the method of this invention. Notice that the artifact induced in the power spectrum resulting from the pedaling does not interfere with the estimation of the peak at the alternans frequency or the reference noise band. In this spectrum one also sees an artifact resulting from respiration which in this case also does not interfere with the alternans measurement.

Example: Other Exercise

Similar considerations apply to the conduct of a treadmill stress test, except that the during a treadmill test motion artifact is introduced into the electrocardiogram at a fundamental frequency of half the step rate and the harmonics of the fundamental. So the desired step rate ranges may be calculated by setting SR = 2PR where SR is the step rate on the treadmill and PR is the pedal rate calculated above for bicycle exercise. The operator may select a protocol with designated heart rate and step rate ranges. The apparatus may indicate to the subject his actual step rate and target range (for example near 1/3 the heart rate), using a means of measuring step rate such as an accelerometer or means to analyze the electrocardiogram signal noise induced by stepping. A metronome may be used to indicate the desired stepping rate. The operator may set the speed of the treadmill to make achieving the desired step rate comfortable to the subject, and then the incline of the treadmill may be adjusted manually by the operator or automatically by the apparatus so that the heart rate is maintained within the desired range. The analysis algorithm may ignore data segments in which the target heart rates and pedal rates do not fall within the desired ranges.

Similar considerations apply to conduct of stair stepping tests as well as to bicycle and treadmill tests.

B. Respiration Artifacts

In one examples the invention may reduce the effect of interfering noise having frequency components that interfere with the alternans measurement generated by respiratory activity in the patient. We have realized that respiration is a rhythmic activity that may interfere with the alternans measurement. The interfering action of respiration is more pronounced during exercise when respiratory effort is intensified. Respiratory activity, or the harmonics of respiratory activity, may overlap with the alternans frequency or the reference noise bands. Generally, the fundamental frequency of respiration falls below the alternans frequency of one half the heart rate and can be simply removed by filtering or by looking at the power spectrum of the signal. However, we have learned that harmonics of respiratory activity may introduce a signal component at the alternans frequency, if the respiration occurs at or near a submultiple of the patient's heart rate. This kind of interference cannot be filtered out with passive filtering techniques.

In a preferred example, the respiratory rate of the subject is controlled to be at a desired rate. The respiratory rate is preferably controlled by having the subject breath in response to a metronome that provides auditory or visual cues to the subject to initiate inspiration. The rate of the metronome is preferably set to a comfortable breathing rate that does not result in interference with alternans measurement resulting from respiration or its harmonics. For example, the metronome rates at either one-third or two-thirds the heart rate are convenient. The heart rate is monitored and the results are used to adjust the frequency of the metronome. In another embodiment, respiratory activity is monitored and respiratory activity signal is used to compensate for the interfering effects of respiration on the determination of alternans. This example is discussed below.

To reduce the effect of respiration on alternans measurements, one may measure a signal closely related to respiration which is derived from the electrocardiogram signal itself. This signal is derived from measurements of the amplitude or vectorcardiograhic spatial angle of electrocardiographic waves such as the QRS complex (e.g. see "Clinical Validation of the ECG-Derived Respiration (EDR) Technique" by G.B. Moody, R.G. Mark, M.A. Bump, J.S. Weinstein, A.D. Berman, J.E. Mietus, A.L. Goldberg, Computers in Cardiology 1986, IEEE Computer Society Press, Washington, D.C., 1987. This approach is convenient because it does not require a separate transducer and apparatus to measure a signal closely related to respiration.

Another way to use the electrocardiogram signal to reduce the effects of respiration is to use the amplitude or orientation of the T-wave itself. One method involves measurement of the energy in the T-wave measurements occurring at frequency bands at even sub-multiples of the heart rate and rejecting data segments where such energy is high (as described above). Another method involves development of a continuous respiration signal which is phase locked to the T-wave (or other respiration-related signal) using phase-locked loop techniques well known in the art, and to develop signals at harmonics of the respiration signal using the same phase-locked loop techniques. The electrocardiogram could then be corrected for respiration using these phase-locked signals and the multidimensional linear finite impulse moving average filter described above.

III  Signal Processing

A.  Abnormal Beats

Grossly abnormal waveforms, such as premature atrial and ventricular beats, disrupt subtle temporal pattern of beat-of-beat variability in waveform morphology, such as alternans. Abnormal beats can cause phase resetting of the alternans sequence so that instead of an ABABABABABAB type of pattern, a pattern of the type ABABAPBABABA might result or a pattern of the type ABABAPABABAB might result, where P designates a premature beat. The premature beat can be readily identified and in the prior art has generally been replaced with a mean beat (average of A and B). However the since the phase of all the beats following the premature beats may be reset, the overall energy at the alternans frequency (i.e., at exactly half the heart rate) may be reduced. Thus, analysis of the beat sequence, even excluding the premature beat itself, is subject to large errors. It is found that premature beats located in the middle section of a data epoch tend to cause the largest error in the estimation of alternans. For example, if the voltage of a point on the T-Wave is represented by the sequence $V_n = V_o + a(-1)^n$ where n is the beat number, a is the amplitude of the alternating component, and $V_o$ is the mean voltage. Then, the squared amplitude of the alternating component, $a^2$, can be obtained by multiplying $V_n$ by $(-1)^n$, summing over n = 1....N where N is the number of beats in the sequence, dividing by N, and squaring the result. This computation is equivalent, within a proportionality constant, to computing the magnitude of the power spectrum at the alternans frequency (0.5 cycles per beat). However, if the sequence $V_n$ has a phase reversal precisely half-way through the sequence, then the computation results in a value of zero. The result of the computation approaches the correct value of $a^2$ as the phase reversal approaches the beginning or end of the sequence

Abnormal beats may be identified by determining if their morphology differs from the normal waveform morphology by more than a predetermined threshold or if the preceding intercycle interval differs from the mean intercycle interval by more than some predetermined threshold. Once the abnormal beats are identified, it is generally advantageous to choose a data epoch with few or no abnormal beats. If some abnormal beats remain in the data epoch to be analyzed for the presence of alternans, the level of alternans may be computed according to one of the following methods

Example: Estimating Alternans Over a Frequency Band

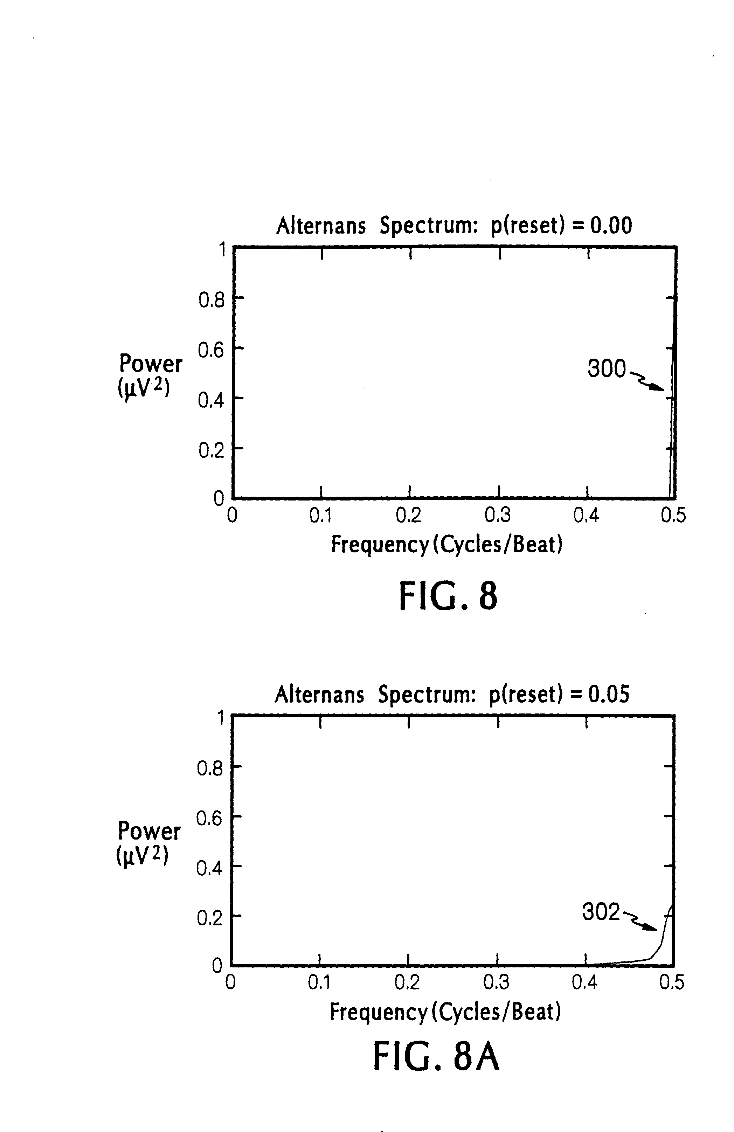

According to one example of the use of the apparatus of the invention, the abnormal beats are replaced with the mean normal beat and then the power spectrum is computed according to one of the methods known in the art (e. g., Cohen et al., U.S. Patent No. 4,802,491, cited above). As shown in Figs. 8 and 8A, the presence of phase resettings tends to broaden the peak at the alternans frequency. Referring to Fig. 8, for a "perfect" alternans sequence [+1, -1, +1, -1, ...] of 128 points, the alternans spectrum has a clean, sharp spike 300 at the alternans frequency. When an alternans sequence has a 5% probability of phase-resetting at a given beat, the alternans peak 302 is broadened, as shown in Fig. 8A. Thus the level of alternans, in the presence of abnormal beats which may reset the phase of the alternans, is computed by integrating the power spectrum over frequencies adjacent to the alternans frequency.

The desired range of frequencies may be estimated by simulating phase-resetting caused by abnormal beats, e.g., by using the sequence $X(i) = (-1)^i$ with a length corresponding to the number of beats in the epoch. This sequence is modified by setting the values of $X(i)$ equal to zero at values of i corresponding to abnormal beats and then for each abnormal beat multiplying the value of all subsequent points in the sequence by 1 or -1 (the value of 1 or -1 for each abnormal beat is chosen randomly). The power spectrum of the resulting sequence is computed and averaged for several realizations of the random phase resetting, and the width of the spectral peak at the alternans (Nyquist) frequency is measured. This width is used to determined the range of frequencies used to integrate the power spectrum obtained from the physiologic data to estimate the level of alternans. It is understood in this preferred embodiment that one may perform operations equivalent to integrating the power spectrum over a wider range of frequencies. In this example, it is preferable to choose a data epoch where the abnormal beats are not located near the center of the epoch.

In another example, instead of replacing the abnormal beats with the mean normal beat, the abnormal beats are eliminated from the beat sequence and from the simulated sequence used to estimate the width of the alternans peak in the power spectrum.

Example: Combining Analyses of Normal Beat Sequences

In another example, one separately analyzes each sequence of,normal beats between abnormal beats and combines the analyses in such a way that the fact that the phases of the different sequences may be different does not affect the alternans measurement. One expects no phase resetting within each sequence of normal beats, but different sequences may have different phases (e.g., one sequence may have a 1, -1, 1, -1... pattern and another sequence may have a -1, 1, -1, ... pattern). In this example , one uses any of the techniques in the art to estimate the level of alternans in each sequence of beats lying between abnormal beats, and then combines these measurements. For example, one may compute the power spectrum of each sequence and then average the power spectra appropriately weighing the spectra for the number of beats contained therein. Alternatively, one may measure in each sequence a mean even beat waveform and a mean odd beat waveform, and compute an unsigned measure of the difference in these two waveforms as a measure of the level of alternans. This unsigned measure of the difference could be, e.g., the integrated absolute difference or the integrated squared difference.

Example: Stitching Together Beat Sequences

Another example involves combining the different sequences of beats between abnormal beats. We have realized that abnormal beats tend to reset the phase in a consistent way and this can be used to reduce the effects of interfering noise on the measurement of alternans. According to this scheme, sequences of normal beats are connected to maintain an alternating phase.

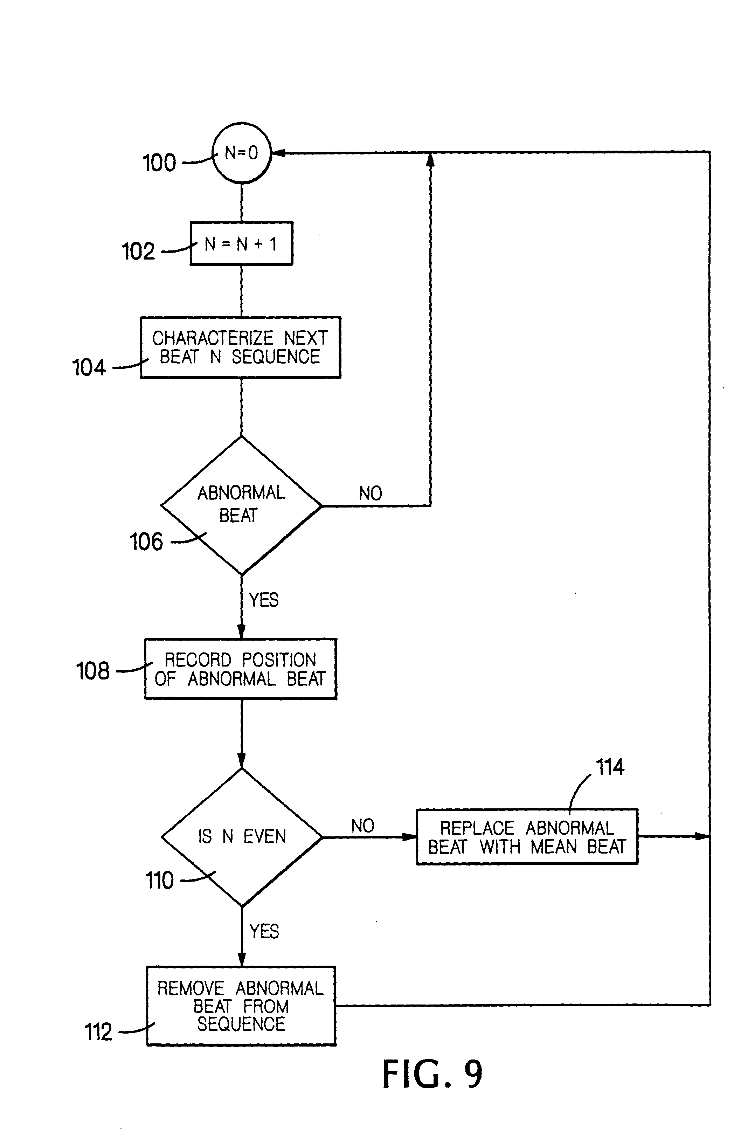

In one presently preferred example, an abnormal beat is removed from a beat sequence to preserve an alternating phase between the beats, or an abnormal beat is replaced by a mean beat, depending on the position of the abnormal beat in the sequence. Abnormal beats are replaced or removed by the process shown in Fig. 9. At an initialization step, the number of beats (N) since the previous abnormal beat is set to zero (100). The value of N is increased by one (102). The next beat in the sequence is characterized (104). If the beat is not abnormal (106), the process returns to the initialization step (100), otherwise the position of the abnormal beat is recorded (108). At this stage, if N is even (110), the abnormal beat is removed from the sequence (112) and the process returns to the initialization step (100). If value of N is odd, the abnoraml beat is replaced with a mean beat (114) and the process returns to the initialization step (100).

For example, using the process shown in Fig. 9, the sequence of beats $A_1B_2A_3B_4P_5A_6B_7A_8P_9A_{10}B_{11}...$ is replaced with the sequence $A_1B_2A_3B_4-A_6B_7A_8A-M-_{10}B_{11}$ (where A represents normal beats of phase A, B represents normal beats of phase B, P represents an abnormal premature beat, and M represents the mean beat). In this preferred embodiment, the resulting sequence of beats may be analyzed to determine the level of alternans using any of the methods in the art.

In another example, a normal beat adjacent an abnormal beat may be removed to preserve an alternating phase. For example, the sequence of beats $A_1B_2A_3B_4P_5A_6B_7A_8P_9A_{10}B_{11}...$ is replaced with the sequence $A_1B_2A_3B_4$-$A_6B_7$-$A_{10}B_{11}$.

B. Compensation for Interfering Variability in Measured Signals

The morphology of the physiologic waveforms may be altered by processes which interfere with the measurement of alternans ('interfering noise sources') such as the beat-to-beat variability in interbeat interval, respiratory activity, and exercise activity. In a preferred embodiment signal (which we will term 'interfering signals') are measured which contain contributions from the interfering noise sources, the interrelationship between the interfering signals and the physiologic waveforms is analyzed, and the measures of variability in the physiologic waveforms are compensated for the interfering variability.

Example: Intercycle Interval Variability

Intercycle interval variability may disrupt the temporal pattern of cycle-to-cycle variability (for example alternans) or make changes in the morphology of the physiologic waveform. Changes in the preceding R-wave to R-wave (RR) interval or intervals of the electrocardiogram cause changes in the shape and timing of the T-wave. These changes in the T-wave can mask the alternans pattern of shape changes and make the measurement of alternans more difficult. Since alternans is clinically most significant when measured during the T-wave, and measuring alternans without electrical pacing of the heart means that there will be variation in the RR interval, the sensitivity of the alternans measurement can be improved if the noise caused by this variation in interval is reduced.

Therefore, in one example the effect of intercycle variability on variation on waveform morphology is reduced by determining a relationship between the variation in intercycle intervals and changes in the waveform morphology. This relationship is then used to adjust the waveforms to compensate for the effect of intercycle interval variation. In one example, the relationship is determined by a multidimensional linear finite impulse moving average filter. Such a filter linearly relates the amplitude of the waveform at each offset from a fiducial point in the waveform to the sequence of preceding intercycle intervals:

$$V_a(i,j) = V(i,j) + \sum_k a(k,j) \cdot \Delta RR(i-k)$$

here $V_a(i,j)$ is the amplitude of the i'th waveform in a particular lead at an offset of j sample points from the fiducial point adjusted to compensate for the effect of intercycle interval variation, $V(i,j)$ is the corresponding unadjusted amplitude, and $\Delta RR(i-k)$ is the deviation of the intercycle interval k beats prior to the i'th beat from the mean. The sum over k ranges from 0 (for the immediately intercycle interval) to a maximum integer value of p. The coefficients $a(k,j)$ relate the variation in the preceding intercycle intervals to the variation in the waveform amplitude. The coefficients $a(k,j)$ can be determined by least squares estimation techniques which are well known in the art. In a preferred embodiment the maximum value of the index k is between 1 and 5 the sampling interval is 40 milliseconds.

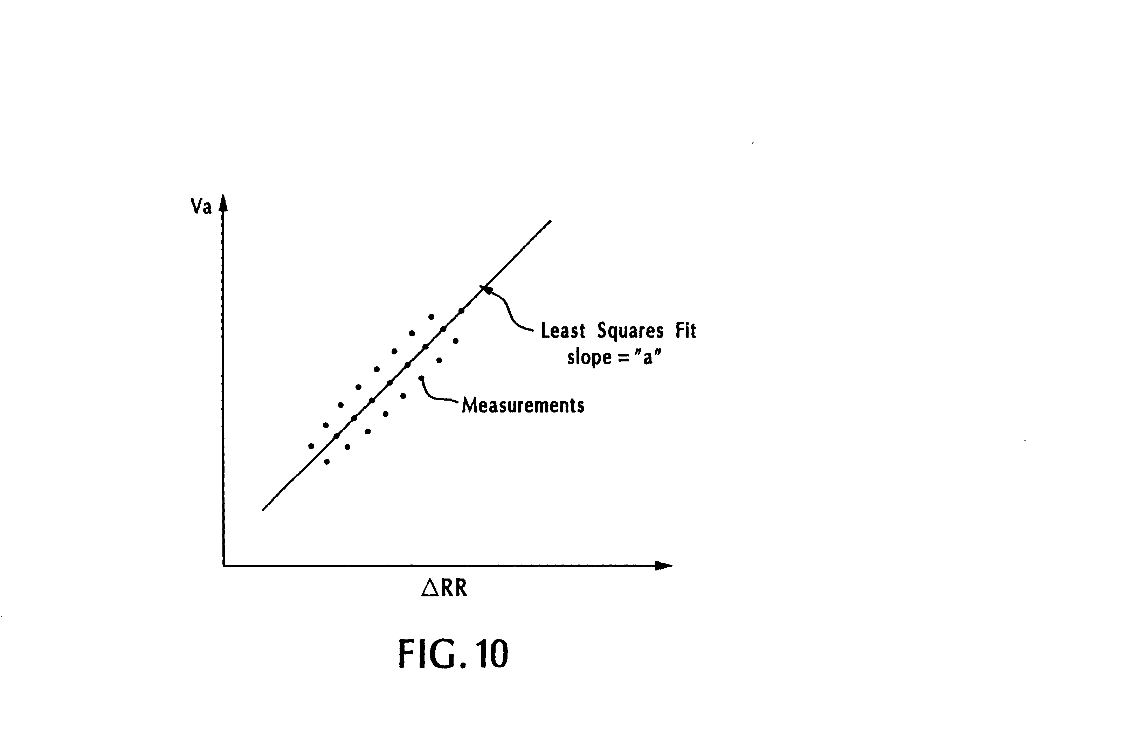

Fig. 10 demonstrates how this works for correcting a single point on the T-wave. Measurements are made of the voltage ($V_a$) of a series of T-waves at this point and these are related to the deviation ($\Delta RR$) of the previous RR interval from the mean RR interval. A least squares fit to these data provides the coefficient "a" (corresponding to the slope), which can be used to correct this point on the T-wave for the variation in the previous RR interval.

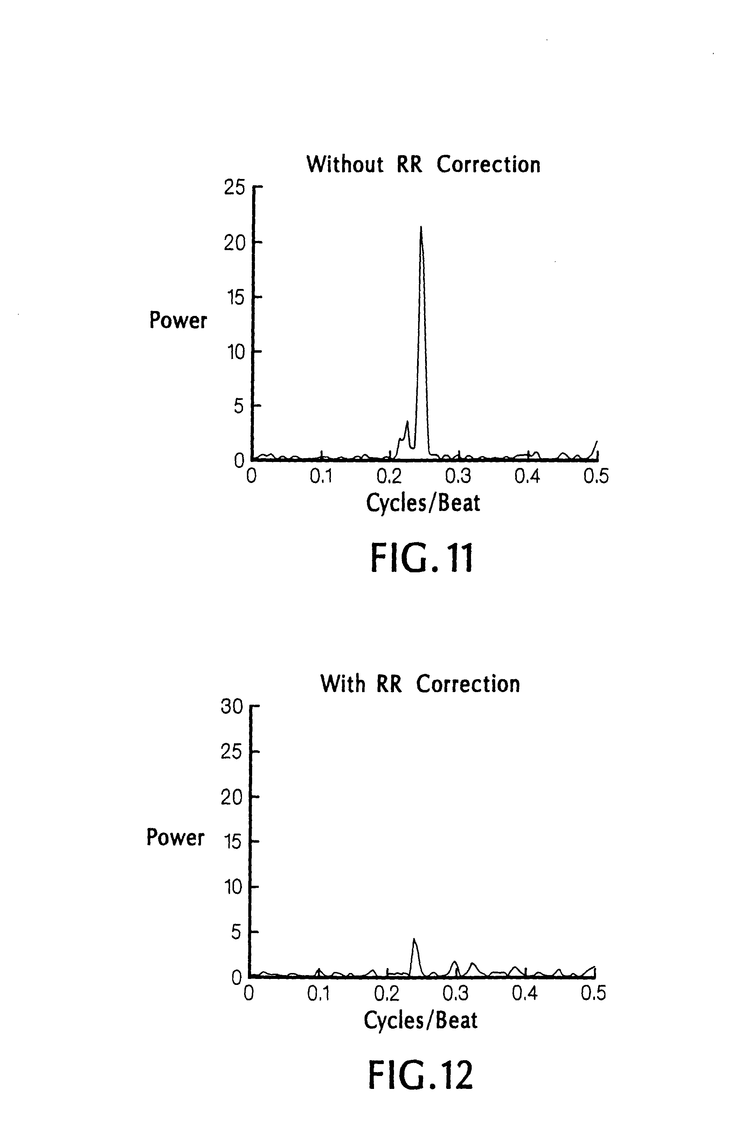

Fig. 11 shows the alternans spectrum calculated by the means described in Smith et al., 1988 (cited above), where RR interval variation has caused an elevation of the noise levels. Fig. 12 shows a spectrum of the same data where the moving average filter described above has been applied. Note that the noise level in the spectrum has been reduced.

Other means are available to perform the same task. For example, each T-wave can be represented as a weighted sum of basis functions. These basis functions can be obtained by performing a principal components analysis of all of the T-waves in the data epoch using methods well known in the art. Then each T-wave is represented as follows:

$$V(i,j) = \sum_{k} c(i,k) \bullet b(i,j-k)$$

where V(i,j) is the amplitude of the i'th waveform at an offset of j sample points from the fiducial point, c(i,k) is the weighting coefficient for the kth basis function for the i'th waveform, and b(k,j) is the value of the $k^{th}$ basis function at the $j^{th}$ sample point offset.

The coefficients c(l,k) may in turn be adjusted to compensate for the effect of interbeat interval variability:

$$c_a(l,k) = c(l,k) + \sum_{m} d(m,k) \bullet \Delta RR(l-m)$$

where $c_a(l,k)$ is the adjusted value of the coefficient, d(m,k) is a set of coefficients relating coefficients for the $k^{th}$ basis function to $\Delta RR(1-m)$, which is the deviation of the preceding RR intervals from the mean. The value m is summed over a range starting with m=0 (the immediately preceding $\Delta RR$). The coefficient d(m,k) may be computed using a least squares minimization procedures well known in the art. The adjusted values of the waveform, $V_a(i,j)$ is then computed:

$$V_a(i,j) = \sum_{k} c_a(i,k) \bullet b(j,k).$$

In another preferred embodiment, the effect of RR interval variability *is* represented as a change in the amplitude and offset of the T-wave: $V_a(i,j) = \alpha(i) \bullet V(i, j-\beta(i))$. Here, $\alpha(i)$ and $\beta(i)$ represented the modified amplitude and offset of the $i^{th}$ waveform. The values of $\alpha(i)$ and $\beta(i)$ may be related to the preceding $\Delta RR$ (1-m) using a filter such as linear moving average filter, as described above.

In a preferred example, these methods for compensating for the effects of intercycle variability are applied during physiologic stress.

Example: Variability in Other Signals

The measurement of the alternans pattern of waveform variability without direct electrical pacing of the heart often involves the detection of generally smaller signal levels, the detection at a broader range of heart rates, and the detection in the presence of other physiological variability which may be caused by exercise or other physiological stress. Therefore, one preferred example of the use of the apparatus is a method for assessing the alternans pattern of cycle-to-cycle variability in physiologic waveforms of a physiologic signal in which the effect of variability in one or more other signals on the variability in the physiological signal being measured is reduced by determining a mathematical relationship which relates measures of the variability in the other signal or signals to measures of the variability in the physiologic signal being measured and using this mathematical relationship to adjust the measures of the variability of the physiologic signal being measured to compensate for the effect of variability in the other signal or signals.

For example, respiration can affect the shape of electrocardiographic complexes thus making it more difficult to measure the alternans pattern of variation. The level of respiration is increased during exercise and physiological stress and therefore the problem becomes more acute when physiological stress is used for the measurement of alternans. By measuring a signal closely related to respiration (such as an impedance plethysmography signal which can be measured from the same electrocardiographic electrodes using techniques well known in the art) as well as the electrocardiogram signals, one can determine a mathematical relationship between the respiration related signal and the morphology of the electrocardiographic waveforms. For example, the mathematical relationship may be characterized as a multidimensional linear finite impulse moving average filter relating the amplitude of the electrocardiographic waveform at each offset from a fiducial point in the waveform to the amplitude of the respiratory signal. One example of such a filter is similar to that described above for the compensation of the effects of variation in intercycle interval

$$V_a(i,j) = V(i,j) + \sum_k a(k,j) \bullet \Delta S(i,j-k)$$

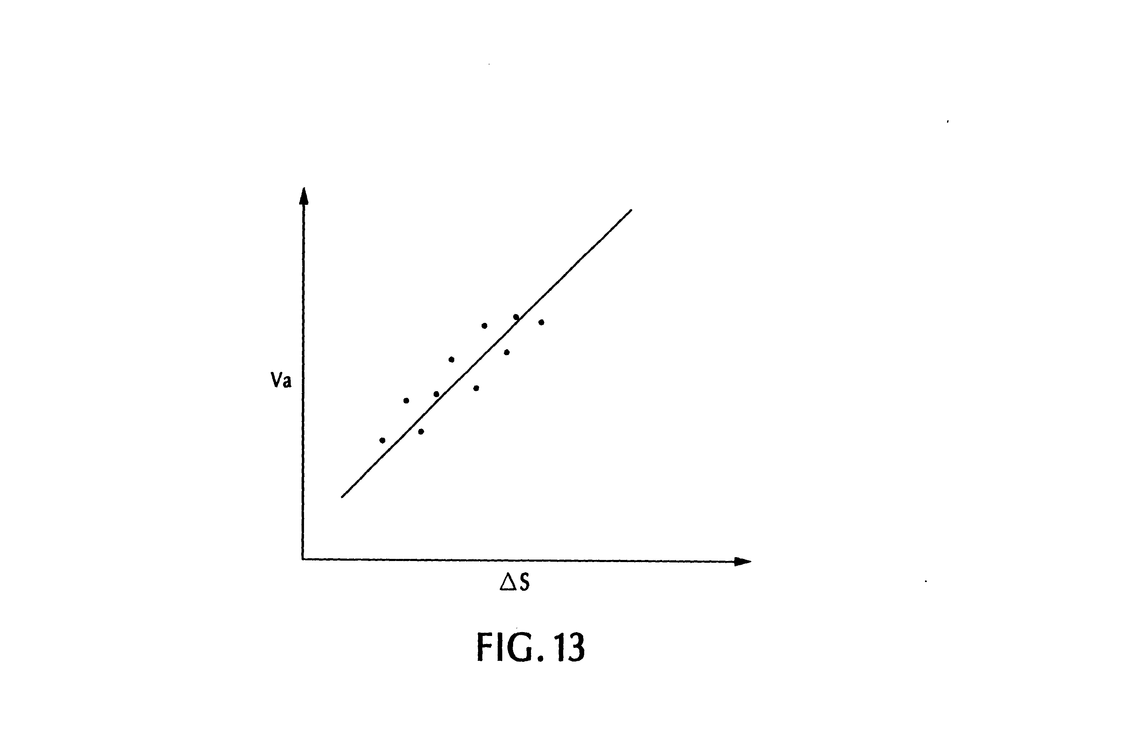

where $\Delta S(i,j-k)$ *is* the deviation of the respiration related signal from its mean value at k sample points preceding the time associated with $V_a(i,j)$. Here the coefficients a(k,j) related the variation in the amplitude of the respiration related signal S with the variation in the electrocardiographic amplitude given by V(i, j) (see Fig. 13). In one preferred example, significant benefit is obtained when the maximum value of the index k is unity. In this example the electrocardiographic amplitude is corrected by the contemporaneous value of the respiratory signal. It may also be necessary to create filters to compensate for signals equal to $\Delta S$ raised to integer powers to adjust for the effects of harmonics of respiration related signals on the morphology of electrocardiographic signals.

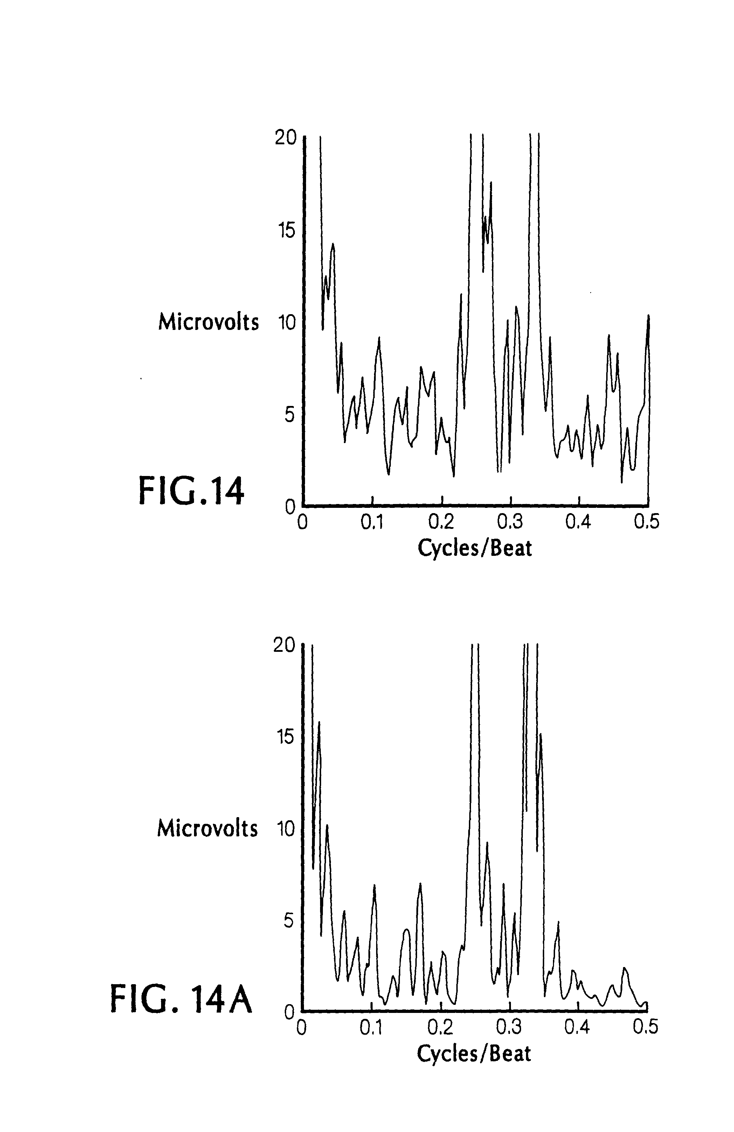

The importance of correcting for the effect of respiration is illustrated by a study conducted by us of the level of alternans in thirty healthy young adults during bicycle exercise. In the absence of compensation for the effects of respiration, eight subjects were found to have a significant level of alternans. This quite surprising result occurred because they were breathing at a sub-multiple of their heart rate. As a result, one of the harmonics of their respiration created a variation in their ECG waveform morphology at the alternans frequency (see Fig. 14, which shows respiration from one of these subjects at one quarter of the heart rate producing a false positive peak at the alternans frequency of one half the heart rate). However, as shown in Fig 14A, when the electrocardiographic waveforms were adjusted using an independently measured impedance plethysmographic respiration related signal, alternans fell to insignificant levels.

C. Use of Multiple Electrodes or Multi-segment Electrodes

Signals recorded from multiple electrodes may be used to reduce the effect of noise which interferes with the measurement of a temporal pattern of cycle-to-cycle interval variation in physiologic waveforms. In one preferred example one simply obtains the measure of the alternans pattern of cycle-to-cycle variation in physiologic waveform morphology in electrocardiographic signals which is the level of alternans in those leads in which the level of alternans exceeds the noise by approximately 2 or 3 standard deviations. This preferred example ignores the leads in which the noise may be so high as to make it impossible to detect alternans.

A description of a systematic method for mathematically analyzing multiple signals to reduce the effect of noise on the measurement of a temporal pattern of cycle-to-cycle variation in physiologic waveforms is described in United States Patent Application 08/339,032, filed November 14, 1994, entitled "Measuring a Physiologic Signal,". In a preferred example, an assessment of the temporal pattern of cycle-to-cycle variation in electrocardiographic waveforms is accomplished by applying to a subject a multiplicity of electrodes, recording signals from the electrodes, determining the mathematical relationship between the signals from the different electrodes, defining an error metric, and finding a mathematical combination of electrode signals to approximate a desired set of electrocardiographic lead signals which also reduces the error metric, and analyzing the temporal pattern of cycle-to-cycle variability in the electrocardiographic waveforms of the mathematical combination of electrode signals. This method may be applied where the temporal pattern being assessed is alternans. In one preferred example, the desired set of electrode lead signals are the X, Y and Z leads of the vectorcardiogram. In another preferred example, the desired set of electrocardiographic lead signals are approximated over one or more segments of the electrocardiographic waveforms. In another preferred example, the mathematical relationship involves the covariance.

In one preferred example, the error metric involves a measurement of noise over one or more segments of the electrocardiographic waveforms, such as the PQ segment or the T-wave. In another preferred example, the error metric involves measurement of noise over one or more frequency bands. In another preferred example, the computation of the error metric involves one or more of the following: obtaining a measure of the noise, obtaining a measure of the magnitude of the temporal pattern of cycle-to-cycle variability being assessed, obtaining a measure of . the relative magnitudes of the temporal pattern and the noise, and obtaining a measure of the statistical significance of the magnitude of the temporal pattern.

In one preferred example, a multiplicity of individual electrodes, or a compound electrode with multiple independent electrical contacts, are applied at a localized anatomic site. The electrocardiographic signals from these multiple electrodes are then used to estimate a reduced noise signal corresponding to the electrocardiographic signal emanating from that localized anatomic site.

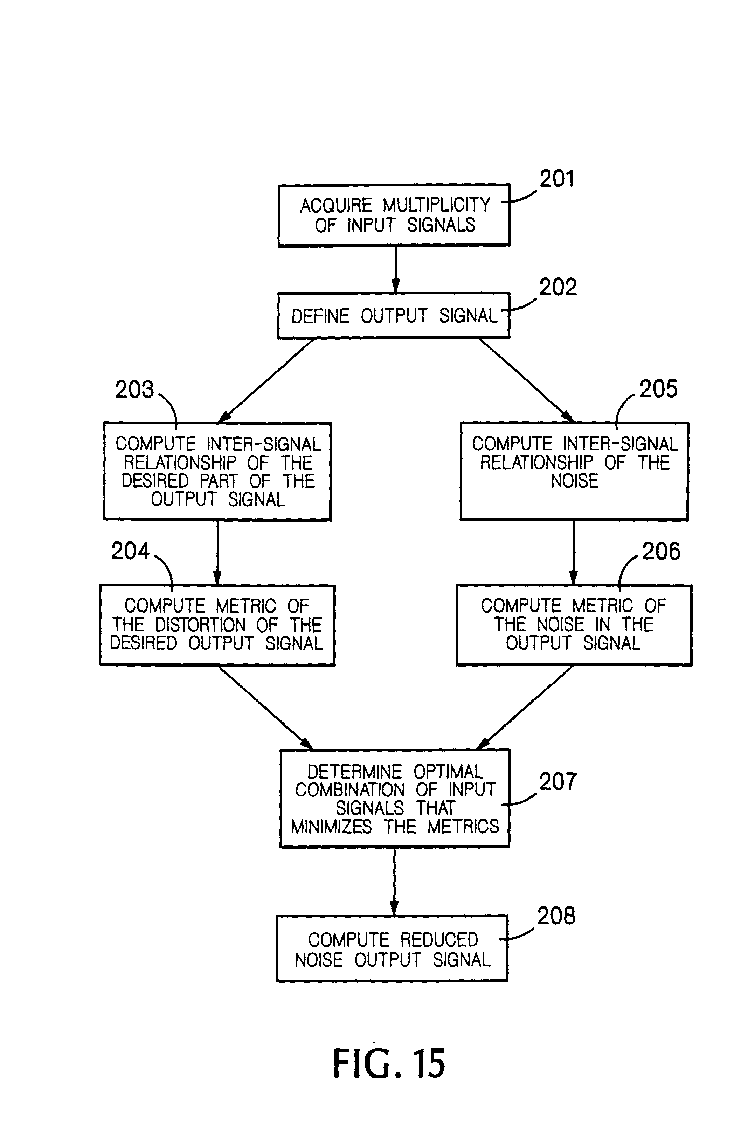

With reference to Fig. 15, a general method of using the apparatus of the invention is summarized beginning with step 201 in which the input signals are acquired. Each signal is assumed to contain some amount of the desired signal or some amount of the noise signal, or both. In step 202 the decision is made as to what kind of output signal is to be generated. Often, the desired output signal is simply one of the input signals but, more generally, it can involve transformations of the input signals.

Steps 203 and 204 define the relationship of the desired signal among the input signals. Step 203 first transforms the input signals by means of a mathematical process that has a filtering effect which preserves the desired features and then computes the inter-signal relationship of the features is by computing the correlation between the processed signals. Then, in 204, a distortion metric is created which uses the correlation function to establish a measure of how any specific combination of input signals will distort the desired features of the output signal.

Steps 205 and 206 are similar to steps 203 and 204 except that the mathematical transformation preserves the noise features of the signal, and the metric measures of how much noise any given combination of input signals contributes to the output signal.

In step 207 the two metrics from steps 203 through 206 are considered simultaneously, and the combination of input signals which minimizes the aggregate metric is computed. The signals are combined in step 208 to produce a low noise output signal. The low noise signal is referred to as the optimized signal.

The apparatus of the invention is discussed with respect to the problem of measuring the electrical alternans in the ECG. The measurement of the electrical alternans presents a challenge because its amplitude is often as low as a microvolt. By contrast, it is not uncommon for the obscuring noise to reach peak levels of a millivolt. The noise is usually comprised of multiple sources such as beat-to-beat variability in the shape of the ECG waveforms caused by respiration, voltage fluctuations generated by displacements at the electrode-skin interface due to motion, and skeletal muscle activity.

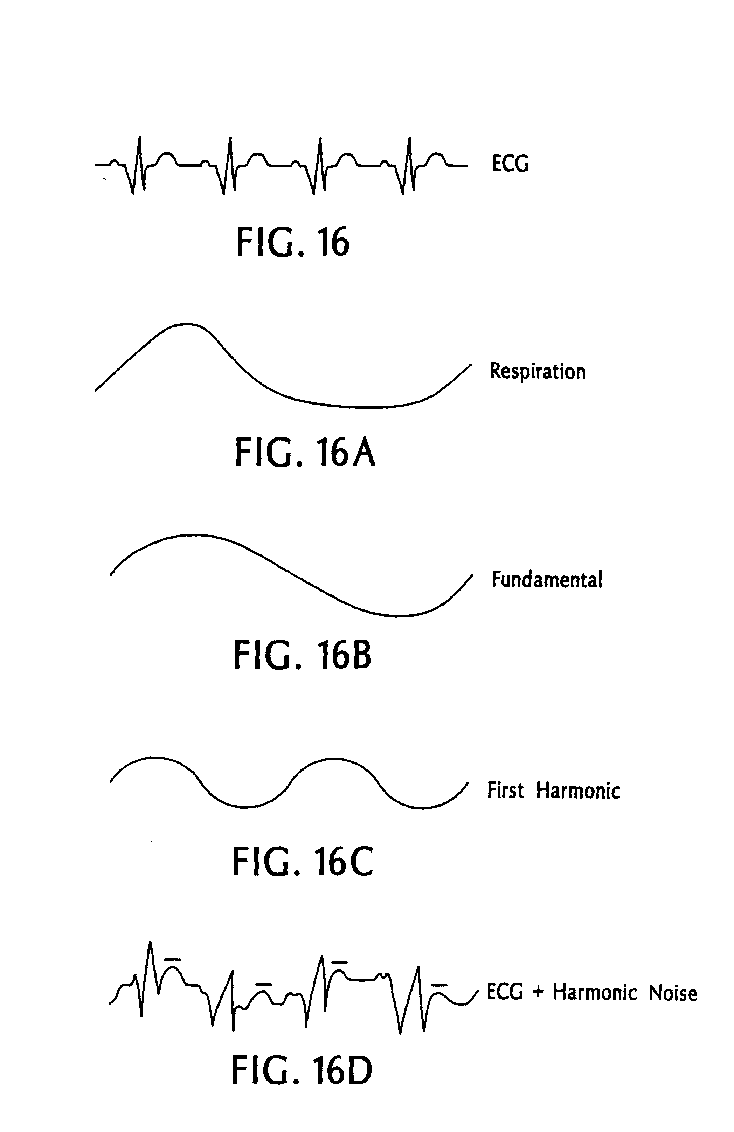

Of the three sources, the first two pose a special problem since they can act to mimic alternans. For example, as shown in Figs. 16-16D, if the patient is breathing at one-fourth or one-sixth of the heart rate, a harmonic multiple of respiration will occur at the alternans frequency. The harmonic can impart alternans to the ECG by means of its influence on the beat-to-beat variability and by means of electrode noise generated by the respiratory motion of the chest.

An even more complicated situation can occur, when noise due to the patient motion occurring with one periodicity interacts with the respiratory effects at a second periodicity, combining in a non-linear fashion to produce noise at a third periodicity which can mimic or mask the presence of alternans. For all these reasons, the reduction of interfering noise is of paramount importance for the measurement of alternans.

The example demonstrates the use of the method to reduce the noise of the ECG in a manner that makes it more suitable for the analysis of electrical alternans. The apparatus measures the ECG, respiration, and ECG electrode impedance. The respiration provides a signal related to noise of the beat-to-beat variability in the ECG, while the impedance provides a signal that is related to noise cause by displacement of the electrode-skin interface.

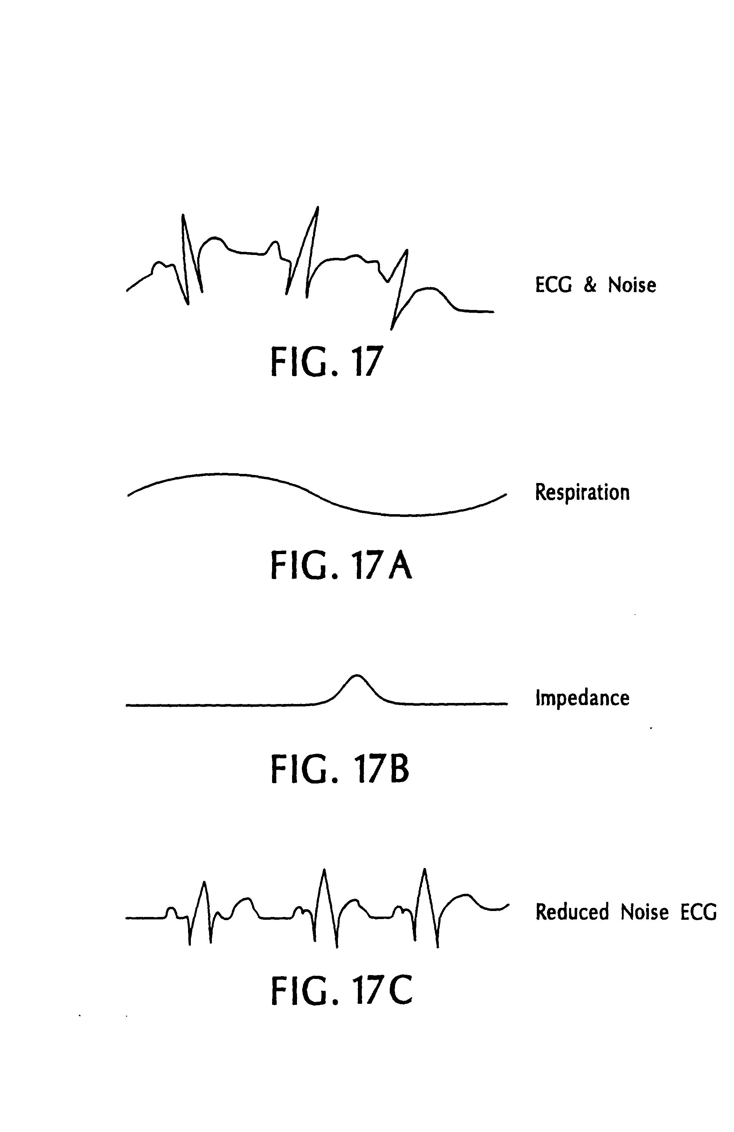

Referring to Figs. 17-17C, respiration and impedance signals are combined to reduce the noise of the measured ECG signal.

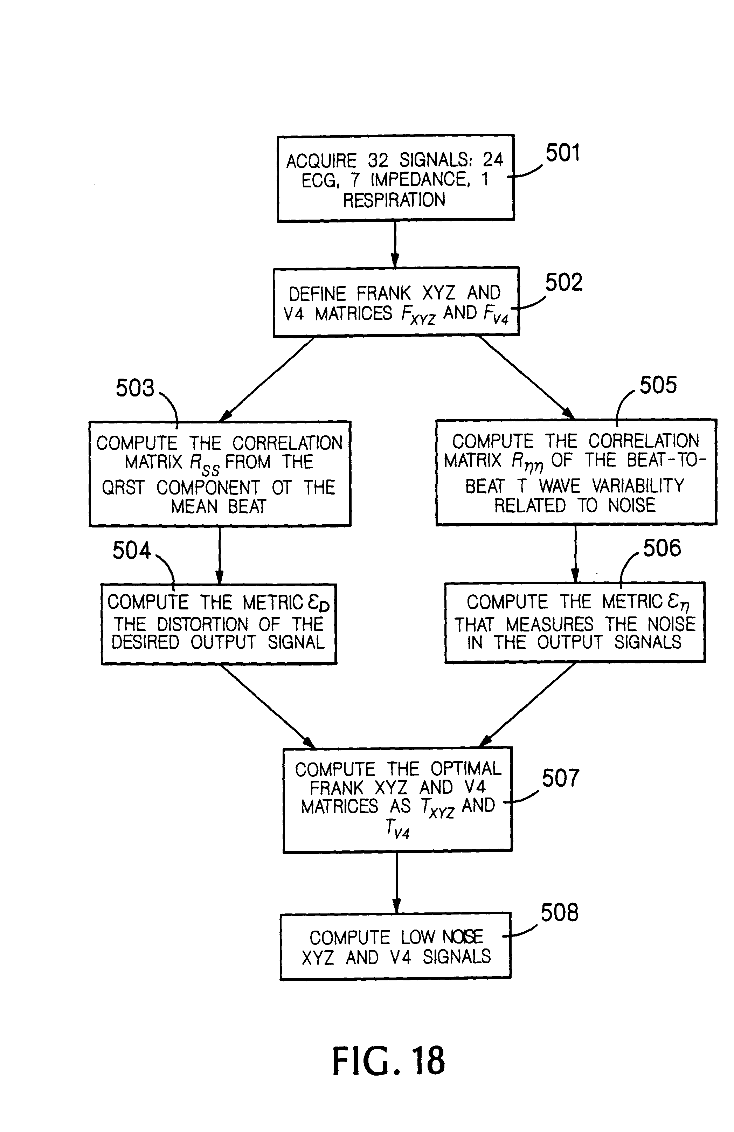

The steps of the preferred example follow the flowchart of Fig. 18, which parallels the general method put forth in Fig. 15. The preferred example uses a matrix formulation to express the method of the invention. Other embodiments may use other implementations such as tensor formulations or polynomial function formulations. The detailed mathematical justification is omitted from the description and can be found in Appendices A, B and C.

Electrode Structure

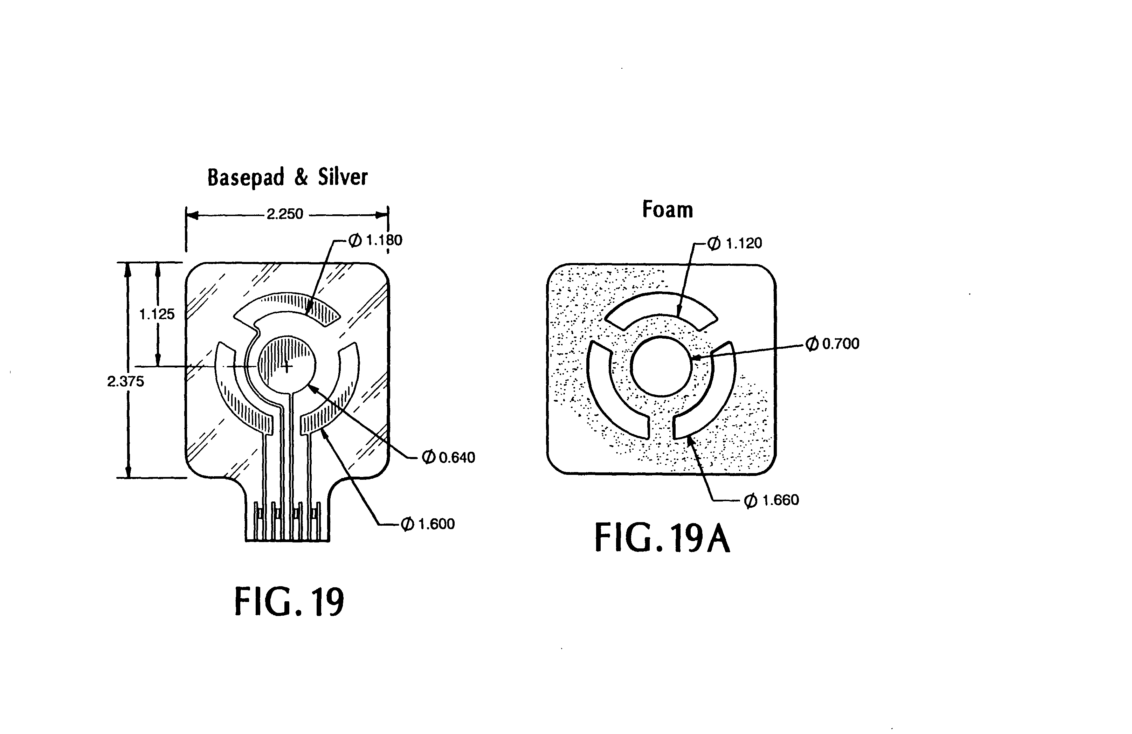

As shown in Figs. 19 and 19A, the multi-segment electrode is constructed of a basepad made of:a film printed with silver-chloride ink. The ink creates segments that provide the electrical connection to the electrode gel while providing adequate defibrillation recovery characteristics. The ink is also used to create traces which continue to the bottom edge of the basepad, where they are brought into a parallel formation suitable for an edge connector.

A plastic flexible foam is attached to the base pad. The foam covers much of the exposed areas of the traces and insulates them. It leaves the areas corresponding to the electrode segments exposed, thereby creating wells that provide convenient method of holding the gel that make the electrical connection from the ink to the skin. The foam has an adhesive on the surface that holds the electrode to the skin of the patient.

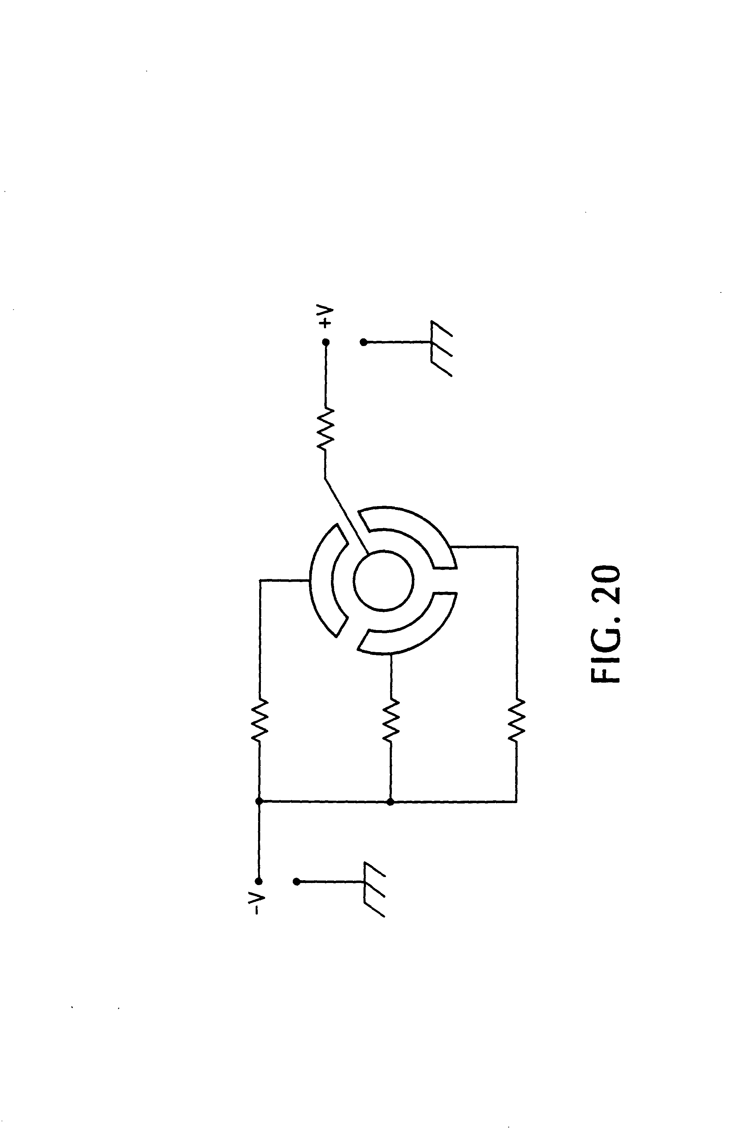

The use of a multi-segment electrode has several advantages. First, the ECG recorded by the segments usually varies differently between the segments than does the noise. This is largely attributable to the differing physical locations of the source of the ECG and the source of the noise. Second, the variation in the noise caused by separation of the segments can be adjusted in a controlled fashion by introducing a small DC bias current on each segment of the electrode. Fig. 20 shows a diagram for application of the DC bias to the multi-segment electrode. The variation can also be adjusted by manufacturing the different segments using different electrolyte mixtures.

Respiration and Impedance Measurement

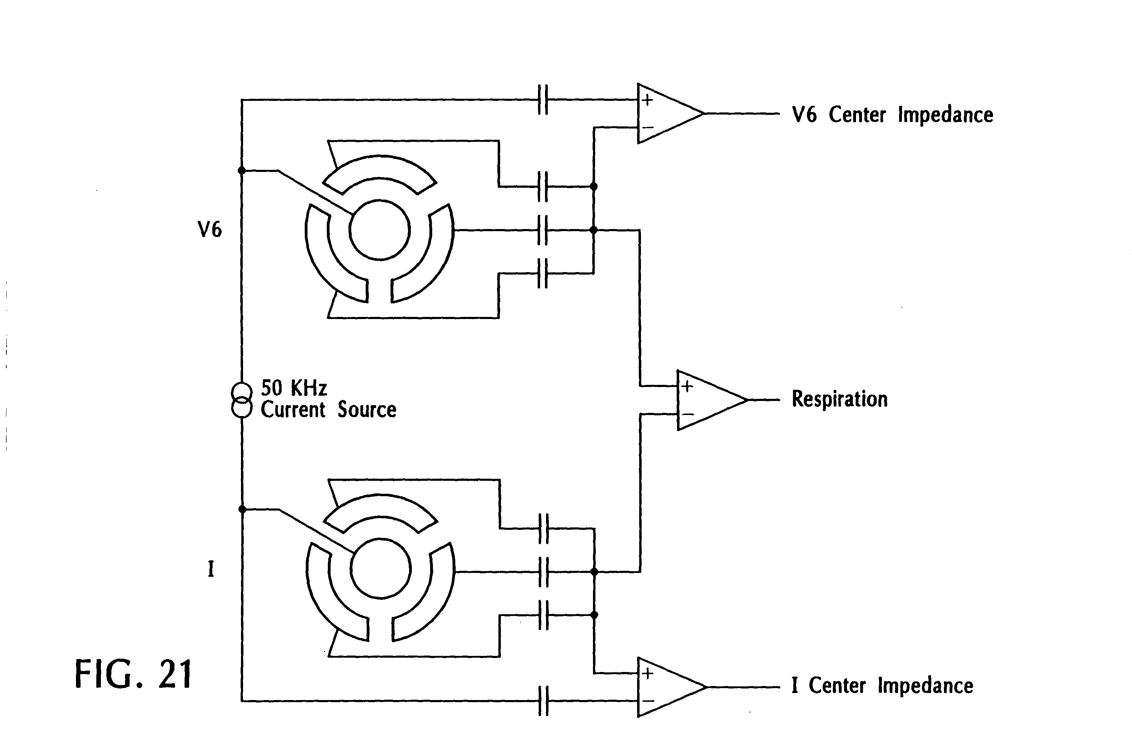

The multi-segment electrode facilitates the measurement of respiration and electrode impedance as described in Fig. 21. Impedance of the skin-electrode interface is measured because it provides a signal representative of noise, which can be used with other measured signals to help cancel noise from an alternans measurement. We belive that this procedure is particularly advantageous because we have found that changes in impedance are correlated with noise artifact in the alternans measurement, which permits noise cancellation.

The measurements are enabled by injection of a small 50 Khz current between the centers of two of the multi-segment electrodes. The outer ring segments are capacitively coupled so that, at 50 kHz, the ring acts as a single segment.

Referring to Fig. 21, respiration is measured as a 4-terminal measurement using the rings as measurement electrodes. As the impedance across the chest changes due to inflation and deflation of the lungs, the measured voltage increases and decreases. The voltage is demodulated and high-pass filtered to provide the respiratory signal.

Impedance of the center segment of each electrode is measured by measuring the voltage drop between the center and ring. Since the measurement draws essentially no current, the ring impedance has little effect on the measured voltage. The voltage is demodulated and high-pass filtered to provide the impedance signal. The multi-segment electrodes are placed at the locations whose signals are most important to the measurement of electrical alternans.

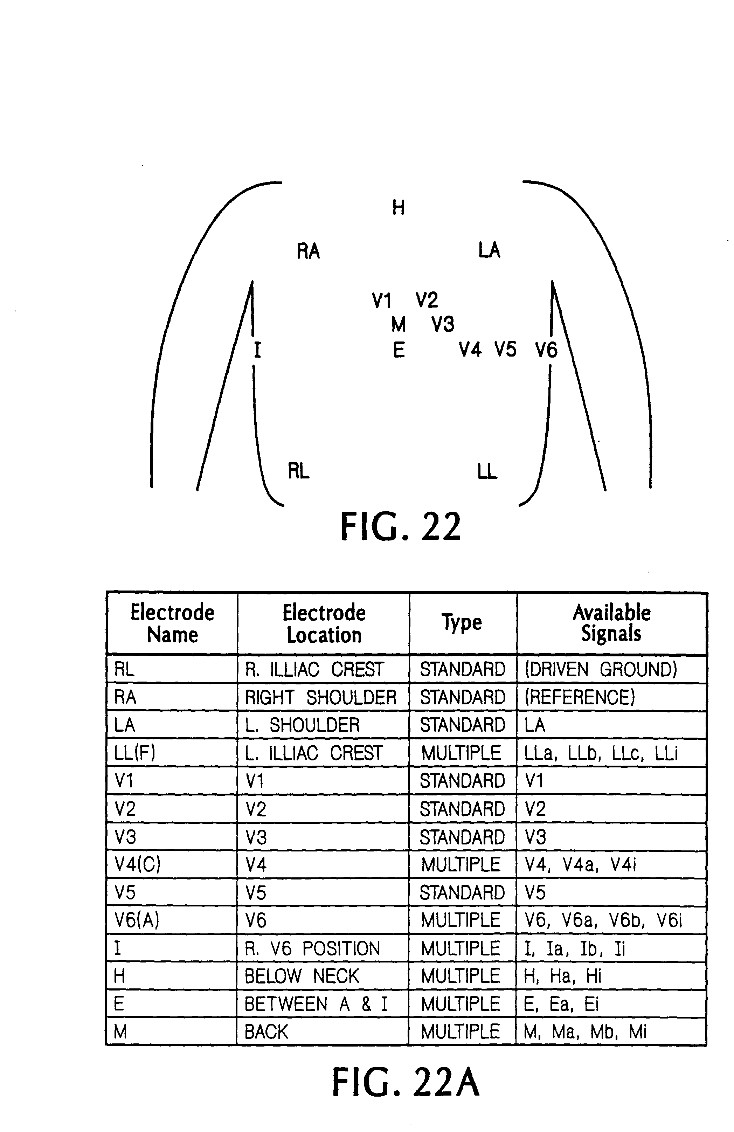

Referring to Figs. 22 and 22A, a total of 32 signals are acquired using the 14 ECG electrodes. Of the 32 channels, there are 24 ECG signals, 7 impedance signals, and 1 respiratory signal. The ECG signals are named after the electrodes, with the suffix "a", "b", or "c" referring to signals from the ring segments. For most of the multi-segment electrodes, two or three of the outer ring segments are joined together to form a larger segment (in order to reduce the number of ECG channels that need to be recorded). All the ECG signals are recorded referenced to Wilson's central terminal, which represents the average of the voltage at the RA, LA, and LL electrodes.

The 7 impedance signals measure the center segment impedance of the 7 multi-segment electrodes. They are given name of the electrode with the suffix "i".

The respiratory signal is a measure of the chest impedance from the right to the left side. As the lungs inflate and deflate, the impedance across the chest changes since air conducts less well than the body tissues. Respiration changes the impedance between the heart and the body surface and thereby changes in the measured ECG. Therefore, measuring respiration by impedance increases its usefulness for canceling changes in the ECG that are cause by respiratory impedance changes.

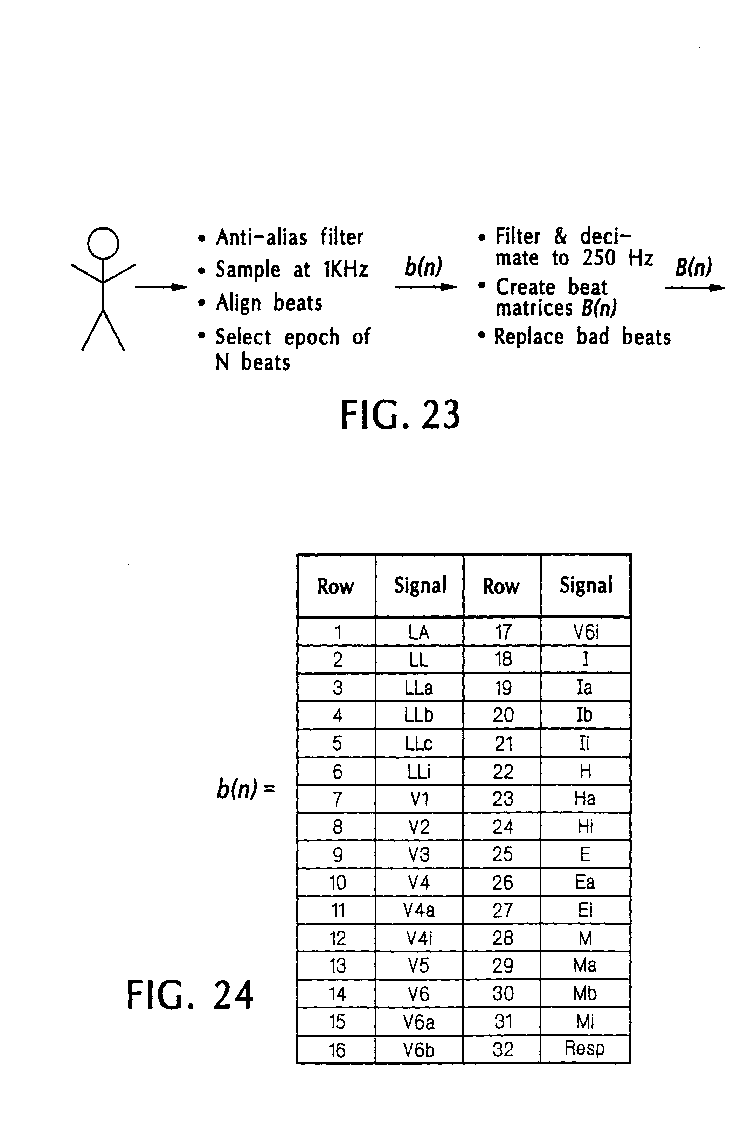

Figs. 23-26 summarize the digitization and the preprocessing of the signals. The ECG, impedance, and respiratory signals are acquired from the patient electrodes as described in Figs. 22 and 22A. The signals are amplified, anti-alias filtered and digitized at 1Khz to create an M=32 dimensional time series b(k). The contents of the rows of b(k) are shown in Fig. 24.

The beats are detected, aligned and classified, and an epoch of N beats is chosen for analysis. The epoch is chosen on the basis of features such as heart rate, the availability of normal beats, and the general noise level. The heart rate is important because it has been found that alternans is more easily detected at heart rates over 100 beats per minute.

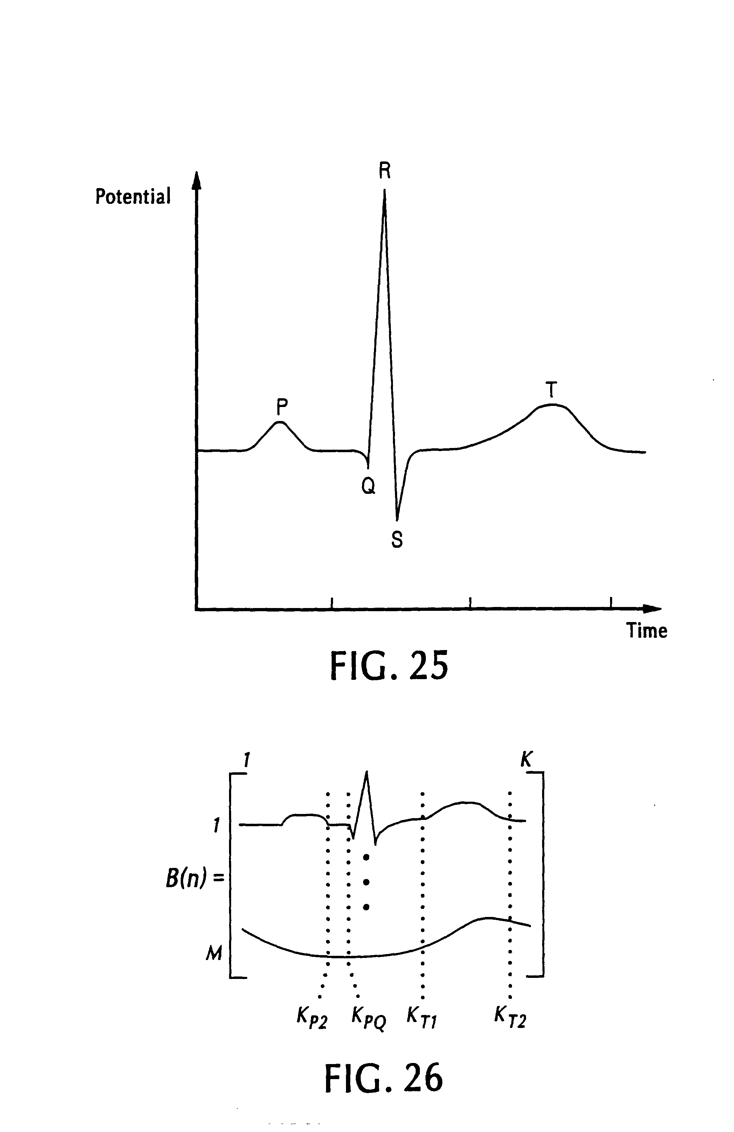

Fig. 25 shows the features of a single ECG beat. Briefly, the P wave corresponds to activity in the atria, while the QRST complex corresponds to ventricular activity. The QRS complex represents the electrical activation of the ventricles, while the T wave represents the electrical recovery. The ST segment is a relatively quiescent period. In humans, it has been found that T wave is the best interval of the ECG complex for detecting alternans.

For each of the n=1,N beats in the epoch, the beat is aligned on the QRS complex, filtered, decimated to 250Hz around the alignment point, and stored in matrix B(n) of dimension MxK. The decimation allows for some data reduction and lowers the data storage requirements.

The structure of B(n) is shown in Fig. 26. Row m of B(n) corresponds to the row m of b(n). The number of columns K is determined by the duration of the longest beat. The k=1...K columns correspond to successive digital samples over the duration of the beat. The samples start before the beginning of the P wave and end after the T wave. The indices $K_{P2}$ and $K_{PQ}$ delineate the PR interval; $K_{T1}$ and $K_{T2}$ delineate the T wave.

Beats which are classified unusable for reasons of morphology, timing, or excessive noise are excluded from analysis. Abnormal morphology is usually the results of beats which originate from an abnormal conduction patterns in the heart. Such beats interfere with the stable manifestation of alternans. Beat which have occurred very early or very late relative to the average time interval between beats also destabilize the alternans. In the preferred embodiment, the beats B(n) which are excluded from the analysis are replaced with the mean value of B(n) computed over the remaining beats. For other preferred embodiments, replacement methods based on interpolation, filtering, or likelihood estimation may be preferable.

Output signals

Step 502 defines the output signals as the Frank XYZ vector signals and the V4 signals. The XYZ signals have the advantage that the computation of the vector magnitude from XYZ creates a signal which is immune to alternans artifact created by rotation of the heart. The V4 signal is representative of the V1-V6 signals, which are known contain information not entirely contained in the XYZ signals.

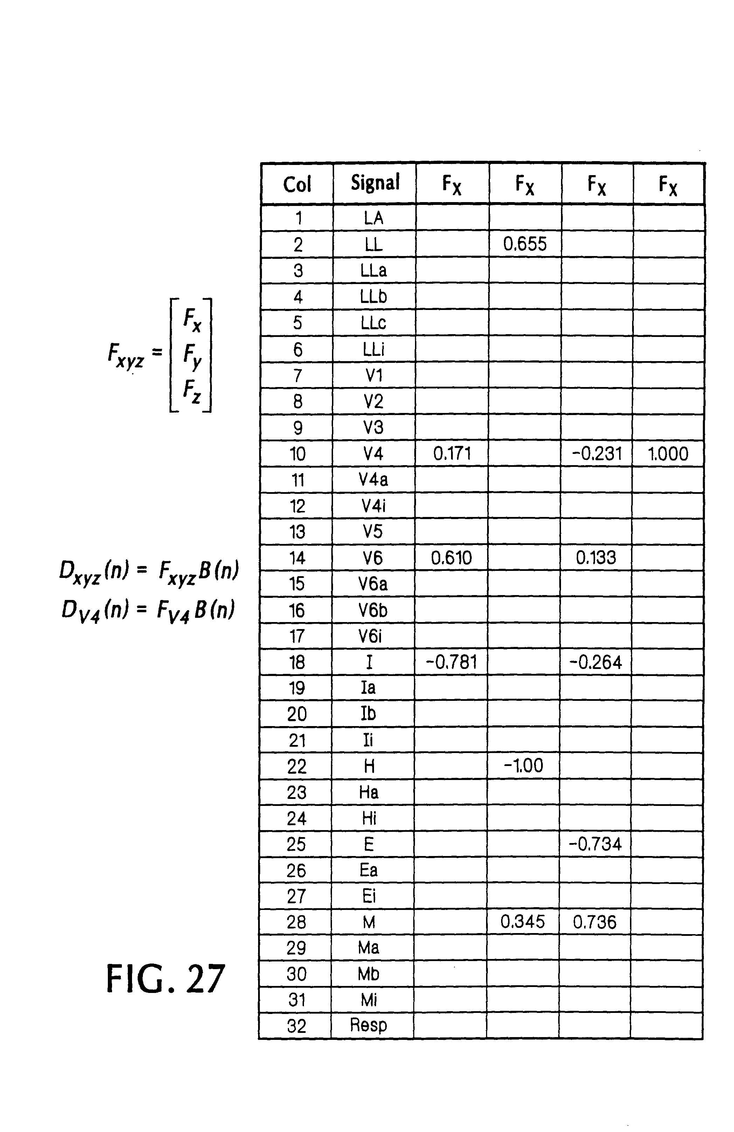

Fig. 27 shows the how the X, Y, Z, and V4 output signals are defined in terms of the input signals. Other embodiments may define output signals that include any or all of the other commonly used ECG signals such as V1-V6, or they may create signals from any generalized combination of input ECG signals.

The coefficients necessary to create the X, Y, Z, and V4 leads are represented in the row vectors $F_X$, $F_Y$, $F_Z$, and FV4 whose coefficients are shown in the table in Fig. 15. The output signals corresponding to B(n) are denoted by D(n); for example, $D_X(n)=F_X B(n)$. The 3xM matrix that consists of $F_X$, $F_Y$, $F_Z$ is defined as $F_{XYZ}$ and creates the 3xK matrix of the XYZ signals denoted by $D_{XYZ}(n)=F_{XYZ}B(n)$.

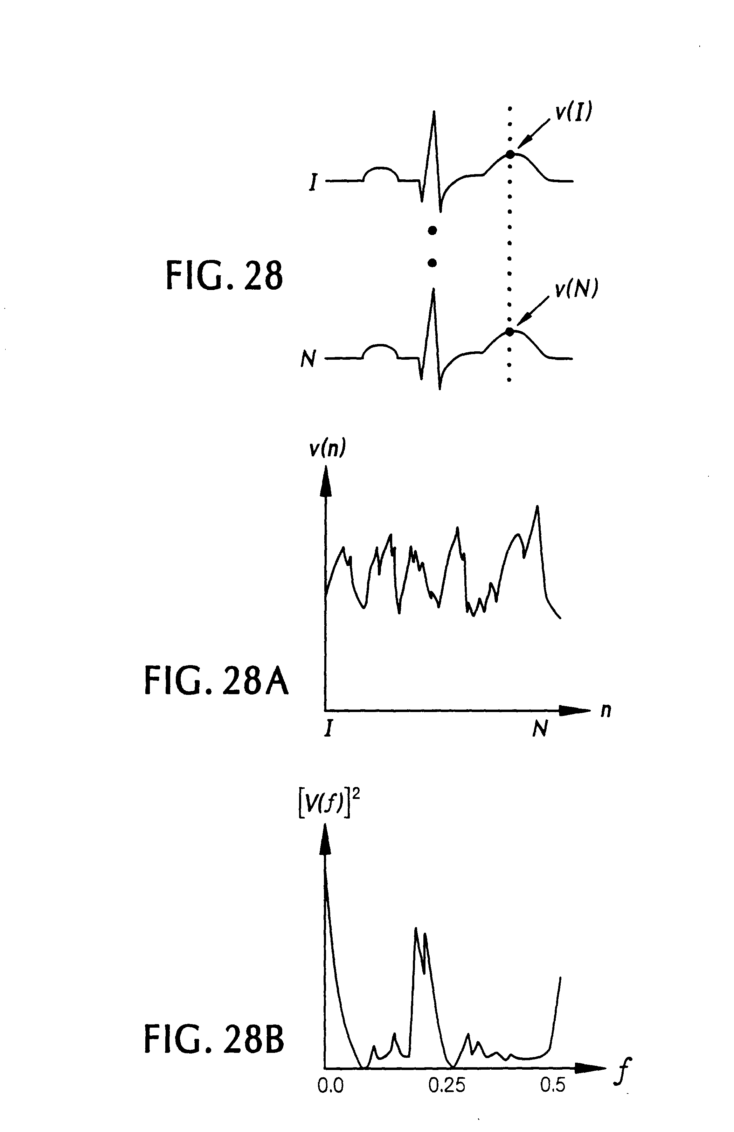

Figs. 28-28B summarize a method of computing alternans from the unoptimized output signals $D_{XYZ}(n)$. The vector magnitude $D_{VM}(n)$ is created and the T-wave region of the vector magnitude beats is divided into columns of points as represented by v(n). The power spectrum of v(n) is computed as V(f). The frequency corresponding to 0.5 cycles per beat is the alternans frequency. The level of alternans is considered significant if it exceeds the level of noise reference band by an amount equal to three standard deviations of the level in the reference band. The alternans in the V4 lead is computed using the same procedure as the used form computing the alternans of the vector magnitude.

The matrices that create the output signals using an optimal combination of the input signals are denoted by $T_X$, $T_Y$, $T_Z$, and $T_{V4}$. The structure of the T matrices parallels that of their F matrix counterparts; however, their coefficients are not pre-defined and are computed based on the specific data obtained for a given individual or recording. Once computed, the $T_X$, $T_Y$, $T_Z$, and $T_{V4}$ replace their counterparts $F_X$, $F_Y$, $F_Z$, and $F_{V4}$ in the computation of alternans.

Computation of signal relationships and error metrics

Step 503 uses the average value of the QEST complex of the ECG beats as the desired signal to be preserved. The mean of B(n) is computed across all the n and is scaled on a point-by-point basis by the corresponding value of the square root of the average vector magnitude computed from $D_{VM}(n)$. This scaled average is denoted by BS. The inter-signal relationship, denoted by $R_{SS}$, is then computed as the MxM covariance matrix of the form $B_S B_S{}^*$, where only the columns of $B_S$ corresponding to the QRST are used in the computation. Step 504 uses the metric of distortion given by

$$\in_D = tr[(T_{XYZ}-F_{XYZ})R_{SS}(T_{XYZ}-F_{XYZ})^*] \tag{1}$$

where the "*" is a transpose operator, and tr[] is the trace of the square matrix. The metric measures the average power of the error in the representation of BS of the optimized output signals relative to the definition of those signals.

Other embodiments may use substantially different methods of creating $B_S$. The input signals B(n) may be transformed by much more complex filtering techniques that preserve the desired features in a series $B_s(n)$, which is then used to compute the covariance $R_{SS}$.

Other embodiments may choose to use an $R_{ss}$ that is an identity matrix. An identity matrix imposes a penalty if the coefficients of $T_{XYZ}$ differ from $T_{XYZ}$. The matrix $R_{ss}$ can also be crafted based on a priori knowledge about the input signal.

Step 505, uses B(n) processed by a filter that has a frequency response of Sin $(2\pi f)$, where f is the frequency as defined by the alternans power spectrum of Figs. 28-28B. The filtering process creates one new $B_\eta(n)$ matrix corresponding to the each of the original B(n) matrices. The inter-signal relationship, denoted by $R_{\eta\eta}$, is then computed as the MxM covariance matrix taking in equal contribution the columns from each $\beta_\eta(n)$. Only the columns of B(n) corresponding to the T wave are used in computing the covariance. If the alternans is to be computed over intervals of the QRST other than the T wave, those intervals must also be included in the computation of the covariance. Step 506 uses the metric of noise given by

$$\in_\eta = tr[T_{XYZ}R_{\eta\eta}T^*_{XYZ}] \tag{2}$$

which measures the average noise power in the optimized output signals.

Preservation of the signal vector direction

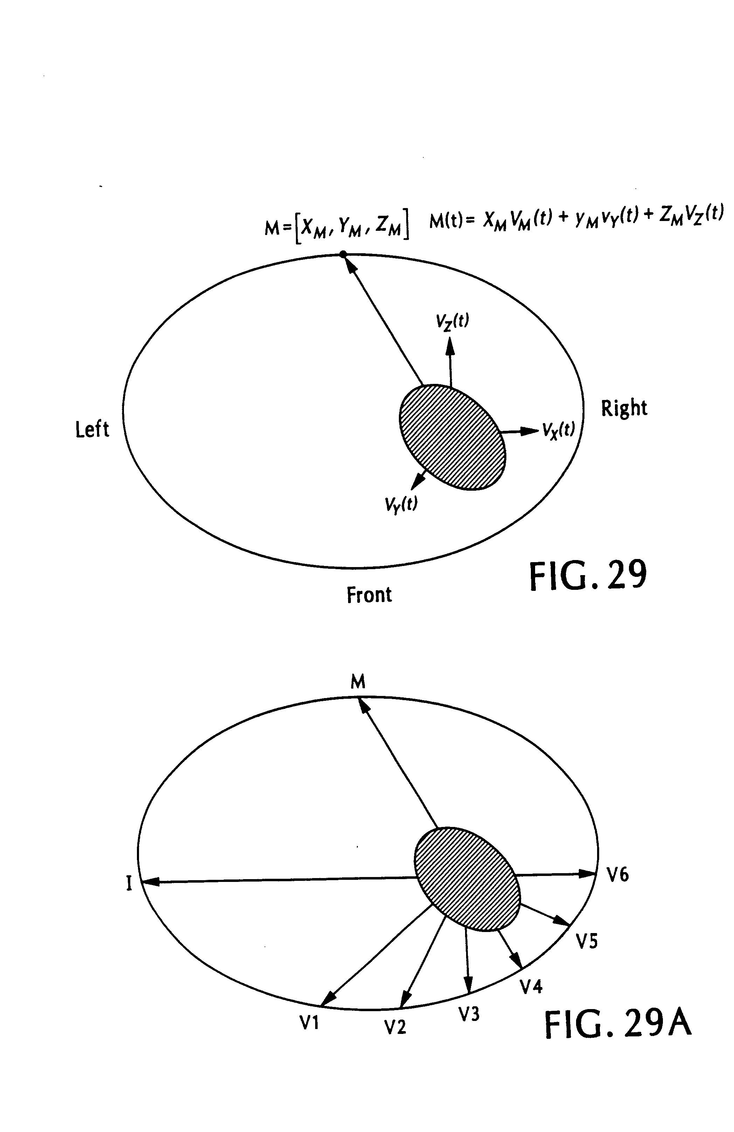

The preferred embodiment constrains the computation of $T_{XYZ}$ to maintain the mathematical orthogonality of the XYZ dipole generator defined in Figs. 29-29A. Examining the TX component of $T_{XYZ}$ we see that it creates a combination of the 24 ECG signals, each of which consists of a specific combination of $[v_X(t), v_Y(t), V_Z(t)]$. When the vector contribution of all 24 ECG signals is summed together scaled by the coefficients of $T_X$, the net sum must be the vector [1 0 0]. That is to say, $T_X$ must be one unit of X contribution from the dipole source and zero units of Y or Z. The analogous computations for $T_Y$ and $T_Z$ must yield [0 1 0] and [0 0 1], respectively.

The matrix $P_{XYZ}$ is defined to contain the X, Y, and Z vector coordinates of each input signals in its three columns. Each row corresponds to a signal, with the order being same order as the columns of $T_{XYZ}$. Rows corresponding to non-ECG signals are assigned a coordinate of [0 0 0].

Using this definition of $P_{XYZ}$, the orthogonal geometry constraint can be stated as

$$T_{XYZ}P_{XYZ} = I \qquad [3]$$

where I is a 3x3 identity matrix. The value of PXYZ used to satisfy equation [3] is given by

$$P_{XYZ} = R_{SS}F^*_{XYZ}(F_{XYZ}R_{SS}F^*_{XYZ})^{-1} \qquad [4]$$

Equation [4] is derived by taking the output signals defined by $F_{XYZ}$ as the $[v_X(t), v_Y(t), v_Z(t)]$ of the dipole source, and then using the $R_{SS}$ inter-signal correlation matrix to compute how much X, Y, and Z is present in each of the input signals.

The solution for $P_{XYZ}$ which enforces the orthogonality constraint for the preferred embodiment, has a more general interpretation. The $P_{XYZ}$ provides the least squares means of reproducing the input signals using the subset of the information in the output signals. Therefore, the constraint defined by equations [3] and [4] may be used for the computation of $T_{V4}$, though the interpolation becomes different.

When T is not $T_{XYZ}$, the constraint is referred to as the "round trip" constraint. It assures that if the output signal is used to estimate the input signals, and those input signals are in turn used to recompute the output signal, then recomputed output will be the same as the original output. Other embodiments may choose to use the "round trip" constraint.

Computing the optimal combination

The two metrics are combined into a single metric given by

$$\in = \in_\eta + \alpha\in_D \qquad [5]$$

and the value of $T_{XYZ}$ is computed to minimize subject to the orthogonality constraint of equation [1]. The value of $T_{XYZ}$ that minimizes equation [3] is given by

$$T_{XYZ} = T_0 + \alpha F_{XYZ}R_{SS}R^{-1}_0(I - P_{XYZ}T_0) \qquad [6]$$

where

$$T_0 = (P^*_{XYZ}R^{-1}_0 P_{XYZ})^{-1}(P^*_{XYZ}R^{-1}_0) \qquad [7]$$

and

$$R_0 = R_{\eta\eta} + \alpha R_{SS} \qquad [8]$$

In the preferred embodiment, the value of $\alpha$ is usually between 10 to 100. For other embodiments, it may not be appropriate to minimize the desired signal distortion. In that case, X is 0 and $T_{XYZ}$ is given by

$$T_{XYZ} = (P^*_{XYZ}R^{-1}_{\eta\eta}P_{XYZ})^{-1}(P^*_{XYZ}R^{-1}_{\eta\eta}) \qquad [9]$$

Other embodiments may choose to minimize the distortion error by reconstructing the original signals from the optimal output signals and comparing them to the original input signals. This is accomplished by replacing the distortion metric in equation [1] with

$$\in_D = tr[(PT - I)R_{SS}(PT - I)^*] \qquad [10]$$

Computing the optimal V4

By analogy to the argument leading to the derivation of equation [3], $T_{V4}$ creates linear combination of the 24 ECG signals, each of which consists of some combination of $[v_X(t), V_Y(t), v_Z(t)]$ as defined in Figs. 29-29A. When all the vector contribution of each of the 24 ECG signals is summed together using the coefficients of $T_X$, the net sum must be the $P_{XYZ}$ row that corresponds to V4. Denoting that row as $Q_{V4}$, the analogy to equation [3] becomes

$$T_{V4}P_{XYZ} = Q_{V4} \qquad [11]$$

The value of $T_{V4}$ is computed to minimize with of equation [5] $F_{V4}$ and $T_{V4}$ in place of FXYZ and TXYZ, subject to the constraint of equation [11] is given by

$$T_{V4} = Q_{V4}T_0 + \alpha F_{V4}R_{SS}R_0^{-1}(I - P_{XYZ}T_0) \qquad [12]$$

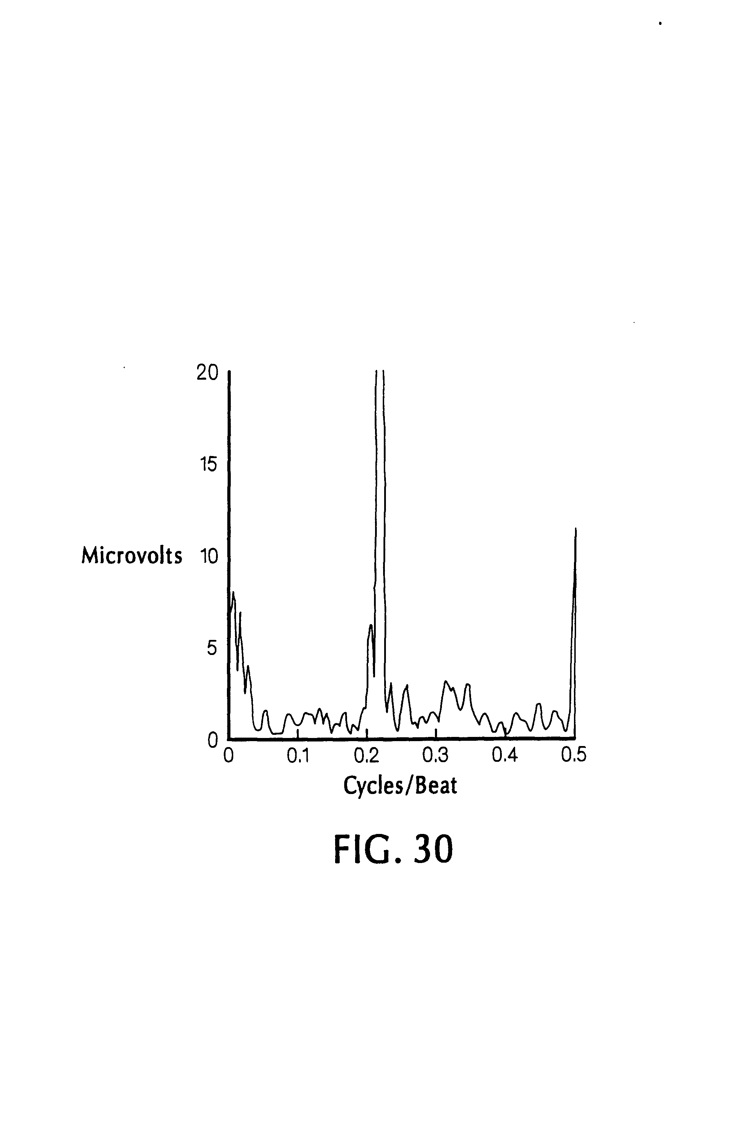

Fig. 30 demonstrates the effectiveness of the use of multiple electrodes according to the method of the invention. This figure corresponds to the same patient data as Fig. 7. The patient had electrodes placed for the measurement of the standard vectorcardiographic X, Y and Z leads and in addition had electrodes placed for measurement of the standard 12 lead electrocardiogram. All the leads were used to compute according to the method of the invention a noise reduced estimate for each of the X,Y and Z leads. The estimates were then combined to compute the magnitude of the electrocardiographic vector (square root of the sum of the squared voltages in leads X, Y, and Z). This vector magnitude signal was then used to compute the power spectrum shown in Fig. 30. In Fig. 7, the vector magnitude signal was computed directly from X, Y and Z leads. Comparing the power spectra in Figs. 7 and 30 one sees the tremendous noise reduction which has been achieved by the use of the multiple electrodes; in particular, the pedaling artifact is essentially eliminated.

IV. Real-Time Analysis

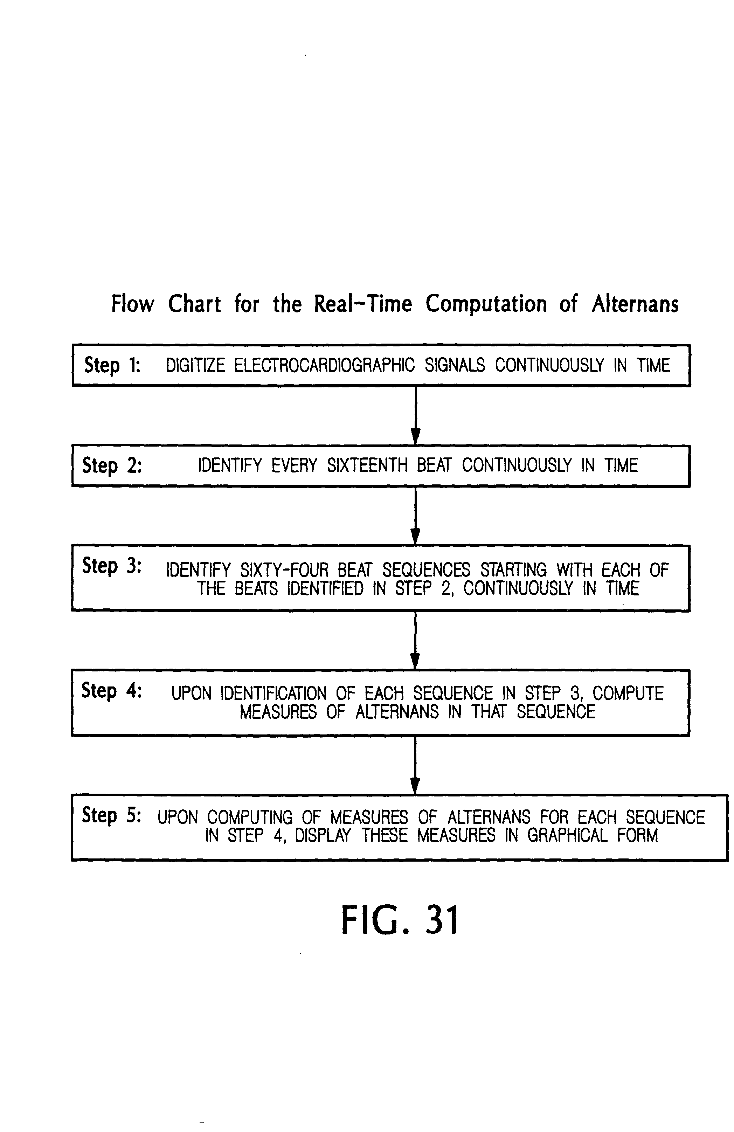

In one preferred example of this invention, measures of alternans or other measures of the temporal pattern of waveform variability are computed and displayed in real-time as the data is accumulated. In a presently preferred embodiment, the analysis of the temporal pattern of cycle-to-cycle variability is performed on successive overlapping segments of data as the data is accumulated and the results of the analysis are displayed on each data segment as it is obtained (see Fig. 31).

Computing and displaying the results of the analysis in this way allows the operator to determine, while the data is being accumulated, whether sufficient data has been accumulated to determine whether a certain feature of waveform variability, such as alternans, is present, and to determine the magnitude of such variability as well as the level of statistical confidence of the measured variability. This enables the operator to determine in real-time whether sufficient data has been accumulated and the measurement can be stopped, or whether data collection needs to be continued, or whether the data is of deficient quality and the collection technique needs to be modified (e.g., one of the electrocardiogram electrodes is too noisy and needs to be replaced).

In order for a successful test for the presence of alternans be made in a patient, it is important that the operator have real-time or near real-time information available to be able to modify the parameters of the data recording while the data is being collected. Otherwise, a noisy electrode or prominent rhythmic artifact will often make the results of the test not interpretable. Such real-time or near real-time feedback enables the operator to modify the parameters of the data recording in order to make measurement of alternans a practical clinical test, particularly in the presence of physiologic stress.

In one preferred example involving the use of bicycle, treadmill or stair stepping exercise as the means of physiologic stress, one may measure variations in the patient's actual heart rate in real-time, compute the desired target pedal or step rate range, and control the step or pedal rate to stay in this range (e.g., by use of a metronome).

## V. Assessment of Statistical Significance of an Alternans Measurement

Cohen et al (U.S. Patent No. 4,802,491) report a method for analyzing the temporal pattern of variability in physiologic waveforms. In this method, a physiological signal is digitized and waveforms are identified using methods well known in the field. The waveforms are aligned using cross-correlation methods and a reference fiducial point is identified for each waveform. Each waveform may be labeled by an index $i$ and sample points within a waveform are labeled by an index $j$ representing the offset, $j \cdot \Delta$, from the fiducial point. Here $\Delta$ is the sampling interval. When multiple electrocardiographic signals are recorded simultaneously in the same subject, each lead may be similarly sampled, and sample points may be referenced. The sampled waveforms are analyzed to determine the level of alternans within a segment of the physiologic waveform. A power spectrum method is used to obtain three parameters: the energy (E) alternans at frequencies of 0.5 cycles/beat, the noise energy (E), and the standard deviation of the noise energy (S). These measures were combined to obtain an index of the level of alternans (e.g., the energy of the alternating component $A = E-N$ and a measure $V_{ALT}$ = the square root of A) or an index of the statistical significance of the alternans ($K = A/S$).

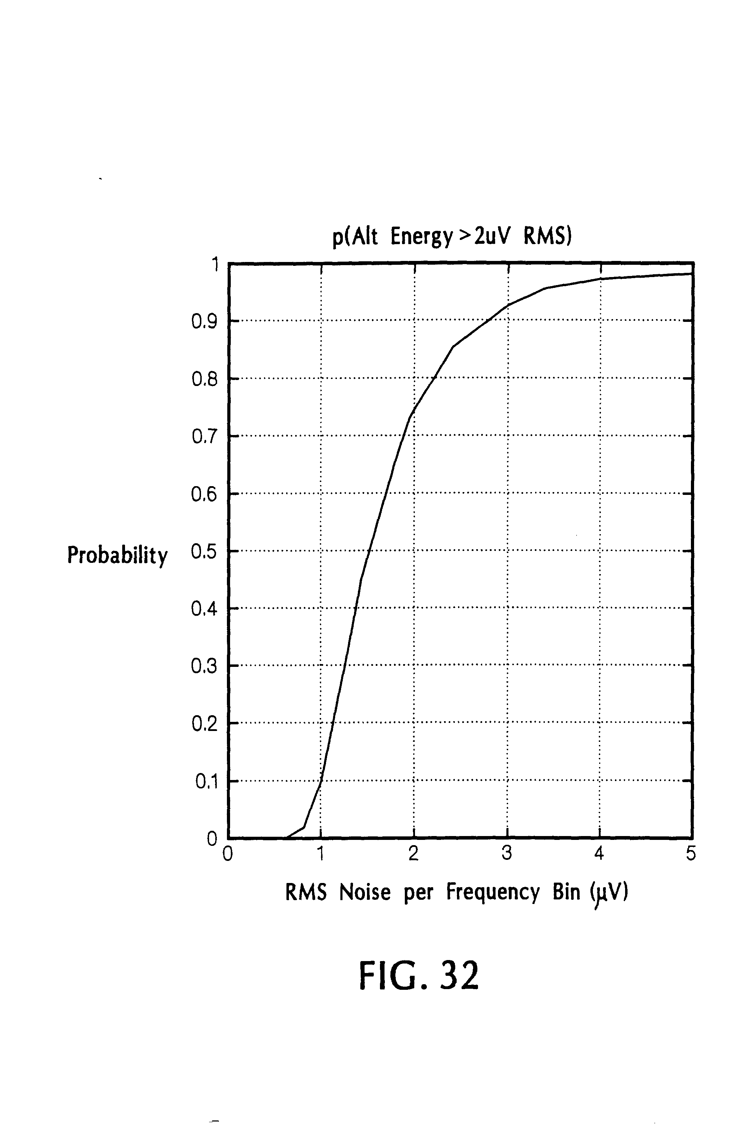

Fig. 32 shows a simulation of the probability that the RMS alternans energy will, purely because of random white noise, exceed a clinically significant threshold of $2_\mu$V RMS as a function of the noise level in the spectrum. (For this simulation, the alternans energy was measured using the method described in U.S. Patent No. 4,802,491, with four independent samples in the T-wave; however, measurement by the method of complex demodulation, 3.g., as described in U.S. Patent No. 5,148,812, would produce the same result.

Note that the probability of a false positive detection of alternans greater than $2_\mu$V increases dramatically in the range of the $1_\mu$V to $3_\mu$V noise typically seen in this measurements.

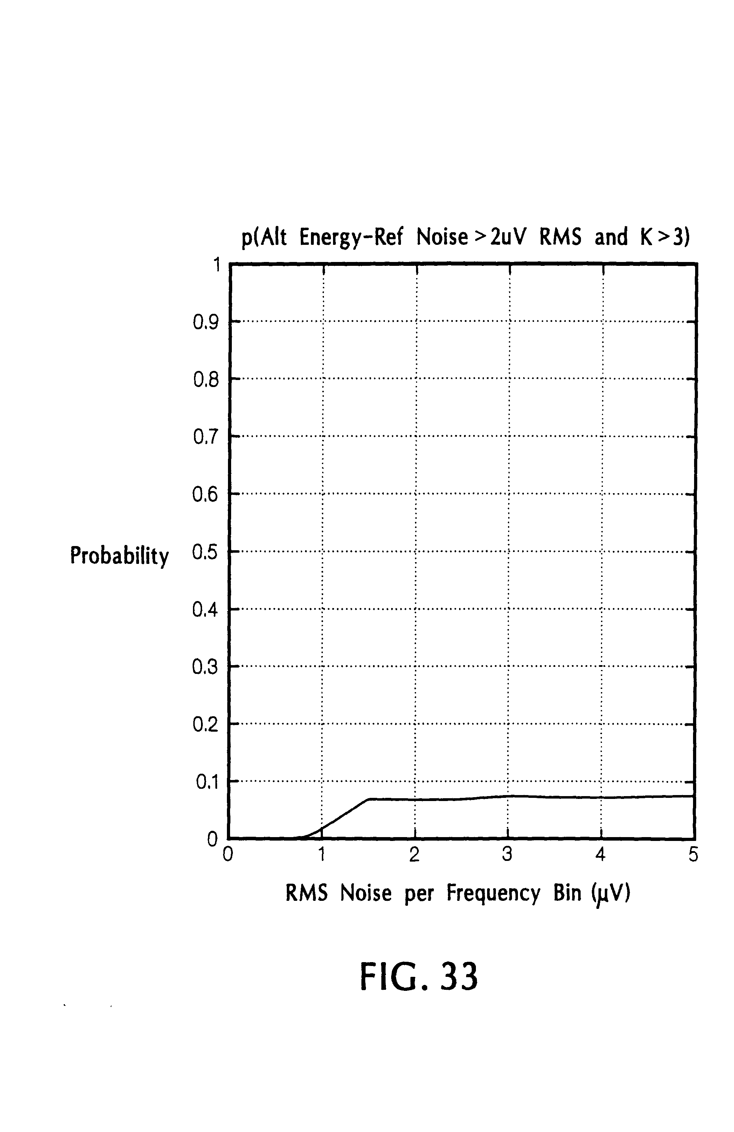

Fig. 33 shows how using a test that combines (1) the <u>difference</u> between the alternans energy and the reference noise band must be $>2_\mu$V and requires that (2) the ratio of this difference to the standard deviation of the noise (called the K score) must be greater than 3 reduces this false positive error rate at all noise levels.

Thus, we have realized that it is desirable to enable one to determine with statistical confidence whether the level of alternans exceeds some upper threshold or conversely whether the level of alternans is less than some lower threshold. This may be of importance if, e.g., it is demonstrated that a subject whose level of alternans exceeds an upper threshold has a high risk of ventricular arrhythmias and a patient whose level of alternans is less than some lower threshold has a low risk of alternans. In this embodiment of the invention, indices are created which enable one to determine with a specified level of statistical certainty whether the alternans level exceeds an upper threshold or is less than some lower threshold (the upper and lower thresholds need not necessarily be different). The outcome of the alternans determination is thus positive (alternans level with statistical confidence exceeds upper threshold), negative (alternans level with statistical confidence is less than lower threshold), or indeterminate (neither positive or negative).

For example, a test that alternans with statistical confidence exceeds an upper threshold level is whether A exceeds the upper threshold by a multiple of S. An example of a test that alternans with statistical confidence is less than a lower threshold is whether A is less than the lower threshold by a multiple of S. In each case, the multiple of S used determines the level of statistical confidence. Values of the multiple between 1 and 5 are conveniently used. A negative test indicating that alternans is not present may also require the heart rate exceeds a minimum level.

In one preferred example, assessment of the alternans pattern of cycle-to-cycle variation in physiologic waveform morphology is performed by comparing the level of the alternans in one portion of the electrocardiographic cycle to a noise level measured over a different portion of the electrocardiographic cycle. For example, the noise level may be computed over the PQ segment of the ECG. The PQ segment is a useful segment to analyze because normally there is little or no physiologic cardiac electrical activity during the PQ segment.

In another preferred example, the significance of a measure of alternans (such as the energy of the alternating component minus the energy of the noise) is determined by determining whether the measure both exceeds an upper threshold and also, with a specified level of statistical certainty, the measure exceeds zero (for example by being larger than a multiple of the standard deviation of the energy of the noise). Conversely, the significance of the alternans measurement is determined based on whether the measure is less than a lower threshold. The significance of the measure of alternans being less than a lower level may depend on a minimum heart rate being achieved.

In another example, the level of alternans (e.g., A) and the uncertainty of the alternans level (e.g., S) are computed for a measurement made in a certain subject. Then an empirical previously determined relationship between the level of alternans and probability of disease (e.g., risk of arrhythmias) is used (the relationship may incorporate the presence of known risk factors in the subject such as history of myocardial infarction and low ejection fraction). Probability of disease in this subject is then determined by integrating the probability distribution of the alternans level (defined in terms of the alternans level and uncertainty) over the empirical relationship between alternans level and risk of disease.

In one preferred example, the alternans test is rejected as indeterminate if the noise exceeds a threshold value. In another preferred embodiment, a positive test requires that an index of the alternans level (such as $V_{ALT}$) exceeds a threshold value and that an index of statistical significance (such as K) exceeds a threshold value.

In one preferred example, a test for susceptibility to ventricular arrhythmias is deemed positive if a significant level of alternans is measured during rest, during exercise which does not achieve the target heart rate, and/or during exercise where the target heart rate is reached. In this preferred example for the test to be deemed negative the target heart rate must be achieved and the criteria for a negative test must be met at this heart rate, and significant alternans must not be present at rest or at levels of exercise where the target heart rate was not achieved.

One advantage of these improved methods for determining the statistical significance of the level of alternans, is that if they are determined during, or shortly following, the data collection process one may determine whether the amount of data collected is sufficient to make a statistically confident prediction of disease risk in an individual or whether additional or less noisy data need to be collected. If sufficient data has been collected to make a determination, the data collection process may be stopped.

Example: Statistical Significance in Multiple Physiologic Signals

Utilization of the vector magnitude ECG signal allows one to detect an alternating component of the ECG parallel to the direction of the instantaneous cardiac electrical vector. The vector magnitude is the square root of the sum of the squares of the voltage signals in three orthogonal vector leads as is obtained in the Frank electrocardiographic lead system. Alternating components of the ECG perpendicular to the instantaneous cardiac vector will not be reflected in the vector magnitude signal. Similarly, alternating components of the ECG perpendicular to the vectorial direction of any single ECG lead will not be reflected in that single ECG lead. Therefore, it is advantageous to measure alternans in multiple leads. However, the levels of noise may be different in different ECG leads, and therefore a high level of alternans in one lead may be insignificant in a lead with a high noise level and a lower level of alternans in another lead may be statistically significant in another lead with a lower noise level. In one preferred embodiment, the level of alternans (such as $V_{ALT}$) and a measure of the statistical significance of the alternans (such as K) is measured in multiple physiologic signals, for example in multiple ECG leads, and this information is combined to assess the level and presence of alternans in the subject.

For example, to assess alternans during exercise one could measure $V_{ALT}$ in the three orthogonal leads of the vectorcardiogram ($V_X$, $V_Y$, $V_Z$) also measure K in each lead. A positive alternans test would require that in one or more leads $V_{ALT}$ be greater than 1.9 microvolts and that K exceed 3 in the corresponding lead.

VI. Data Epoch Selection

As discussed above, a number of factors can interfere with the ability to reliably assess the level and significance of a pattern of cycle to cycle variation in physiologic waveforms, such as alternans, particularly during the application of a physiologic stress. Any measurement of alternans can be corrupted by noise which just happens to have significant energy at half the heart rate and thereby causes a false positive alternans result. Therefore, if one were to look for alternans continuously in a long data record one would be likely to eventually have a positive result irrespective of the patient's susceptibility to ventricular arrhythmias. Similarly, if one were to analyze only a single segment of data and that segment were to contain significant noise or premature beats or to be at too low a heart rate, then the alternans result may be falsely negative. Therefore a means needs to be developed to decide which segments are most suitable for analysis in order to provide the most accurate result. In one preferred embodiment, an epoch or epochs of data are chosen for analysis of the variation by considering measurements made in each epoch including one or more of the following: number or rate of premature or ectopic beats, the number of ectopic beats occurring in central portion of the epoch, a noise metric, a noise metric measured over a particular frequency band, a measure of the amplitude of the temporal pattern being assessed, a measure of the relative amplitudes of the temporal pattern being assessed and the noise metric, a measure of the statistical significance of a temporal pattern of cycle-to-cycle variation, the variability of some portion of the electrocardiographic signal, respiratory rate, heart rate, the patient's pedal or step rate, and/or interbeat interval variability.

As seen in Fig. 33, even when a combined test for level of alternans and for statistical significance is used as described herein, there is a significant probability of a false positive result each time an alternans measurement is made and this probability is a function of the noise level. As a result of this, the more times one looks for alternans, the more probable is a false positive result. If one were to continuously measure for the presence of alternans and to declare the test positive if a specific level of alternans is ever found, then the probability of a false positive result would approach 1.

To prevent this, it is important to minimize the number of independent times that one looks for alternans and to look only in data segments where the level of alternans is expected to be high and the level of interfering noise is low.

Thus, by considering such factors in epoch selection one can reduce the influence the factors which cause interference with the measurement of a particular pattern of cycle-to cycle variability, select the segments where the amplitude of the temporal pattern being assessed is largest and most significant, and select the segments which are most likely to result in the temporal pattern of variability being assessed being detected. For example, epochs with few or no abnormal premature beats or none near the center of the epoch will suffer least from the effects of phase resetting. Epochs with a low level of noise are most likely to reveal the temporal pattern of variability being assessed. This noise can be measured, for example, by considering the variability in the PQ segment of the electrocardiogram; the PQ segment is a useful segment to analyze because normally there is little or no physiologic cardiac electrical activity during the PQ segment. The noise can also be measured in one or more reference frequency bands (see, e.g., Cohen et al., cited above).

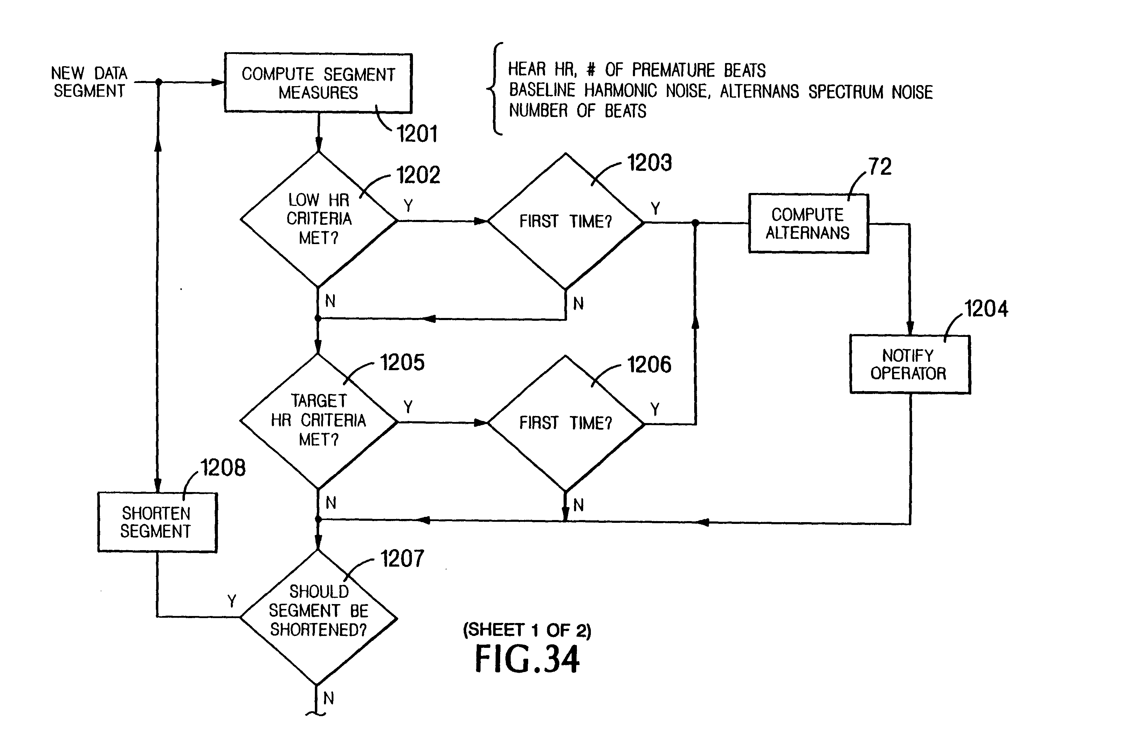

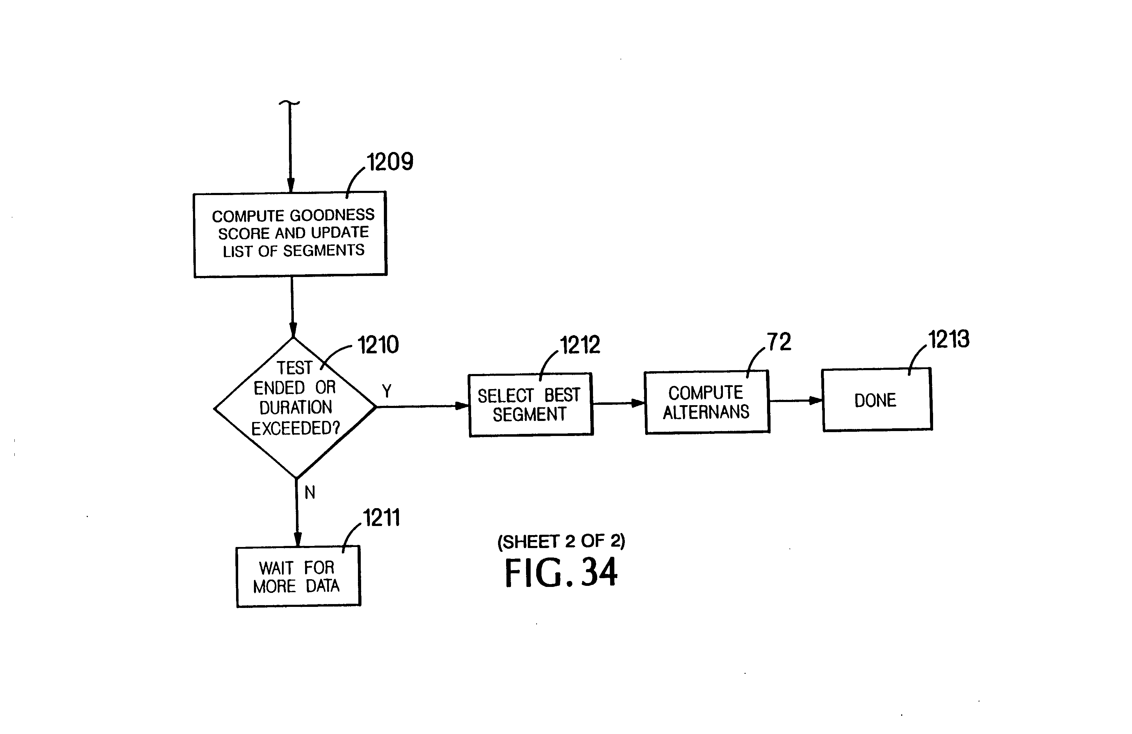

Referring to Fig. 34, in a presently preferred epoch selection scheme, the segment finder maintains a list of segments ranked by their "goodness" which ranges from 0 to 10. In step 1201 a candidate segment is taken from the beat matrix 70 as the next eligible segment from a list of beats. Segments are allowed to begin only on beats whose number is a multiple of an increment of 8 beats. The segment is expected to be 128 beats long; however, if fewer than 128 beats are available, the shorter segment is used if it meets the minimum segment length requirements described in step 1207 below. Since the segment increment of 8 is much smaller than the target number of beats, the segment under consideration overlaps with previous segments. The preferred embodiment limits the possible lengths and origins of the segments for computational simplicity. Other embodiments may implement methods that consider a broader range of segment origins and lengths.

Step 1201 computes the metrics used to create the "goodness" score for the segment under consideration. These metrics include the mean heart rate, the prematurity metrics, noise metrics, and the number of beats. The noise metrics include the baseline harmonic noise and alternans spectrum reference noise. The baseline harmonic noise is computed from the power of the spectrum of PQ levels at 0.167 and 0.25 cycles/ beat. These noise frequencies are of interest because their 3rd and 2nd harmonics, respectively, fall at the alternans frequency of 0.5 cycles/beat. The alternans spectrum reference noise band is computed to approximate the measure N as defined in the introduction. The alternans spectrum reference noise power is also computed from noise-optimized ECG leads. The prematurity metrics include the total number of premature and postmature beats in the entire segment and in the middle half of the segment. Premature beats can reset the alternans, causing the measured alternans level to be lower. The closer that the premature beats fall to the center of the segment, the greater the potential cancellation of the alternans signal due to phase resetting.

Alternate examples may use a variety of other metrics derived from or related to the input signals. The metrics may include the total heart rate variability or heart rate variability in specific frequency bands, or other measures related to the alternans modulation. The metrics may derived from respiration and motion measures, where the measures are estimated from the ECG or measured by some other means. The metrics may include a host of noise measures derived from single beats or beats taken in groups.

Step 1202 tests whether the segment meets the primary criteria for a low heart rate. The low HR primary criteria are: a heart rate of 90-100 beats per minute, no premature beats, baseline harmonic noise less than $2_\mu$V, reference band noise less than 1.2 µV, and at least 100 beats. The first time that the low heart rate criteria are met, the alternans result for that segment is computed as specified in step 72 and the operator is notified of the result in step 1204. Step 1205 tests whether the segment meets the primary criteria for the target heart rate. These criteria are the same as for a low heart rate segment, except that the heart rate range must be 100-120 beats per minute. The first time the target heart rate criteria are met in step 1206, the alternans result for that segment is computed as specified in step 72 and the operator is notified of the result in step 1204.

Step 1207 decides whether a shortened segment should be considered. A shorter segment is created for consideration if the target heart rate criteria have not been met and if the resulting segment is at least 64 beats long. The segment is shortened in step 1208 by removing from the end of the segment the number of beats specified by the increment value and the segment is then returned to step 1201. Other embodiments remove beats based on the significance of the change in one or more of the metrics occurring at the specific beats.

In step 1209 the goodness measure of the segment is computed using a piecewise linear function of the measures. Step 1210 checks to see if there is no more ECG data available, or if the test should be terminated because a pre-specified duration has been exceeded. If the test is to continue, the segment finder waits at step 1211. If the test is completed, the segment with the greatest "goodness" is selected from the segment list in step 1212 and supplied to step 72 to compute the alternans measures. The resulting segment and alternans information is returned in step 1213.

Other examples may choose to consider several segments simultaneously. The segments may be handled separately, with separate metrics and alternans results computed for each segment and combined by a weighted average, or the segments may be joined into one longer segment. One possible embodiment is to join segments of beats that fall between premature beats. The segments must be joined with proper consideration to whether the first beat in each segment is a even or odd number of beats after the premature beat.

Another example further reduces the false positive rate by analyzing multiple independent epochs and analyzing the consistency of the result; i.e., requiring that the test be positive in more than one epoch. This resultant false positive rate will be significantly reduced.

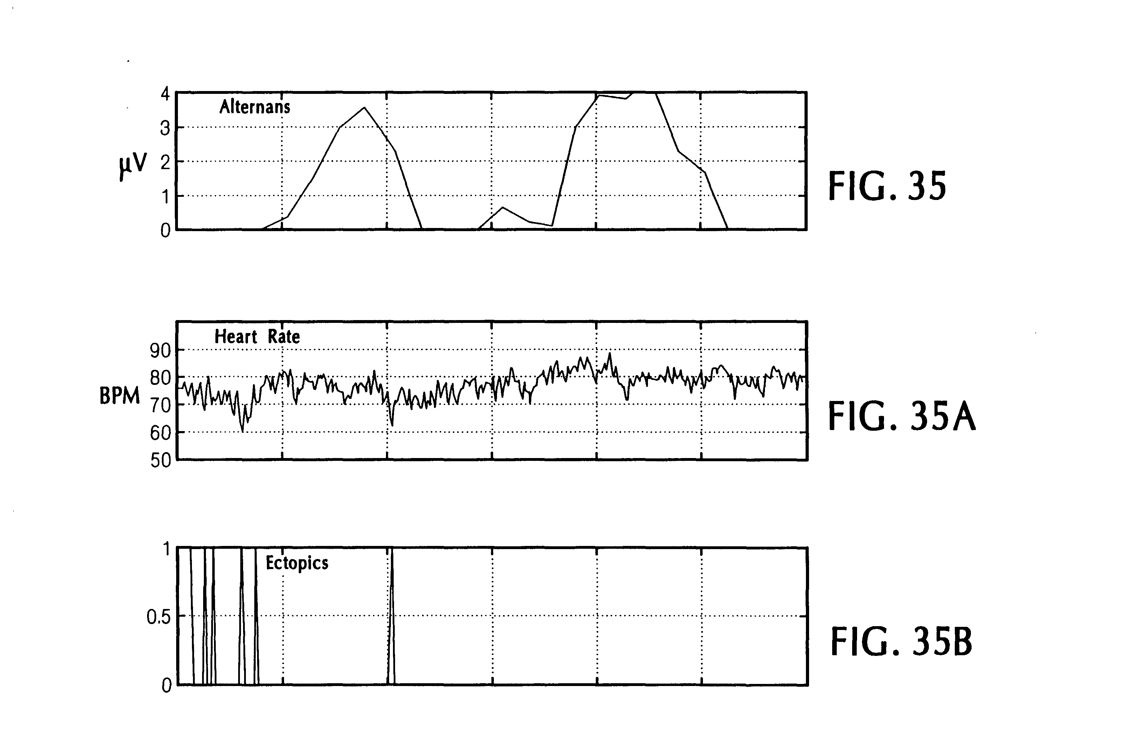

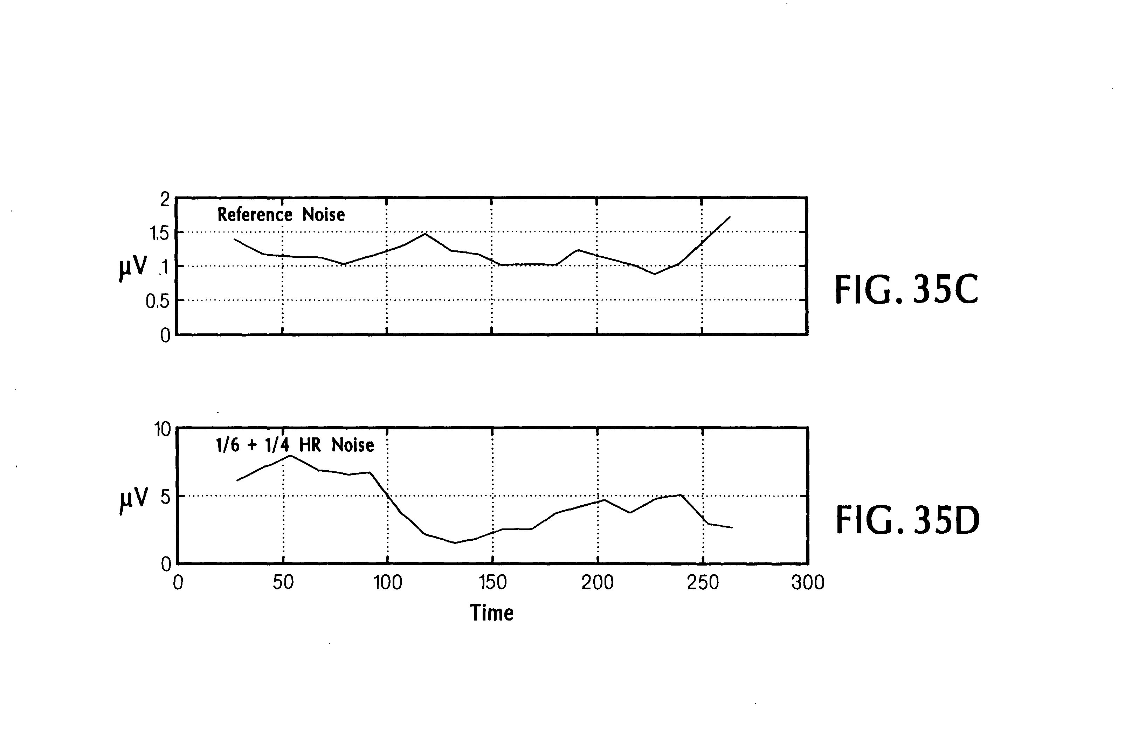

Another example involves outputting the variables used in the epoch selection process and, in effect, allow the operator to select the appropriate epoch and analyze the consistency of the result. Figs. 35-35D show a graph which indicates over time the level of alternans, a flag for ectopic beats, the heart rate the level of overall noise, and the level of noise and respiration occurring at 1/4 and 1/6 of the heart rate. From such an output, the operator can look for appropriate epochs, selectively ignore epochs where there is a high level of interfering noise and determine the consistency of result.

An alternative approach to reducing the effects of respiratory activity on the assessment of an alternans pattern of cycle-to-cycle variability in physiologic waveforms involves monitoring respiration and causing the patient to alter his respiratory rate when this rate is at a sub-multiple of the heart rate. A further approach to reducing the effects of respiratory activity on the assessment of an alternans pattern of cycle-to-cycle variability in physiologic waveforms involves measuring and excluding from the analysis those sections of data which correspond to periods during which respiration is at an even sub-multiple (such as one quarter or one sixth) of the heart rate or where the energy of all potentially interfering signals such as respiration is high at such sub-multiple frequencies.

It should be understood that the concept of epoch selection includes any of a variety of possible means of selecting or emphasizing portions of the recorded data. For example, rather than selecting one or more specific epochs for analysis, the alternans analysis might be performed over the entire data record and a weighing function used to emphasize certain portions of the data based on variables such as mean heart rate, frequency of premature or other abnormal beats, noise, etc. Or, for example, one might display in graphical form variables such as alternans level, noise level, measures of statistical significance of alternans, mean heart rate etc., over a long data epoch so that the operator may choose the portion of the data which is most significant for the interpretation of the alternans measurement.

VII. Applications

It may be advantageous to combine alternans measurement with other diagnostic tests particularly those used for cardiovascular diagnostics. The combinations of alternans measurement with these other tests provides unexpected advantages in terms of diagnostic capability and in terms of dual use and cost effectiveness of diagnostic equipment.

A. Combination of An Alternans Test System with a Standard Exercise Stress Test System

The techniques described above allow an alternans test system to be combined with an exercise stress test system. This combination is particularly advantageous because exercise stress tests, which are given to measure cardiac ischemia caused by the presence of coronary artery disease, are often given to the same patients in whom one would like to measure alternans to determine the risk of ventricular arrhythmia's. Furthermore, in some patients where one would like to make both tests, such as in patients who have recently suffered a heart attack, there is some risk associated with exercise and it is advantageous not to have to exercise the patient twice.

In another preferred example, one analyzes the temporal pattern of cycle-to-cycle variability in a physiologic waveform both at rest and during physiologic stress. The temporal pattern of cycle-to-cycle variability in waveform morphology may or may not be present in any patient at rest but there may also may be less physiologic noise as compared with the data collected during physiologic stress. By combining analyses of data at rest and during physiologic stress a more reliable assessment of the presence and magnitude of the temporal pattern of cycle-to-cycle variability in physiologic waveforms may be obtained. Furthermore, some patients cannot exercise sufficiently to achieve the minimum target heart rate of approximately 100 beats per minute. A measurement made at rest and during reduced levels of exercise which results in a significant level of alternans, would indicate that the patient may be at risk of ventricular arrhythmias. In the absence of such measurements no alternans based information on the patient's risk would be available. Of course, under circumstances where the patient cannot achieve the minimum target heart rate one could not definitively determine that the patient does not have inducible alternans.

Data may be collected during a standard bicycle exercise stress test used for other purposes such as the detection of coronary artery disease, and the analysis process analyzes the data which falls within the specified limits of heart rate and pedal rate if the apparatus has means to measure pedal rate as well as heart rate. Data may be collected during a standard bicycle, treadmill, stair stepping or other exercise stress test conducted for purposes other than assessment of alternans, for example for the purpose of detecting coronary artery disease, and the data segments analyzed for alternans are those in which the step and target heart rates fall within the target ranges.

Standard exercise stress tests generally involve multiple stages described as the Rest Stage, Exercise Stages 1 through N, and the Recovery Stage. Each of the stages has particular advantages for the measurement of alternans.

The Rest Stage has the advantage of low noise but, because the heart rate is not elevated, the incidence of alternans during rest is low. Therefore a positive alternans measurement during rest can be considered a positive result, but the absence of alternans cannot be considered a negative result. However, since some percentage of patients will not be able to achieve a sufficiently high heart rate during exercise and in others the level of premature beats or artifact will make the measurement during exercise impossible, it is worthwhile to measure during rest.

The Recovery Stage which occurs after the cessation of exercise has some advantages as well. The heart rate will still be elevated but the artifact level due to exercise will be reduced. Unfortunately, the heart rate will be falling rapidly and the mean heart rate level for the segment of data may be too low to insure a negative test. In a preferred example measurements during all three periods (Rest, Exercise and Recovery) are used and may include pharmacological stress. The test is considered positive and can be terminated if there was statistical level of alternans in any lead at rest, during exercise or during recovery. The test is considered negative only if there was an acceptable epoch selected with a mean heart rate over the target (generally 100 beats per minute) and the noise measures were low enough to have a statistically significant negative result. Tests which are neither positive or negative are indeterminate and call for further testing. This can mean the addition of a pharmacological stress agent to help reach the target heart rate or the addition of a final exercise stage using a different form of exercise which may cause lower levels of artifact (such as adding a bicycle stage after a treadmill test).

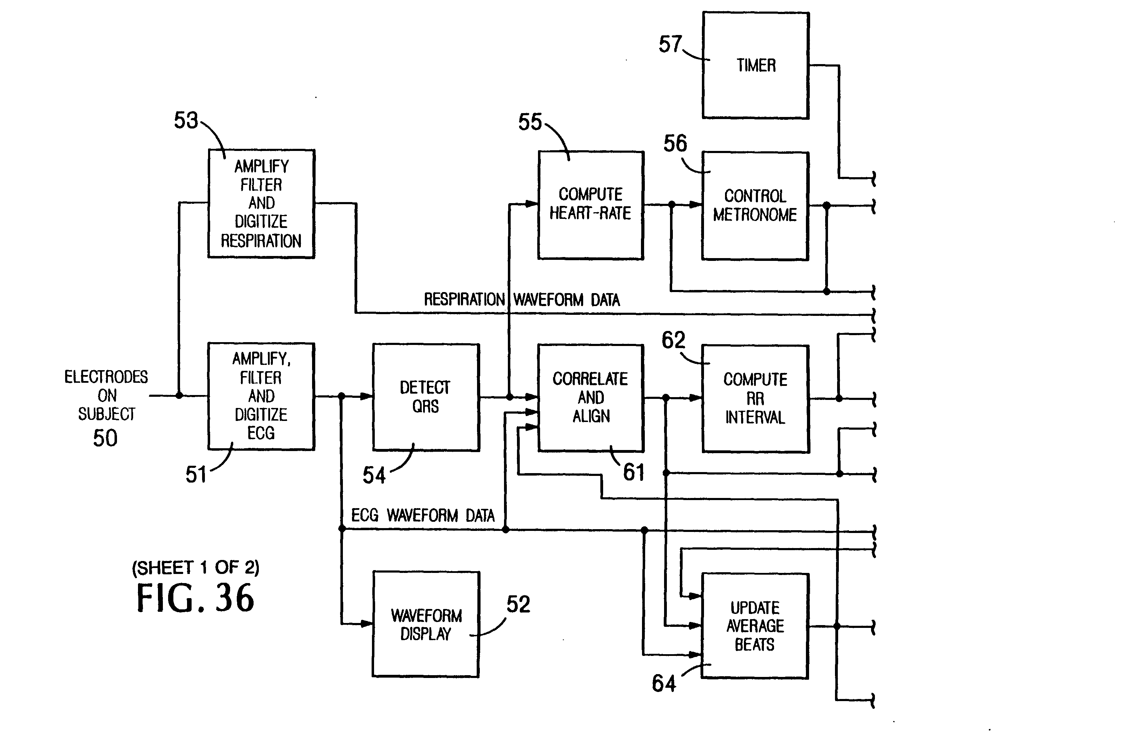

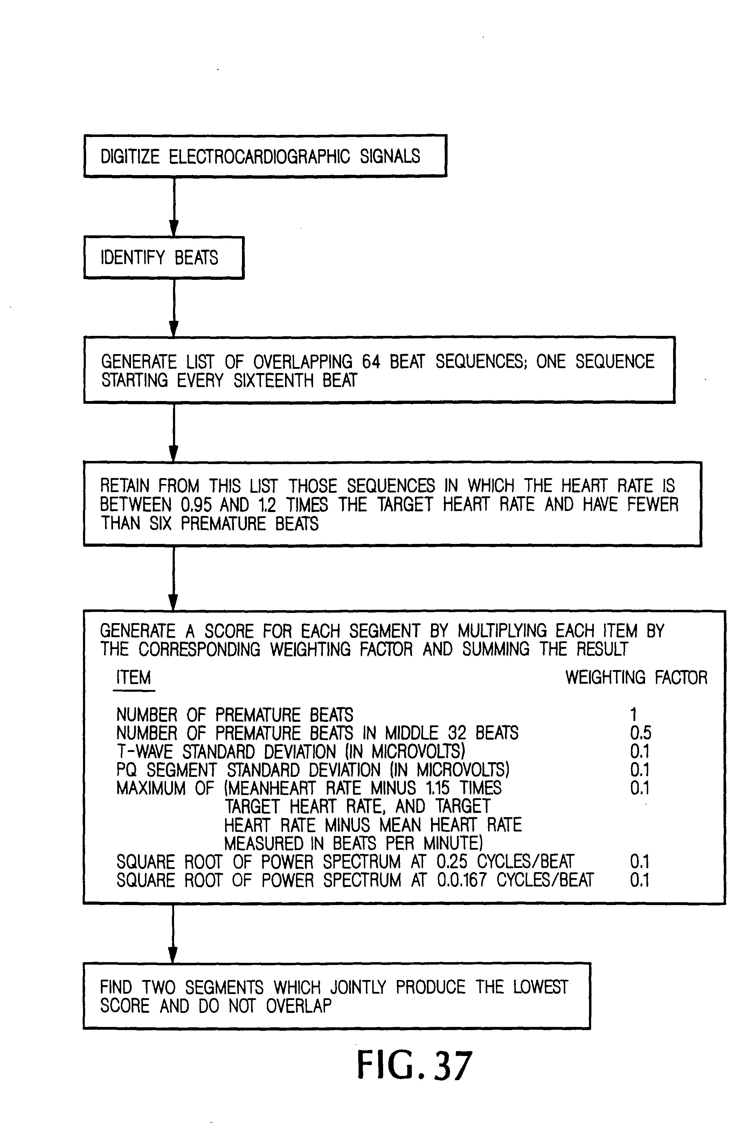

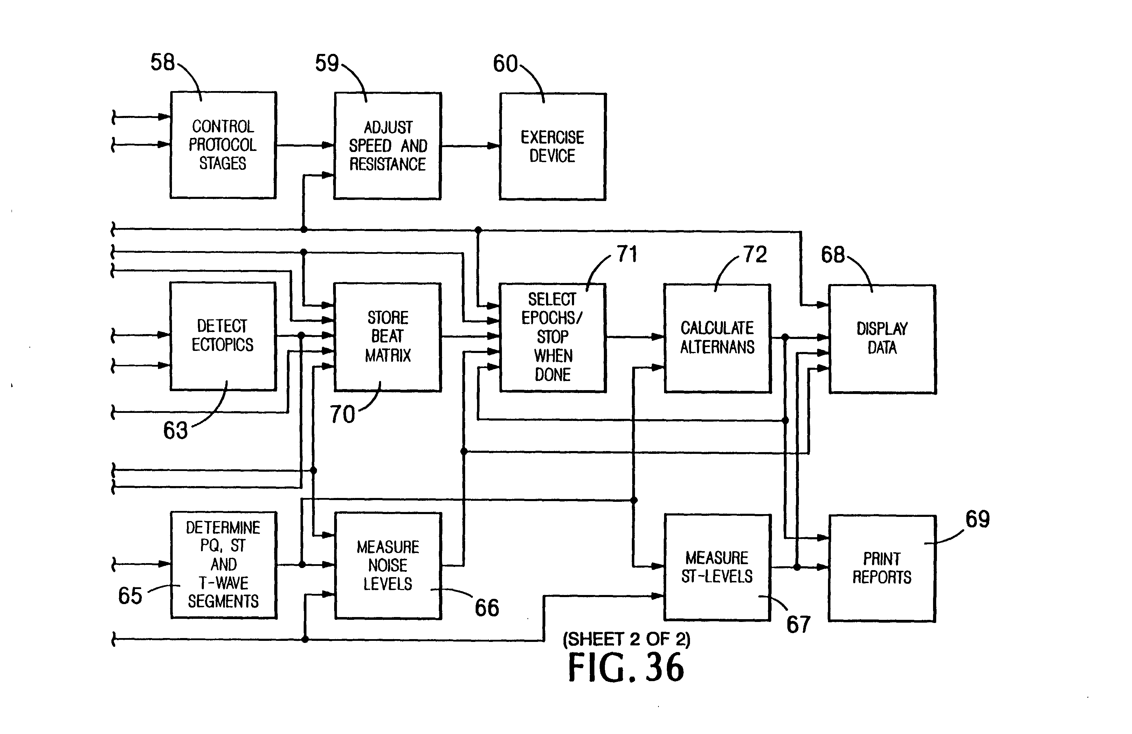

Fig. 36 contains the block diagram for a combined Exercise Stress Test and Alternans System. The subject has ECG electrodes 50 applied at the standard positions used for collecting standard 12 lead ECG data as well as at the positions used for Frank X, Y, Z lead ECG data. The ECG waveforms from the electrodes are amplified, filtered at 0.05 to 150 Hertz and digitized at 1000 Hertz using standard ECG amplifier, bandpass filter and 12 bit A to D converter techniques 51 and the ECG waveform data is displayed 52 for the operator. At the same time a respiration waveform is determined from two of the electrodes on the right and left side of the chest using impedance plethysmography techniques well known in the art and also amplified, filtered at 0.05 to 150 Hertz and digitized (53) at 1000 Hertz.

QRS complexes are detected (54) from the digitized ECG waveform data using standard techniques well known in the art. The time of the detected QRS complexes is used to compute heart rate 55 using standard techniques and this heart rate is used to control a metronome 56 which sounds at a rate of 1/3 or 2/3 (operator selected) of the computed heart rate in the manner described earlier. This metronome is used by the subject to maintain a step rate or pedal rate on the treadmill or bicycle exercise device 60 so that the rhythmic artifact from the exercise does not interfere with the alternans measurement.

At the same time a timer 57 is used to control (58) the various stages of standard exercise protocols. These protocols generally call for a Rest Stage followed by timed Exercise Stages, often three minutes each in length where the level of exercise is increased at each stage. This increase is effected by adjusting (59) the speed and incline of a treadmill or the resistance of the bicycle exercise device (60). In addition, the protocol control function (58) continually monitors the computed heart rate and prompts the operator to terminate the exercise when a target heart rate specific to the type of protocol is achieved. When exercise is terminated for this or other reason (such as fatigue of the patient, chest pain, or excessive ST segment level depression), the protocol control function (58) starts the Recovery Stage, where the patient is allowed to rest while continually being monitored by the system.

The above paragraph describes the protocol control function where it is desired to measure alternans during a standard exercise protocol designed for the detection of coronary artery disease. However, in some cases it may be desired only to measure alternans in which case a protocol optimized for alternans measurement is used. This protocol calls for a Rest Stage followed by a level of exercise which is adjusted (59) based upon the computed heart rate (from 55) to cause the subject's heart rate to achieve and maintain an optimal level for alternans measurement (generally 100 to 120 beats per minute). This level is maintained until sufficient data is collected for a statistically significant alternans computation to be completed.

While the subject is progressing through the stages of the exercise protocol the system measures alternans and computes ST segment levels as described below.

The detected QRS complexes (from 54) are cross correlated (61) with average beat templates (produced by 56) and aligned with these templates at the time of the point of maximum correlation to produce an adjusted time of the QRS which is more stabile and resistant to noise. This adjusted time of the QRS is used to compute the R to R (i. e., RR) intervals (62). These RR intervals and the correlation coefficients (from 61) are used to detect ectopic beats (63). An ectopic beat is defined here as one which has a correlation coefficient with the average beat template of less than 0.9 or whose previous RR interval is less than 90% of an 8-beat running average RR interval.

The average beat templates (one template for each ECG lead) are updated (64) by taking the ECG waveform data surrounding the detected and aligned QRS complex and computing a point by point 8 beat running average of ECG waveform data with the stored average beat templates. This function (62) also uses ectopic detection (63) to reject and not average in to the average beat templates any beat determined to be ectopic.

The average beat templates are analyzed (65) to determine the PQ segment, the junction point, the ST-measurement point and the ST-T wave segment using techniques described below. The PQ segment is determined by computing the Absolute Value Vector Velocity (ASVV) from the sum of the absolute value of the derivative of the X lead, the Y lead and the Z lead and looking for a minimum in a 100 millisecond region preceding the QRS complex. The J point is determined as the point after the QRS where the ASVV falls to 7.5% of its QRS peak. The ST measurement point is defined as 80 milliseconds past the J point. The ST-T wave segment is defined for the purposes here as extending from 60 milliseconds past the J point to 20 milliseconds past the point where the ASW false to 7.5% of its peak in the ST-T wave segment.

The segments determined (by 65) are used to measure noise levels 66 during the PQ and ST-T wave segments by computing the point by point standard deviation of the ECG waveform data for each lead from the means obtained from the average beat templates over the points in these two segments. These measures are displayed for each lead (68) in a bar graph format so that the operator can see if the noise level is too high for the measurement of alternans and take appropriate action such as improving electrode contact.

The ST measurement point is used to measure the ST-Levels for each ECG lead (67). These levels constitute the primary output from a standard stress test and are displayed to the operator (68) and printed in reports 69.

At the same time the times of the aligned QRS complexes are used to trigger the storage of another beat in the beat matrix (70). This matrix contains multiple rows of one beat each aligned on the QRS complexes as described in U.S. Patent No. 4,802,491 cited above. However, instead of just storing the vector magnitude waveform for each beat, all of the leads are stored along with simultaneous respiration data (from 53), the previous RR interval (from 62) and the results of the ectopic detection (from 63). In addition, in recognition that the alternans energy is generally low frequency in nature and to save storage, the ECG waveform data is digitally lowpass filtered at 25 Hertz and subsampled at 50 Hertz before storage in the beat matrix.

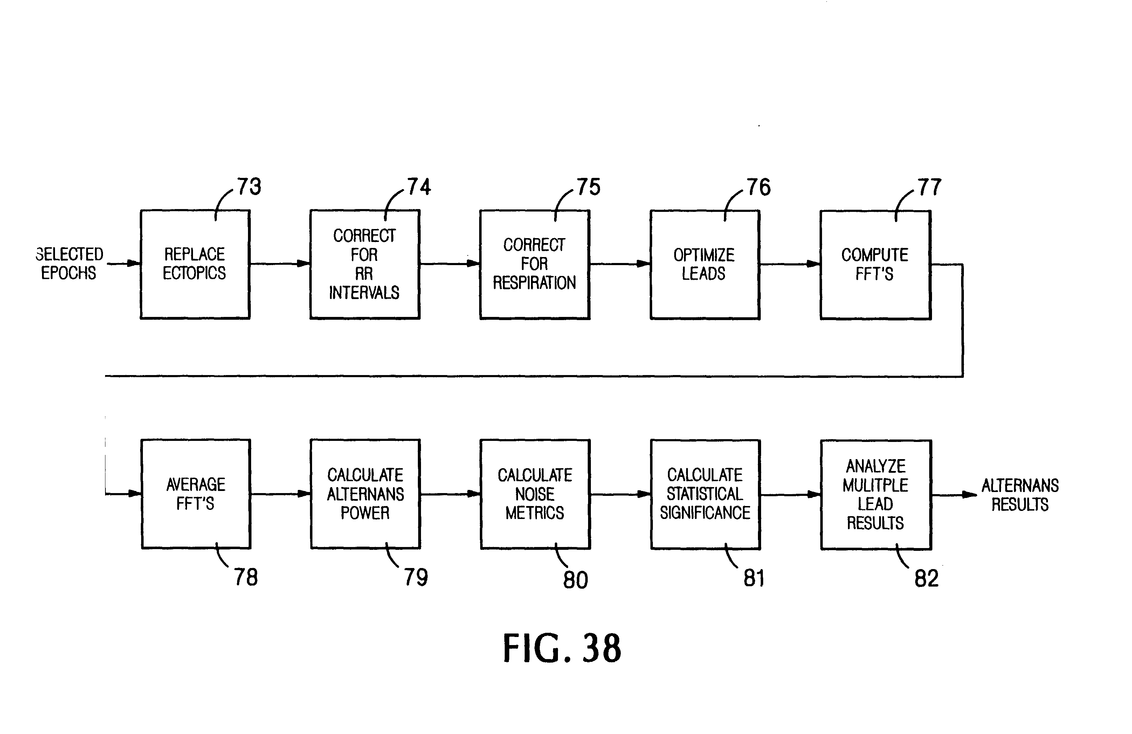

The Select Epochs function (71) uses the heart rate (from 55), the number of ectopic (from 63), and the noise levels (from 66) to determine appropriate segments for analysis. This function is described in the flow chart in Fig. 37 and in detail in the earlier Signal Processing section. The Select Epochs function also uses the alternans measurements (from 72) to determine when there has been a statistically significant alternans result and alternans processing can stop.

The selected epochs are passed to the Calculate Alternans function (72) which calculates the alternans measurements in real time, determines their significance and displays them to the operator (68) and for print-out (69).

The Calculate Alternans function is described in more detail in Fig. 38. First the ectopic detection data is used to replace any ectopic beats with the mean computed over the selected epoch. Then (74) the data in each lead is corrected for RR interval variations over the ST-T wave segments using the techniques described earlier. Then the data is corrected for respiration effects (75). Typically, these two steps are performed simultaneously. Then the optimum (lowest noise) X, Y, and Z leads are computed (76) from the multi-lead ECG data using the methods described in U. S. application Serial No. 08/339,032, filed November 14, 1994, cited earlier. In addition, an optimized V lead, similar to V4 is computed using the same techniques. Then fast Fourier transform spectra (FFT's) are computed (77) for each epoch, for each of X, Y, Z, and V leads and for each point in the ST-T wave segments and these spectra are averaged (78) to form a composite spectra for each of X, Y, Z and v leads. Then the alternans power is computed (79) from the last point in the averaged spectra. The noise mean and standard deviations for the spectra are computed from an adjacent noise band covering as described in Smith et al, 1988, cited earlier. Then the statistical significance of the alternans voltage is calculated (81) as described earlier in the Signal Processing section. Finally, the results in the multiple leads are analyzed in (82) to find the highest level of alternans with a specified level of statistical significance and this result is then output for display and printing.

C. <u>Combination of An Alternans Test System with a Standard ECG Cart</u>

In a preferred example of the use of the apparatus of this invention, a system for measuring alternans is combined with a standard ECG cart. This is a particularly favored combination because it enables one to combine measurement of alternans which is an accurate predictor of the risk of ventricular arrhythmias, with other diagnostic tests that can be performed with an ECG cart. The standard ECG cart is usually used to record and analyze the standard 12 lead electrocardiogram. This test provides a wide range of diagnostic information on the electrical conduction pattern in the heart. The standard ECG, however, provides little information on the risk of future life-threatening ventricular arrhythmias. Some ECG carts have the capability to compute the Signal Average Electrocardiogram (SAECG). The SAECG is obtained by averaging many repetitive ECG waveforms. It has been shown that 'late potentials' which occur at the end of the QRS complex and may be detected in the SAECG may provide some information on the risk of ventricular arrhythmias. We have found that alternans is a much more accurate predictor of ventricular arrhythmias than the SAECG, however, combining SAECG and alternans measurements may provide even better accuracy than either test alone. Further, an ECG cart may be adapted to measure beat-to-beat variability in the QRS complex in a multiplicity of leads. Increased QRS variance (see U.S. Patent No. 5,188,116) has been shown to be an accurate indicator of the presence of coronary artery disease even when the measurement is made without the use of physiologic stress. Further, an ECG cart may be adapted to measure other physiologic signals and perform Physiologic System Identification (see Cohen et al., U.S. Patent No. 4,930,517) to accurately assess autonomic nervous system activity and cardiovascular reflexes. Physiologic System Identification involves the mathematic analysis of the coupling between fluctuations in different physiologic signals to create an individualized physiologic model of closed loop physiologic function. Thus, by combining an ECG cart with an alternans system, a multiplicity of very important and complementary diagnostic tests can be performed with a single system.

It is further advantageous to combine the ECG recording device with an alternans measurement system because much of the same hardware can be used for both systems. For example, the most recording electrodes can be used for both standard ECG analysis as well as alternans recording; the same ECG amplifier and computer system can be used both for the standard ECG analysis and for the alternans measurement. Furthermore, many of the signal processing features used for alternans analysis can also be applied to the other diagnostic functions of the ECG cart (standard 12 lead ECG measurement and analysis, SAECG and/or QRS variance analysis) for example, detection of abnormal beats, adjustment of ECG waveforms to compensate for the effects of interfering signals, epoch selection, noise reduction, etc.

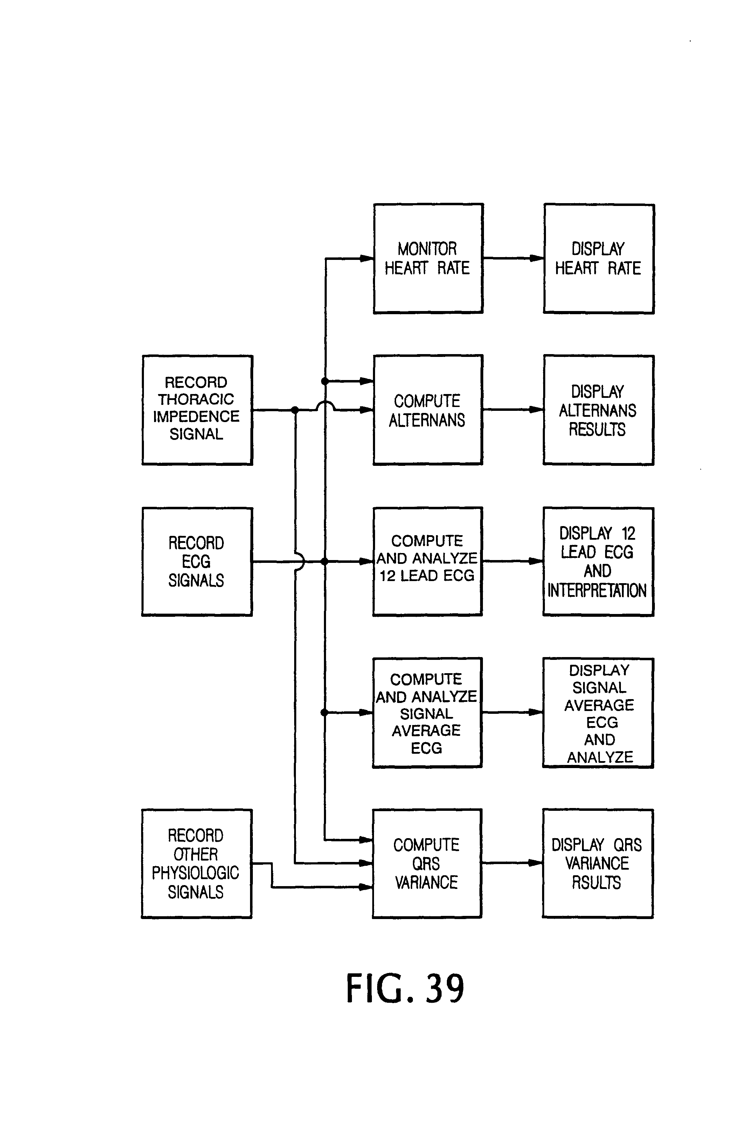

Fig. 39 is a block diagram figure of a combined ECG Cart and Alternans System. In this figure, ECG signals are recorded from body surface electrodes. From the ECG signals the heart rate is monitored. Also, an impedance signal may be measured from the ECG electrodes to monitor respiratory activity. The ECG signals are used to compute alternans in conjunction with the monitored heart rate and impedance signals according to the method of the invention taught above. The alternans measurements are computed and displayed. The ECG signals are used to compute and analyze the 12 lead ECG in any of the manners well known in the art and the 12 lead ECG and its interpretation is displayed. The ECG signals are used to compute and analyze the Signal Averaged ECG in any of the manners well known in the art and the Signal Averaged ECG and its analysis is displayed. The ECG signals are used to the QRS variance in any of the manners well known in the art and the QRS variance results are displayed. The ECG signals are used in conjunction with the impedance signal, the heart rate signal, and other physiologic signals such as non-invasively recorded arterial blood pressure to compute Physiologic System Identification results and the results are displayed.

D. Combination of An Alternans Test System with an Ambulatory ECG (Holter) System

Another example of the use of the apparatus of the invention, involves detection of alternans during naturally occurring physiologic stresses. This example involves recording physiologic signals and determining the presence of physiologic stress by means of a monitored physiologic variable such as heart rate. For example, electrodes may be applied and electrocardiographic signals may be recorded on a portable recorder known in the art as a Holter Monitor. The heart rate may be determined from analysis of the electrocardiographic signals. Periods of naturally occurring stress during the recording period, which may be 24 hours in duration, may be identified as the periods during which the heart rate exceeds some threshold value such as 90 beats per minute. Naturally occurring stresses may result from exercise, emotional stress, bowel movements, sexual activity and the like. The physiologic signals may be analyzed during the periods of naturally occurring stress for the presence of alternans.

One way of measuring cardiac ischemia is by measuring shifts in the ST segment of the electrocardiogram. The presence of myocardial ischemia may alter the interpretation of an alternans pattern of cycle-to-cycle variability in physiologic waveform morphology. For example, the presence of alternans in association with the development of myocardial ischemia may not necessarily indicate that the patient is at risk of ventricular arrhythmias when ischemia is not provoked by a similar stress. Therefore, combining the two systems has further advantages.

There are other advantages to combining an alternans measurement system with a Holter system. These systems are used to determine if a patient is experiencing ventricular or atrial arrhythmia's during a normal 24 hour period. They are often used for patients complaining of fainting spells where such fainting is suspected to be of cardiac origin. These same patients are candidates for alternans testing (the ventricular tachycardia predicted by alternans test being one possible cause of the fainting) and so a combined test is advantageous. Furthermore, the patient can be instructed to be active during the 24 hour period and thereby increase the chance that physiological stress will induce alternans.

One problem with such a combined system is that in a 24 hour period, there is a significant likelihood of finding some artifact occurring at 0.5 of the heart rate and therefore causing a false positive alternans result. As a result the noise measurement, epoch selection, noise reduction, statistical significance and other teachings of this patent become very important.

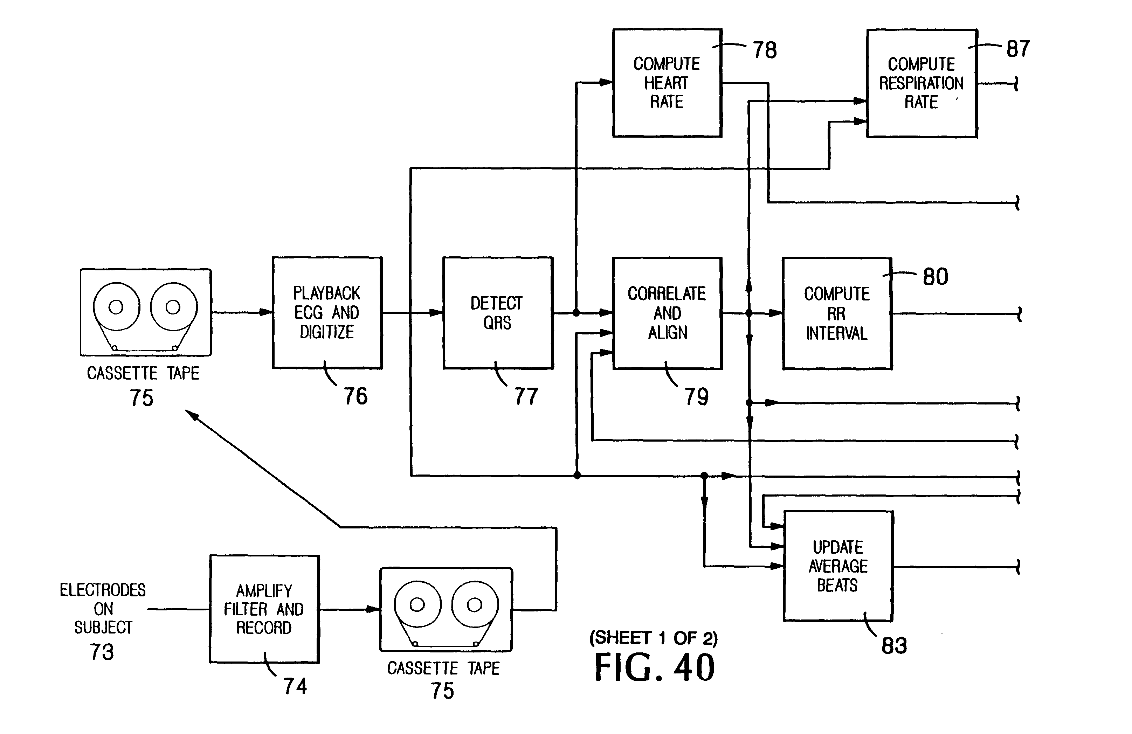

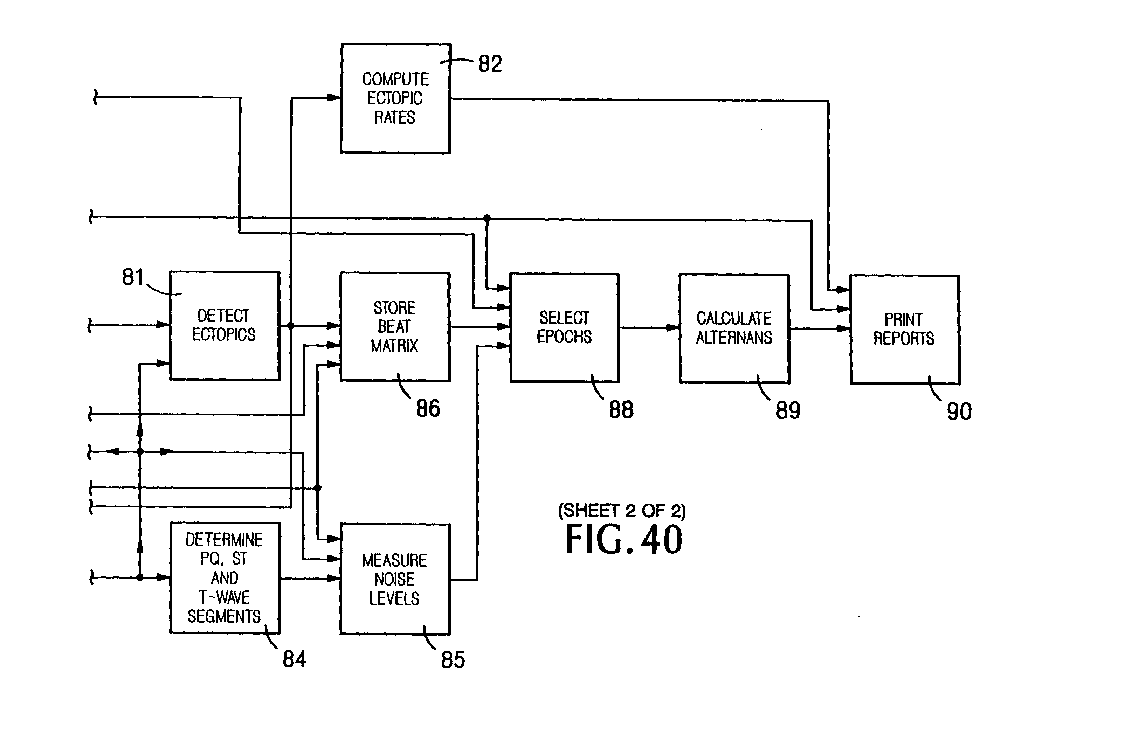

Fig. 40 shows the block diagram of a Combined Ambulatory ECG and Alternans system. The functional blocks are the same as those described for the combined stress test and alternans system described above unless otherwise stated. Electrodes 73 are placed on the subject, the ECG data is amplified and recorded 74 on a cassette tape 75 (alternate embodiments may use digital storage of the data and much of the processing described below may take place in the recording device instead of the playback system). The cassette tape is later played back and digitized 76 and QRS complexes are detected 77 and heart rate is computed 78. The QRS complexes are cross correlated with the average beat templates and aligned in 79, RR intervals are computed 80 and ectopic beats are detected 81. Rates of ectopics are computed 82 as well as the existence of specific rhythms such as runs of ectopics or ventricular tachycardia using techniques well known in the art and these data are included in printed reports 90.

Average beats are updated 83, the PQ, ST an T-wave segments are determined 84, noise levels are computed 85 and the beat matrix is stored 86 all as described above.

However, the strategy for respiration determination and correction is unlike that used in the combined alternans and the stress test system. Most ambulatory recorders do not include a channel for respiration and, although one embodiment would include a respiration channel and possibly epoch selection processing in the recording device, it is desirable to create an analysis system which will work with existing recorders and therefore not require a respiration channel. The preferred strategy therefore is to detect respiration rate from changes in the ECG and to eliminate from consideration epochs where the respiration rate is at an even submultiple of the heart rate. This is accomplished in 86 by measuring the amplitude changes of the QRS complex in one lead, or the orientation of the QRS vector in space with multiple leads, and determining the respiration frequency (for methods see "Clinical Validation of the ECG-Derived Respiration (EDR) Technique", by G.B. Moody et al, 1986, cited earlier). If the respiration rate is within a few percent of 1/4 or 1/6 of the heart rate, then the epoch will not be selected for alternans analysis.

Another embodiment is to compute the FFT's for the alternans spectra as described previously, but then to reject any epochs or spectra which have peaks at 1/4 or 1/6 of the heart rate. Another embodiment is to include the respiratory rate or other indication of respiratory peak in the output of the system in such a manner that the operator can reject any data from inappropriate epochs.

The system then selects epochs for analysis 88 in the manner described previously. However, since the rate of any rhythmic exercise is not controlled by the system, it is more important that any epochs with rhythmic artifact at even submultiples of the heart rate be rejected. The weight given to PQ segment or other noise at an even submultiple of the heart rate is therefore increased. Other embodiments which use recording devices designed for the alternans measurements can include multiple channels or specific measurements of artifact for such use. Finally, alternans is calculated 89 as described previously (without respiration correction), and the results printed in reports 90.

In another example, one records electrocardiographic signals over a long period of time, generally in excess of one hour and often 24 hours in duration. This recording generally may be obtained with a portable electrocardiographic recording device (often called a Holter monitor in the art). During the recording period the patient may experience a variety of physiologic stresses such as exercise, emotional stress, transient myocardial ischemia, sexual activity, and straining during bowel movements. In this preferred embodiment the alternans occurring during these episodes of spontaneous physiologic stress may be measured by analysis of the electrocardiographic waveforms. Such episodes may be identified for example by identifying periods during which the heart rate is elevated. It is also advantageous in this preferred embodiment to measure artifact, resulting for example from walking or running, which may interfere with the alternans measurement in the same manner as described above. This may be accomplished for example by measuring variability in a segment of the electrocardiographic waveform during which there is normally no cardiac electrical activity such as the PQ segment. Thus variability in the PQ segment may be attributed to noise not of cardiac origin. Alternatively, artifact can be measured from recordings made from electrodes located at positions on the body which are expected to result in little recorded cardiac activity. Alternatively a measure of rhythmic artifact may be obtained from an accelerometer worn by the patient. In all cases the data would be analyzed for the level of total artifact, and/ or for the level of artifact occurring at or near the alternans frequency (i.e., half the heart rate), and/or for the level of artifact occurring at a sub-multiple of the alternans frequency (such as one quarter or one sixth the heart rate) where an harmonic of the artifact may interfere with the alternans measurement. Segments of data or measurements made during such data would be ignored or de-rated or the levels of potentially interfering artifact would be reported so that the clinician could consider such in interpreting the significance of the test result.

VIII. <u>Other Embodiments</u>

In one example, one applies a physiologic stress and then collects data for analysis of the temporal pattern of cycle-to-cycle variability in waveform morphology during and/or upon cessation of the stress. In this manner one avoids the problem of physiologic noise induced during the physiologic stress itself, but take advantage of the effect of the stress in augmenting a particular temporal pattern of cycle-to-cycle variability in physiologic waveforms. During physiologic stress tests used for the purpose of detecting coronary artery disease data are generally collected during the period following the cessation of the stress. Thus it is particularly convenient to collect such data in a stress test combined both for the purpose of detecting coronary artery disease and cycle-to-cycle variability in waveform morphology such as alternans.

In certain examples, the techniques described above may be used together with electrical pacing techniques (e.g., using electrodes placed in the patient's heart, using external body surface pacing, esophogeal pacing). For example, the patient's heart rate may be raised to a desired level (e.g., 90-150 beats per minute) and one or more of the signal processing techniques described above are used to measure the level of alternans. One or more of the assessment techniques described above are used to determine the patient's risk to cardiac electrical instability.

IX. Clinical Study

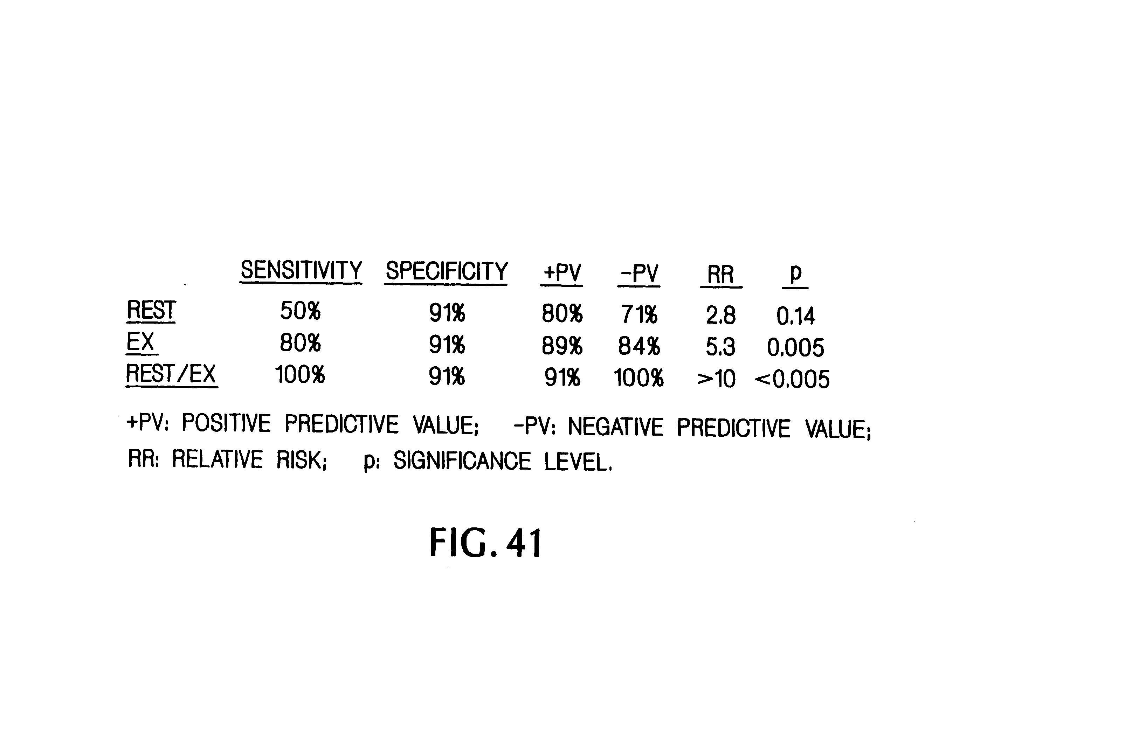

To test the methods described, including the use of physiological stress, noise reduction from multiple electrodes, epoch selection, noise measurement and rejection, and the use of multiple stages a clinical study was performed on 21 patients. Measurements of alternans using all of these techniques were made on 21 patients at rest, and during a bicycle exercise protocol designed to maintain the heart rate between 100-110 beats per minute. The results of this test were compared with the vulnerability to ventricular arrhythmias as define by a prior episode of sustained ventricular tachycardia or fibrillation or induction of sustained ventricular tachycardia or fibrillation during programmed electrophysiologic stimulation. The results (see Fig. 41) show that alternans measured at rest was not a statistically significant predictor of ventricular vulnerability, indicated by the fact that the p value was greater than 0.05. Measurements made during exercise were highly significant (p = 0.005) with a sensitivity of 80% and specificity of 91%. Combined measurements made during rest and exercise were even a better predictor (p < 0.005) with a sensitivity of 100% and specificity of 91%.

X. Conclusion

The various examples described make it possible for alternans to be used as a clinical predictor of ventricular arrhythmias under conditions of clinical practice. Each of the improvements discussed above may be applied individually and each substantially improves the ability to measure alternans in order to assess a patient's cardiac electrical stability. A novel and unexpected result of this invention is that alternans can be used as a non-invasive clinical tool to identify individuals at risk of ventricular arrhythmias.

**Claims**

1.   An apparatus for measuring an alternans pattern of cycle-to-cycle morphology variations in a sequence of substantially repeating physiologic waveforms in at least one signal measured from a patient, the apparatus comprising:

a means for receiving a physiologic signal representative of activity of the heart of a patient comprising one or more transducers adapted for respectively operatively measuring one or more signals from the patient;

a means for digitally processing the physiologic signal to determine a level of alternans in the signal, the physiologic signal representative of the activity of the heart of a patient is representative of the activity of the heart of the patient with an altered physiologic condition as a result of stress to the heart; and, a stressing device, **characterised in that** said

stressing device comprises an exerciser for controllably exercising the patient.

2.   An apparatus for measuring an alternans pattern of cycle-to-cycle morphology variations in sequence of substantially repeating physiological waveforms in at least one signal measured from a patient, the apparatus comprising:

a means for receiving a physiologic signal representative of activity of the heart of a patient comprising one or more transducers adapted for respectively operatively measuring one or more signals from the patient;

a means for digitally processing the physiologic signal to determine a level of alternans in the signal, the physiologic signal representative of the activity of the heart of a patient is representative of the activity of the heart of the patient with an altered physiologic condition as a result of stress to the heart; and a stressing device, **characterised in that** said

stressing device comprises a device adapted to administer a pharmacological agent that controllably alters the heart rate of the patient.

3.   An apparatus for measuring an alternans pattern of cycle-to-cycle morphology variations in sequence of substantially repeating physiological waveforms in at least one signal measured from a patient, the apparatus comprising:

a means for receiving a physiologic signal representative of activity of the heart of a patient comprising one or more transducers adapted for respectively operatively measuring one or more signals from the patient;

a means for digitally processing the physiologic signal to determine a level of alternans in the signal, the physiologic signal representative of the activity of the heart of a patient is representative of the activity of the heart of the patient with an altered physiologic condition as a result of stress to the heart; and a stressing device, **characterised in that** said

stressing device comprises a tilt table adapted to securely hold the patient and further adapted to enable selective alteration of the orientation of the patient's body to be different from a supine position for stressing the patient's heart.

4. The apparatus of any one of Claims 1 to 3, in which the processor is further adapted to determine indices of ischemia from said one or more measured signals.

5. The apparatus of any one of Claims 1 to 4, in which at least one of the one or more transducers comprises a multi-segment electrode having multiple separate surfaces for achieving electrical contact with a region on the surface of the patient.

6. The apparatus of Claim 5, further comprising circuitry coupled to the one or more transducers and adapted to measure a signal representative of the respiratory pattern of the patient.

7. The apparatus of Claim 5, further comprising circuitry coupled to the multi-segment electrode and adapted to measure a signal representative of the electrical impedance between the multi-segment electrode and the patient.

8. The apparatus of any one of Claims 1 to 7, further comprising a recorder coupled to the one or more transducers and adapted to store signals measured by said one or more transducers for a period in excess of one hour, and in which the processor is operatively adapted to characterise portions of the measured signals corresponding to one or more periods according to one or more preselected criteria, and further adapted to obtain, from the one or more stored signals corresponding to the one or more selected periods, a measure of the level of alternans in said at least one representative signal for assessing the condition of the patient's heart.

9. The apparatus of Claim 8, further comprising an output for providing a signal representative of the determined level of alternans and a noise signal.

10. The apparatus of Claim 9, in which the output is further adapted to provide a signal representative of the patient's heart rate.

11. The apparatus of any one of Claims 1 to 10, in which the processor is capable of processing the signals to produce one or more of the following:

a multi-lead electrocardiogram, a signal averaged elctrocardiogram, an indication as to the presence of coronary artery disease, an analysis of ARS variability, or physiologic system identification.

12. The apparatus of any preceding claim, wherein the stressing device is arranged to alter the physiologic condition of the patient so that the patient's heart rate is above a predetermined rate, and preferably is generally in the range of 90 to 150 beats per minute.

13. The apparatus of any proceding claim when dependent on claim 1, wherein the exerciser comprises a bicycle, a treadmill, or a staircase.

14. The apparatus of Claim 13, comprising means for producing a signal for controlling the patient's exercise intensity, preferably to achieve a desired heart rate in the patient.

15. The apparatus of any one of Claims 13 or 14, comprising means for receiving signals representative of the patient's rate of exercise.

16. The apparatus of any one of Claims 13 to 15, comprising means for producing a signal for controlling the patient's rate of exercise.

17. The apparatus of Claim 16, wherein the means for producing a signal is arranged to select the patient's rate of exercise to be between about 28 percent of the patient's heart rate and about 43 percent of the patient's heart rate or between about 57 percent of the patient's heart rate and about 72 percent of the patient's heart rate.

18. The apparatus of Claim 16 or Claim 17, wherein the means for producing a controlling signal is arranged to reduce noise that interferes with determining the level of alternans, preferably noise having a substantially repeating component that repeats at a frequency of about one half the patient's heart rate or at a sub-multiple thereof.

19. The apparatus of any one of Claims 16 to 18, wherein the means for producing a controlling signal is arranged to reduce noise artifacts generated by one or more sources other than the patient's heart.

20. The apparatus of Claim 19, wherein the means for producing a controlling signal is arranged to reduce noise artifacts generated as a result of exercise of the patient.

21. The apparatus of Claim 19, wherein the means for producing a controlling signal is arranged to reduce noise artifacts generated as a result of respiration of the patient.

22. The apparatus of any one of Claims 16 to 21, wherein the means for producing a controlling signal is arranged to produce a signal based on a predetermined exercise protocol specifying target exercise rates.

23. The apparatus of any one of the preceding claims, comprising means for determining the patient's heart rate, preferably comprising means for producing an output signal representative of the patient's heart rate.

24. The apparatus of any one of the preceding claims, comprising means for producing an output signal representative of the patient's respiratory rate.

25. The apparatus of any one of the preceding claims, wherein the means for digitally processing is arranged to process the signal to reduce noise that interferes with determining the level of alternans.

26. The apparatus of Claim 25, wherein the means for digitally processing the signal to reduce noise is arranged to determine the level of alternans on portions of the received physiologic signal corresponding to periods when the level of exercise falls within a predetermined range.

27. The apparatus of Claim 25, wherein the means for digitally processing the signal to reduce the effect of noise is arranged to select, for the determination of the level of alternans, portions of the received signal based on the presence of abnormal beats.

28. The apparatus of Claim 27, wherein the means for digitally processing is arranged to select portions for the determination of alternans based on a comparison of a weighted predetermined level.

29. The apparatus of any one of the preceding claims, wherein the means for digitally processing is arranged to process physiologic signals from periods of time before the physiologic condition of the patient is altered.

30. The apparatus of any one of the preceding claims, wherein the means for digitally processing is arranged to process physiologic signals from period of time after the physiologic condition of the patient is altered.

31. The apparatus of any one of the preceding claims comprising:

    means for obtaining a second signal from the patient; and

    means for determining a relationship between variability in the first and second received signals.

32. The apparatus of Claim 31, wherein the means for determining a relationship is arranged to relate desired features of the first and second signals or to relate noise components of the first and second signals.

33. The apparatus of Claim 31, wherein the means for determining a relationship is arranged to combine first and second signals to cancel noise.

34. The apparatus of Claim 31, wherein the means for obtaining a second signal is arranged to measure interbeat interval variability.

35. The apparatus of Claim 31, wherein the second signal represents noise, preferably impedance artifact or respiration artifact.

36. The apparatus of any one of the preceding claims, comprising means for producing a signal for controlling the physiologic condition of the patient in a manner such that the effects of interfering noise sources is reduced, preferably comprising means for controlling the respiratory rate of the patient.

37. The apparatus of any one of the preceding claims, wherein the means for digitally processing is arranged to process the signal to reduce the effect of noise signals having a frequency of about half of the patient's heart rate or at about a sub-multiple half of the patient's heart rate.

38. The apparatus of Claim 37, wherein the means for digitally processing is arranged adaptively to remove noise signals before a level of alternans is determined.

39. The apparatus of Claim 37 or Claim 38, wherein the means for digitally processing the signal to reduce the effect of noise signals is arranged to base the determination of a level of alternans on portions of the received signal corresponding to periods when the patient's heart rate is above a predetermined level.

40. The apparatus of Claim 25 or 37, or any claim dependent thereon, wherein the means for digitally processing the signal to reduce the effect of noise signals is arranged to base the determination of a level of alternans on portions of the received signal corresponding to periods when the patient's respiratory rate is different from the patient's heart rate and is different from sub-multiples of the patient's heart rate.

41. The apparatus of any one of Claims 37 to 40, wherein the means for digitally processing is further arranged to assign weights to portions of the received signal based on the presence of abnormal beats, and to base the determination of a level of alternans on the assigned weights.

42. The apparatus of any one of the preceding claims, comprising means for determining the patient's heart rate, and simultaneously producing a signal representative of the determined level of alternans and producing a signal representative of the determined heart rate.

43. The apparatus of any one of the preceding claims, wherein the means for digitally processing is arranged to compensate for interfering variability in the morphology of the substantially repeating waveforms in the received signal.

44. The apparatus of Claim 43, wherein the means for digitally processing is arranged to compensate for interfering variability generated by the patient's respiratory activity.

45. The apparatus of Claim 43, wherein the means for digitally processing is arranged to compensate intercycle interval variability.

46. The apparatus of Claim 45, wherein the means for compensating for intercycle interval variability is arranged to determine a relationship between the variation in intercycle intervals and changes in waveform morphology.

47. The apparatus of Claim 46, wherein the means is a filter relating measurements of a given waveform to the sequence of preceding intercycle intervals.

48. The apparatus of Claim 37, wherein the means for digitally processing is arranged to relate the variability in a noise signal to the variability in the morphology of the substantially repeating waveforms.

49. The apparatus of Claim 48, wherein the noise signal represents a measure of the respiratory activity of the patient.

50. The apparatus of Claim 37, comprising:

means for receiving a second physiologic signal representative of activity of the patient's heart;

means for determining a relationship between desired features of the first and second received signals;

means for determining a relationship between noise components of the first and second received signals; and

means for combining the first and second signals to minimise noise in the determination of a level of alternans.

51. The apparatus of Claims 31 or 50, or any claim dependent thereon, wherein the means for receiving the first and second signals are ECG leads, the first and second signals being combined in a manner preserving the orientation of the ECG leads in space.

52. The apparatus of Claims 31 or 50, or any claim dependent thereon, further comprising means for processing the first and second signals to determine a distortion metric, and wherein the first and second received signals are combined in a manner so that the distortion metric is below a prescribed level.

53. The apparatus of any one of the preceding claims, wherein the means for digitally processing is arranged to base the determination of a level of alternans on portions of the received signal corresponding to periods when a level of noise is lower than a preselected level.

54. The apparatus of any one of the preceding claims, wherein the means for digitally processing is arranged to base the determination of a level of alternans on portions of the received signal corresponding to periods when the determined level of alternans is greater than a preselected level.

55. The apparatus of any one of the preceding claims wherein the means for digitally processing is arranged to characterise one or more portions of the received signal based on one or more preselected criteria and use the characterisation of the portions to determine a level of alternans in the signal.

56. The apparatus of Claim 55, wherein the means for digitally processing is arranged to characterise the portions by selecting portions of the received signal to exclude from the determination of alternans step.

57. The apparatus of Claim 56, wherein the means for digitally processing is arranged to exclude portions of the received signal by determining a level of alternans on portions of the received signal corresponding to periods when the patient's heart rate is above a predetermined level.

58. The apparatus of Claim 56, wherein the means for digitally processing is arranged to exclude portions of the received signal by determining a level of alternans on portions of the received signal corresponding to periods when the patient's respiratory rate is different from the patient's heart rate and is different from sub-multiples of the patient's heart rate.

59. The apparatus of Claim 55, wherein the means for digitally processing is arranged to characterise the portions by assigning weights to portions of the received signal based on the presence of abnormal beats, and is further arranged to base the determination of a level of alternans on the assigned weights.

60. The apparatus of Claim 55, comprising means for independently estimating the level of alternans based on sequences of data between abnormal waveforms; and means for combining the independent estimations to determine the measure of the level of alternans.

61. The apparatus of Claim 55, wherein the means for digitally processing is arranged to determine a level of alternans based on a predetermined assumption that a particular abnormal waveform is followed by a normal waveform of a given phase.

62. The apparatus of Claim 61, wherein the means for digitally processing is arranged to exclude abnormal waveforms when determining the level of alternans.

63. The apparatus of Claim 61, wherein means for digitally processing is arranged to replace abnormal waveforms by average waveforms in the determination of the level of alternans.

64. The apparatus of Claim 61, wherein the one or more signals are measured by means for measuring the respiratory rate of the patient, preferably by impedance plethysmography, and wherein the means for digitally processing is arranged to remove from consideration portions of said measured signals corresponding to periods when the respiratory rate of the patient is a sub-multiple of the patient's heart rate.

65. The apparatus of Claim 61, wherein the means for measuring the one or more signals comprises one or more transducers placed on the patient for measuring ECG signals, and wherein the respiratory rate of the patient rate is determined by analysis of the measured ECG signals.

66. The apparatus Claim 55, comprising means for analysing said ECG signals by analysing the amplitude or vectorcardiographic angle of the features of the measured ECG signals.

67. The apparatus of Claim 66, wherein the preselected criterion is the patient's heart rate.

68. The apparatus of Claim 67, comprising means for removing the measured signals from consideration when corresponding to periods when an interfering noise signal varies at a multiple or sub-multiple of the patient's heart rate.

69. The apparatus of Claim 55, wherein the preselected criterion is the patient's respiration rate.

70. The apparatus of Claim 55, wherein the preselected criterion is the presence of abnormal beats in one or more of the measured signals.

71. The apparatus of any one of the preceding claims, comprising means for providing an output representative of the level of altemans simultaneously with the step of processing the physiologic signal.

72. The apparatus of Claim 71 wherein, the means for digitally processing are arranged to, after a predetermined number of the substantially repeating waveforms are received, form a segment of data, and then to process the data segment to determine a level of alternans, to provide the determined level of alternans, and to repeat the steps of receiving, processing, and providing are repeated.

73. The apparatus of Claim 72, comprising:

  means for determining when a sufficient number of partial sequences have been processed based on a preselected criterion for assessment of the condition of the patient's heart; and

  means for terminating the steps of receiving, processing, providing and repeating when said preselected criterion has been satisfied.

74. The apparatus of any one of Claims 71 to 73, comprising:

  means for digitally processing the received physiologic signal to determine a level of noise; and

  means for using the determined level of noise to produce an indication of the condition of the patient's heart by determining with a predetermined level of statistical certainty from said level of noise whether the alternans level is above an upper threshold or is below a lower threshold.

75. The apparatus of Claim 74, wherein the means for using is arranged to compare the level of alternans against a first predetermined threshold and to compare the ratio of the alternans level to the noise level against a second predetermined threshold.

76. The apparatus of Claim 75, wherein the means for using alternans is arranged to determine the level of alternans from a first portion of a measured substantially repeating waveform, and to determine the level of noise is determined from a different portion of the same waveform.

77. The apparatus of Claim 76, wherein the means for receiving the signal is arranged to receive an ECG signal and the means for processing is arranged to determine the level of noise from the PQ segment.

78. The apparatus of Claim 74, wherein the means for using is arranged to compare the level of alternans in one portion of a measured substantially repeating waveform to a measure of noise in a different frequency band of the same waveform.

**79.** The apparatus of Claim 74, comprising:

means for determining the level in an alternating component in the received signal and determining the standard deviation of the energy of noise in the signal; and

means for determining whether the level in the alternating component is greater than a predetermined threshold by one or more standard deviations of the noise level to give an indication that the patient is at risk for cardiac instability.

**80.** The apparatus of Claim 74, wherein the means for determining is arranged to compare the determined noise level against a predetermined threshold, and to determine whether said noise level is greater than predetermined threshold to give an indication that the assessment of the patient's risk for cardiac instability is indeterminate.

**81.** The apparatus of any one of the preceding claims, comprising means for enhancing the measurement of the pattern of cycle-to-cycle morphology variations by reducing the effect of variability in the interval between adjacent waveforms in said at least one representative signal.

**82.** The apparatus of any one of the preceding claims, comprising means for compensating for interfering variability in the morphology of the substantially repeating waveforms in the received signal.

**83.** The apparatus of any one of the preceding claims, comprising means for additionally processing the one or more signals to produce one or more of the following:

a multi-lead electrocardiogram, a signal averaged electrocardiogram, an analysis of ARS complex variability, or physiologic system identification.

**Patentansprüche**

**1.** Gerät zur Messung eines Alternansmusters von Zyklus-zu-Zyklus-Morphologieveränderungen in einer Sequenz von sich in der Regel wiederholenden physiologischen Wellenformen mindestens eines an einem Patienten gemessenen Signals, wobei zu den Bestandteilen des Geräts gehoren:

eine Einrichtung zum Empfang eines physiologischen, die Herztatigkeit eines Patienten verkörpemden Signals, zu der ein oder mehrere Wandler gehören, die jeweils zur operativen Messung eines oder mehrerer Signale des Patienten ausgelegt sind,
eine Einrichtung zur digitalen Verarbeitung des physiologischen Signals zwecks Bestimmung eines Alternansniveaus in dem Signal, wobei das die Herztätigkeit eines Patienten verkörpernde physiologische Signal die Herztätigkeit des Patienten bei verändertem physiologischem Zustand im Ergebnis einer Belastung des Herzens anzeigt, und
eine Belastungseinheit, **dadurch gekennzeichnet, dass**
zu der Belastungseinheit ein Ergometer zur steuerbaren Belastung des Patienten gehört.

**2.** Gerät zur Messung eines Alternansmusters von Zyklus-zu-Zyklus-Morphologieveränderungen in einer Sequenz von sich in der Regel wiederholenden physiologischen Wellenformen mindestens eines am Patienten gemessenen Signals, wobei zu den Bestandteilen des Geräts gehören:

eine Einrichtung zum Empfang eines physiologischen, die Herztätigkeit eines Patienten verkörpernden Signals, zu der ein oder mehrere Wandler gehören, die jeweils zur operativen Messung eines oder mehrerer Signale des Patienten ausgelegt sind,
eine Einrichtung zur digitalen Verarbeitung des physiologischen Signals zwecks Bestimmung eines Alternansniveaus in dem Signal, wobei das die Herztätigkeit eines Patienten verkörpernde physiologische Signal die Herztätigkeit des Patienten bei verändertem physiologischem Zustand im Ergebnis einer Belastung des Herzens anzeigt, und
eine Belastungseinheit, **dadurch gekennzeichnet, dass**

zu der Belastungseinheit eine Einrichtung zur Verabreichung eines Arzneimittels für die steuerbare Änderung der Herzfrequenz des Patienten gehört.

3. Gerät zur Messung eines Alternansmusters von Zyklus-zu-Zyklus-Morphologieveränderungen in einer Sequenz von sich in der Regel wiederholenden physiologischen Wellenformen mindestens eines am Patienten gemessenen Signals, zu dessen Bestandteilen gehören:

eine Einrichtung zum Empfang eines physiologischen, die Herztätigkeit eines Patienten verkörpernden Signals, zu der ein oder mehrere Wandler gehören, die jeweils zur operativen Messung eines oder mehrerer Signale des Patienten ausgelegt sind,
eine Einrichtung zur digitalen Verarbeitung des physiologischen Signals zwecks Bestimmung eines Alternansniveaus in dem Signal, wobei das die Herztätigkeit eines Patienten verkörpernde physiologische Signal die Herztätigkeit eines Patienten bei verändertem physiologischem Zustand im Ergebnis einer Belastung des Herzens anzeigt, und
eine Belastungseinheit, **dadurch gekennzeichnet, dass** zu der Belastungseinheit ein Kipptisch gehört, der zum sicheren Fixieren des Patienten ausgelegt ist und ermöglicht, zur Belastung des Herzen des Patienten wahlweise die Orientierung des Körpers des Patienten gegenüber einer Lage auf dem Rücken zu ändern.

4. Gerät nach einem der Ansprüche 1 bis 3, bei dem der Prozessor des Weiteren so ausgelegt ist, dass er aus dem einen oder den mehreren gemessenen Signalen Ischämieindizes ermittelt.

5. Gerät nach einem der Ansprüche 1 bis 4, bei dem zumindest zu einem Wandler des einen oder der mehreren Wandler eine Mehrsegmentelektrode mit mehreren gesonderten Oberflächen zur Herstellung eines elektrischen Kontakts mit einem Areal an der Hautoberfläche des Patienten gehört.

6. Gerät nach Anspruch 5, zu dem des Weiteren eine Schaltung gehört, die an den einen oder die mehreren Wandler angeschlossen und zur Messung eines Signals ausgelegt ist, das den Respirationsverlauf des Patienten anzeigt.

7. Gerät nach Anspruch 5, zu dem femer eine Schaltung gehört, die an die Mehrsegmentelektrode angeschlossen und zur Messung eines Signals ausgelegt ist, das die elektrische impedanz zwischen der Mehrsegmentelektrode und dem Patienten anzeigt.

8. Gerät nach einem der Ansprüche 1 bis 7, zu dem des Weiteren ein Aufzeichnungsgerät gehört, das an einen oder mehrere Wandler angeschlossen und zur Speicherung der im Verlaufe eines Zeitabschnitts von mehr als einer Stunde mit Hilfe des einen oder der mehreren Wandler gemessenen Signale ausgelegt ist, und bei dem der Prozessor funktionsmäßig so ausgelegt ist, dass er Abschnitte der gemessenen Signale **kennzeichnet**, die einem oder mehreren Zeitabschnitten hinsichtlich des einen oder der mehreren vorgegebenen Kriterien entsprechen, und des Weiteren so ausgelegt ist, dass er aus dem einen oder den mehreren gespeicherten Signalen, die dem einen oder den mehreren ausgewählten Zeitabschnitten entsprechen, zur Bewertung des Zustands des Herzens bei einem Patienten ein Maß des Alternansniveaus in dem mindestens einen der Erkennung dienenden Signal ermittelt.

9. Gerät nach Anspruch 8, zu dem des Weiteren ein Ausgang zur Abgabe eines das ermit telte Alternansniveau verkörpernden Signals und eines Störsignals gehört.

10. Gerät nach Anspruch 9, bei dem der Ausgang des Weiteren zur Abgabe eines die Herzfrequenz des Patienten anzeigenden Signals ausgelegt ist.

11. Gerät nach einem der Ansprüche 1 bis 10, bei dem der Prozessor zur Verarbeitung der Signale mit dem Ziel der Erzeugung eines oder mehrerer der folgenden Diagnosemittel ausgelegt ist:

eines Mehrfachableitungselektrokardiogramms, eines signalgemittelten Elektrokardiogramms, einer Indikation zum Vorliegen einer koronaren Herzerkrankung, einer Analyse der RSA-Variabilität oder einer Feststellung des Zustands eines physiologischen Systems.

12. Gerät nach einem der vorstehenden Ansprüche, bei dem die Belastungseinheit die Aufgabe erfüllt, den physiologischen Zustand des Patienten so zu ändern, dass die Herzfrequenz des Patienten über einem vorgegebenen Wert, vorzugsweise im Wesentlichen im Bereich von 90 bis 150 Schlägen pro Minute liegt.

**13.** Gerät nach einem der vorstehenden Ansprüche, wenn abhängig von Anspruch 1, bei dem das Ergometer ein Fahrrad-, Laufband- oder Stepperergometer ist.

**14.** Gerät nach Anspruch 13, zu dem eine Einrichtung zur Erzeugung eines Signals für die Steuerung der Patientenbelastungsintensität, vorzugsweise für die Erzielung einer Sollherzfrequenz des Patienten, gehört.

**15.** Gerät nach einem der Ansprüche 13 oder 14, zu dem eine Einrichtung zum Empfang von Signalen gehört, die die Herzfrequenz des Patienten während der Betastung anzeigen.

**16.** Gerät nach einem der Ansprüche 13 bis 15, zu dem eine Einrichtung zur Erzeugung eines Signals gehört, durch das die Belastungsintensität des Patienten gesteuert wird.

**17.** Gerät nach Anspruch 16, bei dem die Signalerzeugungseinrichtung die Aufgabe erfülit, die Belastungsintensität des Patienten so auszuwählen, dass sie in einem Bereich zwischen etwa 28 % und etwa 43 % der Herzfrequenz des Patienten oder in einem Bereich zwischen etwa 57 % und etwa 72 % der Herzfrequenz des Patienten liegt.

**18.** Gerät nach einem der Ansprüche 16 oder 17, bei dem die Steuersignalerzeugungseinrichtung die Aufgabe erfüllt, die Störsignale zu reduzieren, die bei der Bestimmung des Alternansniveaus stören, vorzugsweise Störsignale, die einen sich in der Regel wiederholenden Anteil aufweisen und sich mit einer Frequenz von etwa 0,5 der Herzfrequenz des Patienten oder eines ganzzahligen Teilers davon wiederholen.

**19.** Gerät nach einem der Ansprüche 16 bis 18, bei dem die Steuersignalerzeugungseinrichtung die Aufgabe erfüllt, Geräuschartefakte zu reduzieren, die durch eine oder mehrere Quellen entstehen, die nicht das Herz des Patienten sind.

**20.** Gerät nach Anspruch 19, bei dem die Steuersignalerzeugungseinrichtung die Aufgabe erfüllt, Geräuschartefakte zu reduzieren, die durch die Belastung des Patienten entstehen.

**21.** Gerät nach Anspruch 19, bei dem die Steuersignalerzeugungseinrichtung die Aufgabe erfüllt, Geräuschartefakte zu reduzieren, die durch die Atmung des Patienten entstehen.

**22.** Gerät nach einem der Ansprüche 16 bis 21, bei dem die Steuersignalerzeugungseinrichtung die Aufgabe erfüllt, ein Signal auf der Grundlage eines bestimmten, die Zietbelastungsintensitäten festlegenden Belastungsprotokolls zu erzeugen.

**23.** Gerät nach einem der vorstehenden Ansprüche, zu dem eine Einrichtung zur Bestimmung der Herzfrequenz des Patienten, vorzugsweise eine Einrichtung zur Erzeugung eines Ausgangssignals gehört, das die Herzfrequenz des Patienten anzeigt.

**24.** Gerät nach einem der vorstehenden Ansprüche, zu dem eine Einrichtung zur Erzeugung eines Ausgangssignals gehört, das die Atemfrequenz des Patienten anzeigt.

**25.** Gerät nach einem der vorstehenden Ansprüche, zu dem die Einrichtung zur digitalen Verarbeitung die Aufgabe erfüllt, das Signal so zu verarbeiten, dass das Rauschen, welches die Bestimmung des Alternansniveaus stört, reduziert wird.

**26.** Gerät nach Anspruch 25, bei dem die Einrichtung zur digitalen Verarbeitung des Signals zwecks Verringerung des Rauschens die Aufgabe erfüllt, das Alternansniveau auf Abschnitten des empfangenen physiologischen Signals zu bestimmen, die Perioden entsprechen, in denen die Belastungsintensität in einen vorgegebenen Bereich fällt.

**27.** Gerät nach Anspruch 25, bei dem die Einrichtung zur digitalen Verarbeitung des Signals zwecks Verringerung der Auswirkung von Rauschen die Aufgabe erfüllt, für die Bestimmung des Alternansniveaus auf der Grundlage auftretender abnormaler Schläge Abschnitte des empfangenen Signals auszuwählen.

**28.** Gerät nach Anspruch 27, bei dem die Einrichtung zur digitalen Verarbeitung die Aufgabe erfüllt, auf der Grundlage eines Vergleichs eines gewichteten bestimmten Niveaus Abschnitte für die Bestimmung von Alternans auszuwählen.

29. Gerät nach einem der vorstehenden Ansprüche, bei dem die Einrichtung zur digitalen Verarbeitung die Aufgabe erfüllt, physiologische Signale von Zeitabschnitten vor der Änderung des physiologischen Zustands des Patienten zu verarbeiten.

30. Gerät nach einem der vorstehenden Ansprüche, bei dem die Einrichtung zur digitalen Verarbeitung die Aufgabe erfüllt, physiologische Signale eines Zeitabschnitts nach der Änderung des physiologischen Zustands des Patienten zu verarbeiten.

31. Gerät nach einem der vorstehenden Ansprüche, zu dem gehören:

> eine Einrichtung zur Gewinnung eines zweiten patientenbezogenen Signals und
> eine Einrichtung zur Bestimmung einer Relation zwischen der Variabilität des ersten und
> zweiten empfangenen Signals.

32. Gerät nach Anspruch 31, bei dem die Relationsbestimmungseinrichtung die Aufgabe erfüllt, eine Relation zwischen vorgegebenen Kennwerten des ersten und zweiten Signals oder eine Relation zwischen den Rauschkomponenten des ersten und zweiten Signals zu bestimmen.

33. Gerät nach Anspruch 31, bei dem die Relationsbestimmungseinrichtung die Aufgabe erfüllt, das erste und das zweite Signal miteinander zu kombinieren, um das Rauschen zu beseitigen.

34. Gerät nach Anspruch 31, bei dem die Einrichtung zur Gewinnung eines zweiten Signals die Aufgabe erfüllt, die Schlagintervallvariabilität zu messen.

35. Gerät nach Anspruch 31, bei dem das zweite Signal ein Rauschen verkörpert, vorzugsweise ein impedanz- oder ein Atmungsartefakt.

36. Gerät nach einem der vorstehenden Ansprüche, zu dem eine Signalerzeugungseinrichtung für die Steuerung des physiologischen Zustands des Patienten in einer Weise gehört, dass die Auswirkungen von Störsignalquellen reduziert werden, und zu dem vorzugsweise eine Einrichtung zur Steuerung der Atemfrequenz des Patienten gehört.

37. Gerät nach einem der vorstehenden Ansprüche, bei dem die Einrichtung zur digitalen Verarbeitung die Aufgabe erfüllt, das Signal zu verarbeiten, um die Auswirkung von Störsignalen mit einer Frequenz von etwa 0,5 der Herzfrequenz des Patienten oder etwa eines ganzzahligen Teilers der Hälfte der Herzfrequenz des Patienten zu verringern.

38. Gerät nach Anspruch 37, bei dem die Einrichtung zur digitalen Verarbeitung die Aufgabe erfüllt, vor der Bestimmung eines Alternansniveaus adaptiv die Störsignale zu beseitigen.

39. Gerät nach Anspruch 37 oder 38, bei dem die Einrichtung zur digitalen Verarbeitung des Signals zwecks Verringerung der Auswirkung von Störsignalen die Aufgabe erfüllt, bei der Bestimmung des Alternansniveaus Abschnitte des empfangenen Signals als Grundlage zu verwenden, die den Zeiträumen entsprechen, in denen die Herzfrequenz des Patienten über einem vorgegebenen Wert liegt.

40. Gerät nach Anspruch 25 oder 37 oder einem davon abhängigen Anspruch, bei dem die Einrichtung zur digitalen Verarbeitung des Signals zwecks Verringerung der Auswirkung von Störsignalen die Aufgabe erfüllt, bei der Bestimmung des Alternansniveaus Abschnitte des empfangenen Signals als Grundlage zu verwenden, die Zeiträumen entsprechen, in denen sich die Atemfrequenz des Patienten von seiner Herzfrequenz sowie von den ganzzahligen Teilern seiner Herzfrequenz unterscheidet.

41. Gerät nach einem der Ansprüche 37 bis 40, bei dem die Einrichtung zur digitalen Verarbeitung des Weiteren die Aufgabe erfüllt, Abschnitten des empfangenen Signals auf der Grundlage des Vorhandenseins abnormaler Schläge Wichtungen zuzuweisen und die Bestimmung eines Alternansniveaus auf der Grundlage der Wichtungen vorzunehmen.

42. Gerät nach einem der vorstehenden Ansprüche, zu dem eine Einrichtung gehört, die die Herzfrequenz des Patienten bestimmt und gleichzeitig ein Signal erzeugt, das das ermittelte Alternansniveau anzeigt, und ein Signal erzeugt, das die ermittelte Herzfrequenz anzeigt.

43. Gerät nach einem der vorstehenden Ansprüche, bei dem die Einrichtung zur digitalen Verarbeitung die Aufgabe erfüllt, eine störende Variabilität in der Morphologie der sich in der Regel wiederholenden Wellenformen in dem empfangenen Signal auszugleichen.

44. Gerät nach Anspruch 43, bei dem die Einrichtung zur digitalen Verarbeitung die Aufgabe erfüllt, eine durch die Atmungsaktivität des Patienten bedingte störende Variabilität auszugleichen.

45. Gerät nach Anspruch 43, bei dem die Einrichtung zur digitalen Verarbeitung die Aufgabe erfüllt, die Zyklusintervallvariabilität auszugleichen.

46. Gerät nach Anspruch 45, bei dem die Einrichtung zum Ausgleichen der Zyklusintervallvariabilität die Aufgabe erfüllt, eine Relation zwischen der Änderung der Zyklusintervalle und Änderungen in der Wellenformmorphologie zu ermitteln.

47. Gerät nach Anspruch 46, bei dem die Einrichtung ein Filter ist, das die Messungen einer bestimmten Wellenform mit der Sequenz der vorhergehenden Zyklusintervalle in Relation setzt.

48. Gerät nach Anspruch 37, bei dem die Einrichtung zur digitalen Verarbeitung die Aufgabe erfüllt, die Variabilität eines Störsignals mit der Variabilität der Morphologie der sich in der Regel wiederholenden Wellenformen in Relation zu setzen.

49. Gerät nach Anspruch 48, bei dem das Störsignal ein Maß der Atmungsaktivität des Patienten darstellt.

50. Gerät nach Anspruch 37, zu dem folgenden Bestandteile gehören:

   eine Einrichtung zum Empfang eines zweiten physiologischen Signals, das die Herztätigkeit des Patienten anzeigt,
   eine Einrichtung zur Bestimmung einer Relation zwischen vorgegebenen Kennwerten des ersten und zweiten empfangenen Signals,
   eine Einrichtung zur Bestimmung einer Relation zwischen Rauschkomponenten des ersten und zweiten Signals und
   eine Einrichtung zur Kombination des ersten und zweiten Signals zwecks Minimierung des Rauschens bei der Bestimmung eines Altemansniveaus.

51. Gerät nach einem der Ansprüche 31, 50 oder einem davon abhängigen Anspruch, bei dem die Einrichtung zum Empfang des ersten und zweiten Signals EKG-Leitungen sind, wobei das erste und zweite Signal so miteinander kombiniert werden, dass die Orientierung der EKG-Leitungen im Raum erhalten bleibt.

52. Gerät nach einem der Ansprüche 31, 50 oder einem davon abhängigen Anspruch, zu dem des Weiteren eine Einrichtung zur Verarbeitung des ersten und zweiten Signals zwecks Bestimmung einer Verzerrungsmetrik gehört, und bei dem das erste und zweite empfangene Signal so miteinander kombiniert werden, dass die Verzerrungsmetrik unter einem vorgegebenen Niveau liegt.

53. Gerät nach einem der vorstehenden Ansprüche, bei dem die Einrichtung zur digitalen Verarbeitung die Aufgabe erfüllt, bei der Bestimmung des Alternansniveaus Abschnitte des empfangenen Signals als Grundlage zu verwenden, die den Zeiträumen entsprechen, in denen ein Störniveau unter einem vorgegebenen Niveau liegt.

54. Gerät nach einem der vorstehenden Ansprüche, bei dem die Einrichtung zur digitalen Verarbeitung die Aufgabe erfüllt, bei der Bestimmung des Alternansniveaus Abschnitte des empfangenen Signals als Grundlage zu verwenden, die den Zeiträumen entsprechen, in denen das ermittelte Alternansniveau über einem vorgegebenen Niveau liegt.

55. Gerät nach einem der vorstehenden Ansprüche, bei dem die Einrichtung zur digitalen Verarbeitung die Aufgabe erfüllt, einen oder mehrere Abschnitte des empfangenen Signals auf der Grundlage eines oder mehrerer vorgegebener Kriterien zu kennzeichnen und die Kennzeichnung der Abschnitte zur Ermittlung eines Alternansniveaus im Signal zu nutzen.

**56.** Gerät nach Anspruch 55, bei dem die Einrichtung zur digitalen Verarbeitung die Aufgabe erfüllt, die Abschnitte zu kennzeichnen, indem sie Abschnitte des empfangenen Signals auswählt, die aus dem Schritt der Alternansbestimmung auszuschließen sind.

**57.** Gerät nach Anspruch 56, bei dem die Einrichtung zur digitalen Verarbeitung die Aufgabe erfüllt, Abschnitte des empfangenen Signals auszuschließen, indem sie ein Alternansniveau anhand von Abschnitten des empfangenen Signals bestimmt, die Zeitabschnitten entsprechen, in denen die Herzfrequenz des Patienten über einem vorgegebenen Wert liegt.

**58.** Gerät nach Anspruch 56, bei dem die Einrichtung zur digitalen Verarbeitung die Aufgabe erfüllt, Abschnitte des empfangenen Signals auszuschließen, indern sie ein Alternansniveau anhand von Abschnitten des empfangenen Signals bestimmt, die Zeitabschnitten entsprechen, in denen sich die Atemfrequenz des Patienten von der Herzfrequenz des Patienten und von ganzzahligen Teilem der Herzfrequenz des Patienten unterscheidet.

**59.** Gerät nach Anspruch 55, bei dem die Einrichtung zur digitalen Verarbeitung die Aufgabe erfüllt, die Abschnitte zu kennzeichnen, indem sie in Abhängigkeit vom Vorhandensein abnormaler Schläge Abschnitten des empfangenen Signals Wichtungen zuweist, und des Weiteren die Aufgabe erfüllt, bei der Bestimmung eines Alternansniveaus die zugewiesenen Wichtungen als Grundlage zu verwenden.

**60.** Gerät nach Anspruch 55, zu dem eine Einrichtung zur autonomen Abschätzung des Alternansniveaus anhand von Datensequenzen zwischen abnormalen Wellenformen und eine Einrichtung zur Kombination der autonomen Abschätzungen zwecks Bestimmung des Maßes des Alternansniveaus gehören.

**61.** Gerät nach Anspruch 55, bei dem die Einrichtung zur digitalen Verarbeitung die Aufgabe erfüllt, ein Alternansniveau anhand einer vorgegebenen Annahme zu bestimmen, dass auf eine besondere abnorme Wellenform eine normale Wellenform mit einer bestimmten Phase folgt.

**62.** Gerät nach Anspruch 61, bei dem die Einrichtung zur digitalen Verarbeitung die Aufgabe erfüllt, bei der Bestimmung des Alternansniveaus abnormale Wellenformen auszuschließen.

**63.** Gerät nach Anspruch 61, bei dem die Einrichtung zur digitalen Verarbeitung die Aufgabe erfüllt, bei der Bestimmung des Alternansniveaus abnormale Wellenformen durch Durchschnittsweltenformen zu ersetzen.

**64.** Gerät nach Anspruch 61, bei dem das eine oder die mehreren Signale mit Hilfe einer Einrichtung zur Messung der Atemfrequenz des Patienten, vorzugsweise mittels Impedanzplethysmographie, gemessen werden und bei dem die Einrichtung zur digitalen Verarbeitung die Aufgabe erfüllt, aus der Betrachtung Abschnitte der gemessenen Signale auszusondern, die Zeitabschnitten entsprechen, in denen die Atemfrequenz des Patienten gleich einem ganzzahligen Teiler der Herzfrequenz des Patienten ist.

**65.** Gerät nach Anspruch 61, bei dem zu der Einrichtung zur Messung des einen oder der mehreren Signale ein oder mehrere Wandler gehören, die zum Messen der EKG-Signale an dem Patienten positioniert sind, und bei dem die Atemfrequenz des Patienten mittels Analyse des gemessenen EKG-Signals ermittelt wird.

**66.** Gerät nach Anspruch 55, zu dem eine Einrichtung zur Analyse der EKG-Signale durch Analyse der Amplitude oder des vektorkardiographischen Winkels der Kennwerte der gemessenen EKG-Signale gehört.

**67.** Gerät nach Anspruch 66, bei dem das im Voraus gewählte Kriterium die Herzfrequenz des Patienten ist.

**68.** Gerät nach Anspruch 67, zu dem eine Einrichtung zum Ausschließen der gemessenen Signale aus der Betrachtung gehört, wenn diese Zeiträumen entsprechen, in denen ein Störsignal um ein Vielfaches oder einen ganzzahligen Teiler der Herzfrequenz des Patienten schwankt.

**69.** Gerät nach Anspruch 55, bei dem das im Voraus gewählte Kriterium die Atemfrequenz des Patienten ist.

**70.** Gerät nach Anspruch 55, bei dem das im Voraus gewählte Kriterium das Vorhandensein abnormaler Schläge in einem oder mehreren der gemessenen Signale ist.

**71.** Gerät nach einem der vorstehenden Ansprüche, zu dem eine Einrichtung zur Abgabe eines das Alternansniveau anzeigenden Ausgangssignals gleichzeitig mit der Ausführung des Schritts der Verarbeitung des physiologischen Signals gehört.

**72.** Gerät nach Anspruch 71, bei dem die Einrichtung zur digitalen Verarbeitung die Aufgabe erfüllt, nachdem eine vorgegebene Anzahl der sich in der Regel wiederholenden Wellenformen empfangen wurden, ein Datensegment zu bilden und zur Ermittlung eines Alternansniveaus das Datensegment zu verarbeiten, dieses ermittelte Alternansniveau bereitzustellen und die Schritte Empfang, Verarbeitung und Bereitstellung zu wiederholen.

**73.** Gerät nach Anspruch 72, zu dem gehören:

ein Einrichtung zur Ermittlung, wann eine ausreichende Anzahl von Teilsequenzen auf der Grundlage eines im Voraus gewählten Kriteriums zur Bewertung des Zustandes des Herzens eines Patienten verarbeitet wurde, und
eine Einrichtung zur Beendung der Schritte Empfang, Verarbeiten, Bereitstellen und
Wiederholen, wenn das im Voraus gewählte Kriterium erfüllt ist.

**74.** Gerät nach einem der Ansprüche 71 bis 73, zu dem gehören:

eine Einrichtung zur digitalen Verarbeitung des empfangenen physiologischen Signals zwecks Bestimmung eines Rauschniveaus und
eine Einrichtung zur Nutzung des ermittelten Rauschniveaus zum Aufzeigen des Zustandes des Herzens eines Patienten, indem aus dem Rauschniveau mit einem vorgegebenen Grad statistischer Sicherheit ermittelt wird, ob das Alternansniveau über einem oberen Schwellwert oder unter einem unteren Schwellwert liegt.

**75.** Gerät nach Anspruch 74, bei dem die Einrichtung zur Nutzung die Aufgabe erfüllt, das Alternansniveau gegenüber einem ersten vorgegebenen Schwellwert und das Verhältnis des Alternansniveaus zum Rauschniveau gegenüber einem zweiten vorgegebenen Schwellwert zu vergleichen.

**76.** Gerät nach Anspruch 75, bei dem die Einrichtung zur Nutzung des Alternans die Aufgabe erfüllt, das Alternansniveau aus einem ersten Abschnitt einer gemessenen, sich in der Regel wiederholenden Wellenform und das Rauschniveau aus einem anderen Abschnitt derselben Wellenform zu bestimmen.

**77.** Gerät nach Anspruch 76, bei dem die Einrichtung zum Empfang des Signals die Aufgabe erfüllt, ein EKG-Signal zu empfangen, und die Verarbeitungseinrichtung die Aufgabe erfüllt, das Rauschniveau aus der PQ-Strecke zu ermitteln.

**78.** Gerät nach Anspruch 74, bei dem die Einrichtung zur Nutzung die Aufgabe erfüllt, das Alternansniveau in einem Abschnitt einer gemessenen, sich in der Regel wiederholenden Wellenform mit einem Maß des Rauschens in einem anderen Frequenzband derselben Wellenform zu vergleichen.

**79.** Gerät nach Anspruch 74, zu dem gehören:

eine Einrichtung zur Bestimmung des Niveaus einer alternierenden Komponente des empfangenen Signals und zur Bestimmung der Standardabweichung der Rauschenergie des Signals und
eine Einrichtung zur Ermittlung, ob das Niveau der alternierenden Komponente um eine oder mehrere Standardabweichungen des Rauschniveaus größer als ein vorgegebener Schwellwert ist, um einen Anhaltspunkt dafür zu liefern, dass bei dem Patienten das Risiko der Herzinstabilität besteht.

**80.** Gerät nach Anspruch 74, bei dem die Bestimmungseinrichtung die Aufgabe erfüllt, das ermittelte Rauschniveau mit einem vorgegebenen Schwellwert zu vergleichen und zu ermitteln, ob das Rauschniveau größer als der vorgegebene Schwellwert ist, um einen Anhaltspunkt dafür zu liefern, dass die Bewertung des Risikos der Herzinstabilität des Patienten unklar ist.

**81.** Gerät nach einem der vorstehenden Ansprüche, zu dem eine Einrichtung zur Intensivierung der Messung des Musters von Zyklus-zu-Zyklus-Morphologieveränderungen durch Verminderung der Auswirkungen der Variabilität im Intervall zwischen benachbarten Wellenformen auf das zumindest ein repräsentatives Signal gehört.

**82.** Gerät nach einem der vorstehenden Ansprüche, zu dem eine Einrichtung zum Ausgleich der störenden Variabilität in der Morphologie der sich in der Regel wiederholenden Wellenformen des empfangenen Signals gehört.

**83.** Gerät nach einem der vorstehenden Ansprüche, zu dem eine Einrichtung zur zusätzlichen Verarbeitung eines oder mehrerer Signale zur Gewinnung eines oder mehrerer der folgenden Diagnosemittel gehört:

eines Mehrfachableitungselektrokardiogramms, eines signalgemittelten Elektrokardiogramms, einer Analyse der RSA-Komplexvariabilität oder einer Feststellung des Zustands eines physiologischen Systems.

**Revendications**

**1.** Appareil de mesure d'un modèle d'alternance de variations de morphologie cycle à cycle dans une séquence de formes d'ondes physiologiques sensiblement répétitives dans au moins un signal mesuré sur un patient, l'appareil comprenant :

un moyen de réception d'un signal physiologique représentatif de l'activité cardiaque d'un patient comprenant un ou plusieurs transducteurs conçus pour la mesure respectivement opératoire d'un ou de plusieurs signaux sur le patient ;
un moyen de traitement numérique du signal physiologique pour déterminer un niveau d'alternance dans le signal, le signal physiologique représentatif de l'activité cardiaque d'un patient est représentatif de l'activité cardiaque du patient présentant un état physiologique modifié résultant d'une contrainte du coeur ;
un dispositif de contrainte, **caractérisé en ce que** ledit dispositif de contrainte comprend un développeur pour l'exercice contrôlable du patient.

**2.** Appareil de mesure d'un modèle d'alternance de variations de morphologie cycle à cycle en séquence de formes d'ondes physiologiques sensiblement répétitives dans au moins un signal mesuré sur un patient, l'appareil comprenant :

un moyen de réception d'un signal physiologique représentatif de l'activité cardiaque d'un patient comprenant un ou plusieurs transducteurs conçus pour la mesure respectivement opératoire d'un ou de plusieurs signaux sur le patient ;
un moyen de traitement numérique du signal physiologique pour déterminer un niveau d'alternance dans le signal, le signal physiologique représentatif de l'activité cardiaque d'un patient est représentatif de l'activité cardiaque du patient présentant un état physiologique modifié résultant d'une contrainte du coeur ;
un dispositif de contrainte, **caractérisé en ce que** ledit dispositif de contrainte comprend un dispositif conçu pour administrer un agent pharmacologique qui modifie de façon contrôlable le rythme cardiaque du patient.

**3.** Appareil de mesure d'un modèle d'alternance de variations de morphologie cycle à cycle en séquence de formes d'ondes physiologiques sensiblement répétitives dans au moins un signal mesuré sur un patient, l'appareil comprenant :

un moyen de réception d'un signal physiologique représentatif de l'activité cardiaque d'un patient comprenant un ou plusieurs transducteurs conçus pour la mesure respectivement opératoire d'un ou de plusieurs signaux sur le patient ;
un moyen de traitement numérique du signal physiologique pour déterminer un niveau d'alternance dans le signal, le signal physiologique représentatif de l'activité cardiaque d'un patient est représentatif de l'activité cardiaque du patient présentant un état physiologique modifié résultant d'une contrainte du coeur ;
un dispositif de contrainte **caractérisé en ce que** ledit dispositif de contrainte comprend une table basculante conçue pour maintenir fermement le patient et en outre conçue pour permettre la modification sélective de l'orientation du corps du patient dont la position doit être autre qu'un décubitus dorsal en vue de la contrainte du coeur du patient.

**4.** Appareil selon l'une quelconque des revendications 1 à 3, dans lequel le processeur est en outre conçu pour déterminer des indices d'ischémie à partir du dit un ou plusieurs signaux mesurés.

**5.** Appareil selon l'une quelconque des revendications 1 à 4, dans lequel l'un au moins des un ou plusieurs transducteurs comprend une électrode à segments multiples ayant de multiples surfaces séparées permettant d'établir le contact électrique avec une région de la surface du patient.

**6.** Appareil selon la revendication 5, comprenant en outre des composants de circuit couplés au(x) transducteur(s) et conçu pour mesurer un signal représentatif du modèle respiratoire du patient.

**7.** Appareil selon la revendication 5, comprenant en outre des composants de circuit couplés à l'électrode à segments multiples et conçu pour mesurer un signal représentatif de l'impédance électrique entre l'électrode à segments multiples et le patient.

**8.** Appareil selon l'une quelconque des revendications 1 à 7, comprenant en outre un enregistreur couplé au(x) transducteur(s) et conçu pour enregistrer les signaux mesurés par ledit ou lesdits transducteurs pour une période supérieure à une heure, et dans lequel le processeur est fonctionnellement conçu pour **caractériser** des parties des signaux mesurés correspondant à une ou plusieurs périodes selon un ou plusieurs critères présélectionnés, et en outre conçu pour obtenir, à partir du ou des signaux enregistrés correspondant à la ou les périodes sélectionnées, une mesure du niveau d'alternance dans ledit au moins un signal représentatif à des fins d'évaluation de l'état cardiaque du patient.

**9.** Appareil selon la revendication 8, comprenant en outre une sortie destinée à fournir un signal représentatif du niveau déterminé d'alternance et un signal de bruit.

**10.** Appareil selon la revendication 9, dans lequel la sortie est en outre adaptée pour fournir un signal représentatif du rythme cardiaque du patient.

**11.** Appareil selon l'une quelconque des revendications 1 à 10, dans lequel le processeur est capable de traiter les signaux pour produire un ou plusieurs des éléments suivants : un électrocardiogramme à dérivations multiples, un électrocardiogramme à moyenne de signal, une indication de l'existence de maladie coronarienne, une analyse de la variabilité ARS, ou une identification du système physiologique.

**12.** Appareil selon l'une quelconque des revendications précédentes, dans lequel le dispositif de contrainte est conçu pour modifier l'état physiologique du patient de telle sorte que le rythme cardiaque du patient dépasse un rythme prédéterminé, et soit de préférence généralement dans la gamme des 90 à 150 battements par minute.

**13.** Appareil selon la revendication dépendante de la revendication 1, dans lequel le développeur comprend un vélo, un tapis roulant ou un translateur oblique.

**14.** Appareil selon la revendication 13, comprenant des moyens de production d'un signal pour le contrôle de l'intensité d'exercice du patient, de préférence pour atteindre un rythme cardiaque souhaité chez le patient.

**15.** Appareil selon l'une quelconque des revendications 13 ou 14, comprenant des moyens de réception des signaux représentatifs du rythme d'exercice du patient.

**16.** Appareil selon l'une quelconque des revendications 13 à 15, comprenant des moyens de production d'un signal pour le contrôle du rythme d'exercice du patient.

**17.** Appareil selon la revendication 16, dans lequel les moyens de production d'un signal sont définis pour sélectionner le rythme d'exercice du patient qui doit être entre environ 28 pour cent et environ 43 pour cent du rythme cardiaque du patient ou entre 57 pour cent environ et 72 pour cent environ du rythme cardiaque du patient.

**18.** Appareil selon la revendication 16 ou 17, dans lequel le moyens de production d'un signal de contrôle est défini pour réduire le bruit qui interfère avec la détermination du niveau d'alternance, de préférence le bruit présentant un composant sensiblement répétitif qui se répète à une fréquence d'environ la moitié du rythme cardiaque du patient ou à un sous-multiple de celle-ci.

**19.** Appareil selon l'une quelconque des revendications 16 à 18, dans lequel les moyens de production d'un signal de contrôle sont définis pour réduire les artefacts de bruits générés par une ou plusieurs sources autres que le coeur du patient.

**20.** Appareil selon la revendication 19, dans lequel le moyen de production d'un signal de contrôle est concç pour réduire les artefacts de bruits générés par suite de l'exercice du patient.

21. Appareil selon la revendication 19, dans lequel le moyen de production d'un signal de contrôle est défini pour réduire les artefacts de bruits générés par la respiration du patient.

22. Appareil selon l'une quelconque des revendications 16 à 21, dans lequel les moyens de production d'un signal de contrôle sont définis pour produire un signal basé sur un protocole d'exercice prédéterminé spécifiant les rythmes d'exercice cibles.

23. Appareil selon l'une quelconque des revendications précédentes, comprenant des moyens de détermination du rythme cardiaque du patient, comprenant de préférence des moyens de production d'un signal de sortie représentatif du rythme cardiaque du patient.

24. Appareil selon l'une quelconque des revendications précédentes, comprenant des moyens de production d'un signal de sortie représentatif du rythme respiratoire du patient.

25. Appareil selon l'une quelconque des revendications précédentes, dans lequel les moyens de traitement numérique sont définis pour traiter le signal afin de réduire le bruit qui interfère avec la détermination du niveau d'alternance.

26. Appareil selon la revendication 25, dans lequel les moyens de traitement numérique du signal pour réduire le bruit sont définis pour déterminer le niveau d'alternance sur des parties du signal physiologique reçu correspondant aux périodes pendant lesquelles le niveau d'exercice chute dans une gamme prédéterminée.

27. Appareil selon la revendication 25, dans lequel les moyens de traitement numérique du signal pour réduire l'effet du bruit sont définis pour sélectionner, en vue de la détermination du niveau d'alternance, des parties du signal reçu basé sur la présence de battements anormaux.

28. Appareil selon la revendication 27, dans lequel les moyens de traitement numérique sont définis pour sélectionner des parties en vue de la détermination de l'alternance sur la base d'une comparaison d'un niveau prédéterminé pondéré.

29. Appareil selon l'une quelconque des revendications précédentes, dans lequel le moyen de traitement numérique est conçu pour traiter des signaux physiologiques issus de périodes de temps précédant la modification de l'état physiologique du patient.

30. Appareil selon l'une quelconque des revendications précédentes, dans lequel le moyen de traitement numérique est conçu pour traiter des signaux physiologiques issus d'une période de temps consécutive à la modification de l'état physiologique du patient.

31. Appareil selon l'une quelconque des revendications précédentes, comprenant :

   des moyens d'obtention d'un second signal provenant du patient ; et
   des moyens de détermination d'une relation entre la variabilité dans les premier et second signaux reçus.

32. Appareil selon la revendication 31, dans lequel le moyen de détermination d'une relation est conçu pour établir un rapport entre les caractéristiques souhaitées du premier et du second signal ou pour établir un rapport entre les composants de bruit du premier et du second signal.

33. Appareil selon la revendication 31, dans lequel le moyen de détermination d'une relation est défini pour combiner le premier et le second signal afin de supprimer le bruit.

34. Appareil selon la revendication 31, dans lequel le moyen d'obtention d'un second signal est défini pour mesurer la variabilité des intervalles de battements intermédiaires.

35. Appareil selon la revendication 31, dans lequel le second signal représente le bruit, de préférence l'artefact d'impédance ou l'artefact de respiration.

36. Appareil selon l'une quelconque des revendications précédentes, comprenant des moyens de production d'un signal pour le contrôle de l'état physiologique du patient de telle sorte que les effets des sources de bruit interférant soient réduites, de préférence comprenant des moyens de contrôle du rythme respiratoire du patient.

**37.** Appareil selon l'une quelconque des revendications précédentes, dans lequel le moyen de traitement numérique est conçu pour traiter le signal afin de réduire l'effet des signaux de bruit ayant une fréquence représentant environ la moitié du rythme cardiaque du patient ou un sous-multiple de celle-ci représentant la moitié du rythme cardiaque du patient.

**38.** Appareil selon la revendication 37, dans lequel le moyen de traitement numérique est conçu de façon adaptable pour retirer les signaux de bruit avant qu'un niveau d'alternance soit déterminé.

**39.** Appareil selon la revendication 37 ou 38, dans lequel lé moyen de traitement numérique du signal pour réduire l'effet des signaux de bruit est conçu pour baser la détermination d'un niveau d'alternance sur des parties du signal reçu correspondant aux périodes pendant lesquelles le rythme cardiaque du patient est supérieur à un niveau prédéterminé.

**40.** Appareil selon la revendication 25 ou 37, ou l'une quelconque des revendications qui en dépendent, dans lequel le moyen de traitement numérique du signal pour réduire l'effet des signaux de bruit est conçu pour baser la détermination d'un niveau d'alternance sur des parties du signal reçu correspondant aux périodes pendant lesquelles le rythme respiratoire du patient est différent du rythme cardiaque du patient et est différent des sous-multiples du rythme cardiaque du patient.

**41.** Appareil selon l'une quelconque des revendications 37 à 40, dans lequel le moyen de traitement numérique est en outre conçu pour affecter des valeurs significatives à des parties du signal reçu sur la base de la présence de battements anormaux, et pour baser la détermination d'un niveau d'alternance sur les valeurs significatives affectées.

**42.** Appareil selon l'une quelconque des revendications précédentes, comprenant des moyens de détermination du rythme cardiaque du patient, et de production simultanée d'un signal représentatif du niveau déterminé d'alternance et de production d'un signal représentatif du rythme cardiaque déterminé.

**43.** Appareil selon l'une quelconque des revendications précédentes, dans lequel le moyen de traitement numérique est défini pour compenser la variabilité d'interférence dans la morphologie des formes d'ondes sensiblement répétitives dans le signal reçu.

**44.** Appareil selon la revendication 43, dans lequel le moyen de traitement numérique est conçu pour compenser la variabilité d'interférence générée par l'activité respiratoire du patient.

**45.** Appareil selon la revendication 43, dans lequel le moyen de traitement numérique est conçu pour compenser la variabilité des intervalles d'intercycles.

**46.** Appareil selon la revendication 45, dans lequel le moyen de compensation de la variabilité des intervalles d'intercycles est conçu pour déterminer une relation entre la variation des intervalles d'intercycles et les changements de morphologie des formes d'ondes.

**47.** Appareil selon la revendication 46, dans lequel le moyen est un filtre établissant un rapport entre les mesures d'une forme d'onde donnée et la séquence des intervalles précédents d'intercycles.

**48.** Appareil selon la revendication 37, dans lequel le moyen de traitement numérique est conçu pour établir un rapport entre la variabilité dans un signal de bruit et la variabilité dans la morphologie des formes d'ondes sensiblement répétitives.

**49.** Appareil selon la revendication 48, dans lequel le signal de bruit représente une mesure de l'activité respiratoire du patient.

**50.** Appareil selon la revendication 37, comprenant :

des moyens de réception d'un second signal physiologique représentatif de l'activité cardiaque du patient ;
des moyens de détermination d'une relation entre les caractéristiques souhaitées du premier et du second signal reçus ;
des moyens de détermination d'une relation entre les composants de bruit du premier et du second signal reçus; et

des moyens de combinaison du premier et du second signal pour minimiser le bruit dans la détermination d'un niveau d'alternance.

51. Appareil selon les revendications 31 ou 50, ou l'une quelconque des revendications qui en dépendent, dans lequel les moyens de réception du premier et du second signal sont des dérivations d'électrocardiogramme (ECG), le premier et le second signal étant combinés de telle sorte à préserver l'orientation spatiale des dérivations d'électrocardiogramme.

52. Appareil selon les revendications 31 ou 50, ou l'une quelconque des revendications qui en dépendent, comprenant en outre des moyens de traitement des premier et second signaux pour déterminer une métrique de distorsion, et dans lequel le premier et le second signal reçus sont combinés de telle sorte que la métrique de distorsion se trouve en dessous d'un niveau prescrit.

53. Appareil selon l'une quelconque des revendications précédentes, dans lequel les moyens de traitement numérique sont définis pour baser la détermination d'un niveau d'alternance sur des parties du signal reçu correspondant aux périodes pendant lesquelles un niveau de bruit est inférieur à un niveau présélectionné.

54. Appareil selon l'une quelconque des revendications précédentes, dans lequel les moyens de traitement numérique sont conçus pour baser la détermination d'un niveau d'alternance sur des parties du signal reçu correspondant aux périodes pendant lesquelles le niveau déterminé d'alternance est supérieur à un niveau présélectionné.

55. Appareil selon l'une quelconque des revendications précédentes, dans lequel le moyen de traitement numérique est défini pour **caractériser** une ou plusieurs parties du signal reçu sur la base d'un ou de plusieurs critères présélectionnés et pour utiliser la caractérisation des parties en vue de la détermination d'un niveau d'alternance dans le signal.

56. Appareil selon la revendication 55, dans lequel le moyen de traitement numérique est défini pour **caractériser** les parties en sélectionnant des parties du signal reçu à exclure de la détermination de l'étape d'alternance.

57. Appareil selon la revendication 56, dans lequel le moyen de traitement numérique est conçu pour exclure des parties du signal reçu en déterminant un niveau d'alternance sur des parties du signal reçu correspondant aux périodes pendant lesquelles le rythme cardiaque du patient est au-dessus d'un niveau prédéterminé.

58. Appareil selon la revendication 56, dans lequel le moyen de traitement numérique est conçu pour exclure des parties du signal reçu en déterminant un niveau d'alternance sur des parties du signal reçu correspondant aux périodes pendant lesquelles le rythme respiratoire du patient est différent du rythme cardiaque du patient et est différent des sous-multiples du rythme cardiaque du patient.

59. Appareil selon la revendication 55, dans lequel le moyen de traitement numérique est défini pour **caractériser** les parties par l'affectation de valeurs significatives aux parties du signal reçu sur la base de la présence de battements anormaux, et est en outre défini pour baser la détermination d'un niveau d'alternance sur les valeurs significatives affectées.

60. Appareil selon la revendication 55, comprenant des moyens d'estimation indépendante du niveau d'alternance sur la base de séquences de données entre les formes d'ondes anormales ; et des moyens de combinaison des estimations indépendantes pour déterminer la mesure du niveau d'alternance.

61. Appareil selon la revendication 55, dans lequel le moyen de traitement numérique est défini pour déterminer un niveau d'alternance sur la base d'une hypothèse prédéterminée selon laquelle une forme d'onde anormale particulière est suivie d'une forme d'onde normale d'une phase donnée.

62. Appareil selon la revendication 61, dans lequel le moyen de traitement numérique est défini pour exclure les formes d'ondes anormales lors de la détermination du niveau d'alternance.

63. Appareil selon la revendication 61, dans lequel des moyens de traitement numérique sont définis pour remplacer les formes d'ondes anormales par des formes d'ondes moyennes dans la détermination du niveau d'alternance.

**64.** Appareil selon la revendication 61, dans lequel le ou les signaux sont mesurés par des moyens de mesure du rythme respiratoire du patient, de préférence par la pléthysmographie d'impédance, et dans lequel les moyens de traitement numérique sont définis pour ne plus prendre en compte des parties desdits signaux mesurés correspondant aux périodes pendant lesquelles le rythme respiratoire du patient est un sous-multiple du rythme cardiaque du patient.

**65.** Appareil selon la revendication 61, dans lequel le moyen de mesure du ou des signaux comprend un ou plusieurs transducteurs placés sur le patient à des fins de mesure des signaux d'ECG, et dans lequel le rythme respiratoire du patient est déterminé par l'analyse des signaux d'ECG mesurés.

**66.** Appareil selon la revendication 55, comprenant des moyens d'analyse desdits signaux d'ECG par l'analyse de l'amplitude ou de l'angle vectocardiographique des caractéristiques des signaux d'ECG mesurés.

**67.** Appareil selon la revendication 66, dans lequel le critère présélectionné est le rythme cardiaque du patient.

**68.** Appareil selon la revendication 67, comprenant des moyens permettant de ne plus prendre en compte les signaux mesurés lorsqu'ils correspondent aux périodes pendant lesquelles un signal de bruit interférant varie sur un multiple ou sous-multiple du rythme cardiaque du patient.

**69.** Appareil selon la revendication 55, dans lequel le critère présélectionné est le rythme respiratoire du patient.

**70.** Appareil selon la revendication 55, dans lequel le critère présélectionné est la présence de battements anormaux dans un ou plusieurs des signaux mesurés.

**71.** Appareil selon l'une quelconque des revendications précédentes, comprenant des moyens de fourniture d'une sortie représentative du niveau d'alternance simultanément à l'étape de traitement du signal physiologique.

**72.** Appareil selon la revendication 71, dans lequel le moyen de traitement numérique est conçu, après la réception d'un nombre prédéterminé de formes d'ondes sensiblement répétitives, pour former un segment de données, puis pour traiter le segment de données pour déterminer un niveau d'alternance, pour fournir le niveau déterminé d'alternance, et pour répéter les étapes répétitives de réception, de traitement et de fourniture.

**73.** Appareil selon la revendication 72, comprenant :

des moyens permettant de déterminer lorsqu'un nombre suffisant de séquences partielles a été traité sur la base d'un critère présélectionné pour l'évaluation de l'état cardiaque du patient; et
des moyens d'achèvement des étapes de réception, de traitement, de fourniture et de répétition une fois ledit critère présélectionné satisfait.

**74.** Appareil selon l'une quelconque des revendications 71 à 73, comprenant :

des moyens de traitement numérique du signal physiologique reçu pour déterminer un niveau de bruit ; et
des moyens d'utilisation du niveau déterminé de bruit pour produire une indication de l'état cardiaque du patient en déterminant à l'aide d'un niveau prédéterminé de certitude statistique à partir dudit niveau de bruit, si le niveau d'alternance est au-dessus d'un seuil supérieur ou en dessous d'un seuil inférieur.

**75.** Appareil selon la revendication 74, dans lequel le moyen d'utilisation est conçu pour comparer le niveau d'alternance par rapport à un premier seuil prédéterminé et pour comparer le rapport du niveau d'alternance au niveau de bruit avec un second seuil prédéterminé.

**76.** Appareil selon la revendication 75, dans lequel le moyen d'utilisation de l'alternance est défini pour déterminer le niveau d'alternance à partir d'une première partie d'une forme d'onde mesurée sensiblement répétitive, et pour déterminer le niveau de bruit à partir d'une partie différente de la même forme d'onde.

**77.** Appareil selon la revendication 76, dans lequel le moyen de réception du signal est défini pour recevoir un signal d'ECG et les moyens de traitement sont définis pour déterminer le niveau de bruit à partir du segment PQ.

**78.** Appareil selon la revendication 74, dans lequel le moyen d'utilisation est défini pour comparer le niveau d'alternance dans une partie d'une forme d'onde mesurée sensiblement répétitive à une mesure de bruit dans une bande de fréquence différente de la même forme d'onde.

**79.** Appareil selon la revendication 74, comprenant :

des moyens de détermination du niveau dans une composante alternative du signal reçu et de détermination de la déviation standard de l'énergie de bruit dans le signal ; et
des moyens permettant de déterminer si le niveau de la composante alternative est supérieur à un seuil prédéterminé par une ou plusieurs déviations standard du niveau de bruit pour donner une indication sur le risque d'instabilité cardiaque du patient.

**80.** Appareil selon la revendication 74, dans lequel le moyen de détermination est défini pour comparer le niveau de bruit déterminé par rapport à un seuil prédéterminé, et pour déterminer si ledit niveau de bruit est supérieur au seuil prédéterminé pour donner une indication sur le fait que l'évaluation du risque d'instabilité cardiaque du patient est indéterminée.

**81.** Appareil selon l'une quelconque des revendications précédentes, comprenant des moyens d'amélioration de la mesure du modèle des variations de morphologie cycle à cycle en réduisant l'effet de la variabilité dans l'intervalle compris entre les formes d'ondes adjacentes dans ledit au moins un signal représentatif.

**82.** Appareil selon l'une quelconque des revendications précédentes, comprenant des moyens de compensation de la variabilité d'interférence dans la morphologie des formes d'ondes sensiblement répétitives dans le signal reçu.

**83.** Appareil selon l'une quelconque des revendications précédentes, comprenant des moyens de traitement supplémentaire du ou des signaux pour produire un ou plusieurs des éléments suivants : un électrocardiogramme à dérivations multiples, un électrocardiogramme à moyenne de signal, une analyse de la variabilité complexe ARS, ou l'identification système physiologique.

Electrocardiogram:

A　B　A　B　A・・・

~10

~20

FIG.1

Power
Spectrum

~11

Std Deviation
of Reference
Noise Level

11

17

Alternans
Power

16~ Reference
Noise Level

12

0.5

Frequency
(cycles/beat)

Reference
Noise
Baud

FIG.1A

EP 0 746 229 B1

FIG. 2

DEVICE FOR
CUING
PATIENT — 42

44

APPARATUS
FOR ALTERING
PHYSIOLOGIC
CONDITION
OF PATIENT — 26

46

PATIENT — 24

40

MONITOR

36

ONE OR MORE
TRANDUCERS — 28

AMPLIFIER

30

PROCESSOR

38

32

DISPLAY

34

FIG. 3

FIG. 4

FIG. 5

Microvolts

20

15

10

5

0

0    0.1    0.2    0.3    0.4    0.5
Cycles/Beat

FIG. 6

Microvolts

20

15

10

5

0

0    0.1    0.2    0.3    0.4    0.5
Cycles/Beat

FIG. 7

**Alternans Spectrum: p(reset) = 0.00**

Power ($\mu V^2$)

300

0 0.1 0.2 0.3 0.4 0.5
Frequency (Cycles/Beat)

## FIG. 8

**Alternans Spectrum: p(reset) = 0.05**

Power ($\mu V^2$)

302

0 0.1 0.2 0.3 0.4 0.5
Frequency (Cycles/Beat)

## FIG. 8A

FIG. 9

FIG. 10

EP 0 746 229 B1

## Without RR Correction

## FIG. 11

## With RR Correction

## FIG.12

FIG. 13

FIG.14

FIG. 14A

ACQUIRE MULTIPLICITY
OF INPUT SIGNALS — 201

DEFINE OUTPUT SIGNAL — 202

203 —
COMPUTE INTER-SIGNAL
RELATIONSHIP OF THE
DESIRED PART OF THE
OUTPUT SIGNAL

205
COMPUTE INTER-SIGNAL
RELATIONSHIP OF THE
NOISE

204 —
COMPUTE METRIC OF
THE DISTORTION OF THE
DESIRED OUTPUT SIGNAL

206
COMPUTE METRIC OF
THE NOISE IN THE
OUTPUT SIGNAL

DETERMINE OPTIMAL
COMBINATION OF INPUT
SIGNALS THAT
MINIMIZES THE METRICS — 207

COMPUTE REDUCED
NOISE OUTPUT SIGNAL — 208

FIG. 15

FIG. 16 — ECG

FIG. 16A — Respiration

FIG. 16B — Fundamental

FIG. 16C — First Harmonic

FIG. 16D — ECG + Harmonic Noise

ECG & Noise

## FIG. 17

Respiration

## FIG. 17A

Impedance

## FIG. 17B

Reduced Noise ECG

## FIG. 17C

ACQUIRE 32 SIGNALS: 24 ECG, 7 IMPEDANCE, 1 RESPIRATION — 501

DEFINE FRANK XYZ AND V4 MATRICES $F_{XYZ}$ AND $F_{V4}$ — 502

503 — COMPUTE THE CORRELATION MATRIX $R_{SS}$ FROM THE QRST COMPONENT OT THE MEAN BEAT

505 — COMPUTE THE CORRELATION MATRIX $R_{\eta\eta}$ OF THE BEAT-TO-BEAT T WAVE VARIABILITY RELATED TO NOISE

504 — COMPUTE THE METRIC $\mathcal{E}_D$ THE DISTORTION OF THE DESIRED OUTPUT SIGNAL

506 — COMPUTE THE METRIC $\mathcal{E}_\eta$ THAT MEASURES THE NOISE IN THE OUTPUT SIGNALS

COMPUTE THE OPTIMAL FRANK XYZ AND V4 MATRICES AS $T_{XYZ}$ AND $T_{V4}$ — 507

COMPUTE LOW NOISE XYZ AND V4 SIGNALS — 508

FIG. 18

Basepad & Silver

2.250

Ø 1.180

1.125

2.375

Ø 0.640

Ø 1.600

FIG. 19

Foam

Ø 1.120

Ø 0.700

Ø 1.660

FIG. 19A

FIG. 20

FIG. 21

EP 0 746 229 B1

FIG. 22

| Electrode Name | Electrode Location | Type | Available Signals |
|---|---|---|---|
| RL | R. ILLIAC CREST | STANDARD | (DRIVEN GROUND) |
| RA | RIGHT SHOULDER | STANDARD | (REFERENCE) |
| LA | L. SHOULDER | STANDARD | LA |
| LL(F) | L. ILLIAC CREST | MULTIPLE | LLa, LLb, LLc, LLi |
| V1 | V1 | STANDARD | V1 |
| V2 | V2 | STANDARD | V2 |
| V3 | V3 | STANDARD | V3 |
| V4(C) | V4 | MULTIPLE | V4, V4a, V4i |
| V5 | V5 | STANDARD | V5 |
| V6(A) | V6 | MULTIPLE | V6, V6a, V6b, V6i |
| I | R. V6 POSITION | MULTIPLE | I, Ia, Ib, Ii |
| H | BELOW NECK | MULTIPLE | H, Ha, Hi |
| E | BETWEEN A & I | MULTIPLE | E, Ea, Ei |
| M | BACK | MULTIPLE | M, Ma, Mb, Mi |

FIG. 22A

- Anti-alias filter
- Sample at 1KHz
- Align beats
- Select epoch of N beats

*b(n)*

- Filter & decimate to 250 Hz
- Create beat matrices *B(n)*
- Replace bad beats

*B(n)*

## FIG. 23

*b(n)* =

| Row | Signal | Row | Signal |
|-----|--------|-----|--------|
| 1 | LA | 17 | V6i |
| 2 | LL | 18 | I |
| 3 | LLa | 19 | Ia |
| 4 | LLb | 20 | Ib |
| 5 | LLc | 21 | Ii |
| 6 | LLi | 22 | H |
| 7 | V1 | 23 | Ha |
| 8 | V2 | 24 | Hi |
| 9 | V3 | 25 | E |
| 10 | V4 | 26 | Ea |
| 11 | V4a | 27 | Ei |
| 12 | V4i | 28 | M |
| 13 | V5 | 29 | Ma |
| 14 | V6 | 30 | Mb |
| 15 | V6a | 31 | Mi |
| 16 | V6b | 32 | Resp |

## FIG. 24

FIG. 25

FIG. 26

$$F_{xyz} = \begin{bmatrix} F_x \\ F_y \\ F_z \end{bmatrix}$$

$$D_{xyz}(n) = F_{xyz}B(n)$$

$$D_{V4}(n) = F_{V4}B(n)$$

FIG. 27

| Col | Signal | $F_X$ | $F_X$ | $F_X$ | $F_X$ |
|---|---|---|---|---|---|
| 1 | LA | | | | |
| 2 | LL | | 0.655 | | |
| 3 | LLa | | | | |
| 4 | LLb | | | | |
| 5 | LLc | | | | |
| 6 | LLi | | | | |
| 7 | V1 | | | | |
| 8 | V2 | | | | |
| 9 | V3 | | | | |
| 10 | V4 | 0.171 | | −0.231 | 1.000 |
| 11 | V4a | | | | |
| 12 | V4i | | | | |
| 13 | V5 | | | | |
| 14 | V6 | 0.610 | | 0.133 | |
| 15 | V6a | | | | |
| 16 | V6b | | | | |
| 17 | V6i | | | | |
| 18 | I | −0.781 | | −0.264 | |
| 19 | Ia | | | | |
| 20 | Ib | | | | |
| 21 | Ii | | | | |
| 22 | H | | −1.00 | | |
| 23 | Ha | | | | |
| 24 | Hi | | | | |
| 25 | E | | | −0.734 | |
| 26 | Ea | | | | |
| 27 | Ei | | | | |
| 28 | M | | 0.345 | 0.736 | |
| 29 | Ma | | | | |
| 30 | Mb | | | | |
| 31 | Mi | | | | |
| 32 | Resp | | | | |

FIG. 28

$v(I)$

$I$

$v(N)$

$N$

FIG. 28A

$v(n)$

$I$ $N$ $n$

FIG. 28B

$[V(f)]^2$

0.0 0.25 0.5 $f$

$$M = \begin{bmatrix} X_M, Y_M, Z_M \end{bmatrix} \quad M(t) = X_M V_M(t) + y_M v_Y(t) + Z_M V_Z(t)$$

Left

$V_Z(t)$

Right

$V_X(t)$

$V_Y(t)$

Front

**FIG. 29**

M

I

V6

V5

V4

V1

V2

V3

**FIG. 29A**

FIG. 30

## Flow Chart for the Real-Time Computation of Alternans

| | |
|---|---|
| **Step 1:** | DIGITIZE ELECTROCARDIOGRAPHIC SIGNALS CONTINUOUSLY IN TIME |

↓

| | |
|---|---|
| **Step 2:** | IDENTIFY EVERY SIXTEENTH BEAT CONTINUOUSLY IN TIME |

↓

| | |
|---|---|
| **Step 3:** | IDENTIFY SIXTY-FOUR BEAT SEQUENCES STARTING WITH EACH OF THE BEATS IDENTIFIED IN STEP 2, CONTINUOUSLY IN TIME |

↓

| | |
|---|---|
| **Step 4:** | UPON IDENTIFICATION OF EACH SEQUENCE IN STEP 3, COMPUTE MEASURES OF ALTERNANS IN THAT SEQUENCE |

↓

| | |
|---|---|
| **Step 5:** | UPON COMPUTING OF MEASURES OF ALTERNANS FOR EACH SEQUENCE IN STEP 4, DISPLAY THESE MEASURES IN GRAPHICAL FORM |

# FIG. 31

FIG. 32

p(Alt Energy-Ref Noise > 2uV RMS and K > 3)

Probability

RMS Noise per Frequency Bin (μV)

FIG. 33

NEW DATA
SEGMENT

COMPUTE SEGMENT
MEASURES

HEAR HR, # OF PREMATURE BEATS
BASELINE HARMONIC NOISE, ALTERNANS SPECTRUM NOISE
NUMBER OF BEATS

1201

LOW HR
CRITERIA
MET?

1202

Y

FIRST TIME?

1203

Y

COMPUTE
ALTERNANS

72

N

N

NOTIFY
OPERATOR

1204

TARGET
HR CRITERIA
MET?

1205

Y

FIRST TIME?

1206

Y

1208

SHORTEN
SEGMENT

N

N

SHOULD
SEGMENT BE
SHORTENED?

1207

Y

N

(SHEET 1 OF 2)
FIG.34

1209

COMPUTE GOODNESS
SCORE AND UPDATE
LIST OF SEGMENTS

1210
TEST
ENDED OR
DURATION
EXCEEDED?

Y

1212
SELECT BEST
SEGMENT

72
COMPUTE
ALTERNANS

1213
DONE

N

1211
WAIT FOR
MORE DATA

(SHEET 2 OF 2)
FIG. 34

EP 0 746 229 B1

EP 0 746 229 B1

FIG. 35

FIG. 35A

FIG. 35B

FIG. 35C

FIG. 35D

(SHEET 1 OF 2)

# FIG. 36

DIGITIZE ELECTROCARDIOGRAPHIC SIGNALS

IDENTIFY BEATS

GENERATE LIST OF OVERLAPPING 64 BEAT SEQUENCES; ONE SEQUENCE STARTING EVERY SIXTEENTH BEAT

RETAIN FROM THIS LIST THOSE SEQUENCES IN WHICH THE HEART RATE IS BETWEEN 0.95 AND 1.2 TIMES THE TARGET HEART RATE AND HAVE FEWER THAN SIX PREMATURE BEATS

GENERATE A SCORE FOR EACH SEGMENT BY MULTIPLYING EACH ITEM BY THE CORRESPONDING WEIGHTING FACTOR AND SUMMING THE RESULT

| ITEM | WEIGHTING FACTOR |
|---|---|
| NUMBER OF PREMATURE BEATS | 1 |
| NUMBER OF PREMATURE BEATS IN MIDDLE 32 BEATS | 0.5 |
| T-WAVE STANDARD DEVIATION (IN MICROVOLTS) | 0.1 |
| PQ SEGMENT STANDARD DEVIATION (IN MICROVOLTS) | 0.1 |
| MAXIMUM OF (MEANHEART RATE MINUS 1.15 TIMES TARGET HEART RATE, AND TARGET HEART RATE MINUS MEAN HEART RATE MEASURED IN BEATS PER MINUTE) | 0.1 |
| SQUARE ROOT OF POWER SPECTRUM AT 0.25 CYCLES/BEAT | 0.1 |
| SQUARE ROOT OF POWER SPECTRUM AT 0.0.167 CYCLES/BEAT | 0.1 |

FIND TWO SEGMENTS WHICH JOINTLY PRODUCE THE LOWEST SCORE AND DO NOT OVERLAP

## FIG. 37

(SHEET 2 OF 2)

# FIG. 36

SELECTED
EPOCHS → | REPLACE ECTOPICS |73 → | CORRECT FOR RR INTERVALS |74 → | CORRECT FOR RESPIRATION |75 → | OPTIMIZE LEADS |76 → | COMPUTE FFT'S |77

| AVERAGE FFT'S |78 → | CALCULATE ALTERNANS POWER |79 → | CALCULATE NOISE METRICS |80 → | CALCULATE STATISTICAL SIGNIFICANCE |81 → | ANALYZE MULITPLE LEAD RESULTS |82 → ALTERNANS RESULTS

**FIG. 38**

FIG. 39

CASSETTE TAPE
75

PLAYBACK
ECG AND
DIGITIZE
76

DETECT
QRS
77

CORRELATE
AND
ALIGN
79

COMPUTE
HEART
RATE
78

COMPUTE
RESPIRATION
RATE
87

COMPUTE
RR
INTERVAL
80

UPDATE
AVERAGE
BEATS
83

ELECTRODES
ON
SUBJECT
73

AMPLIFY
FILTER AND
RECORD
74

CASSETTE TAPE
75

(SHEET 1 OF 2)
FIG. 40

EP 0 746 229 B1

(SHEET 2 OF 2)
# FIG. 40

EP 0 746 229 B1

|  | SENSITIVITY | SPECIFICITY | +PV | −PV | RR | p |
|---|---|---|---|---|---|---|
| REST | 50% | 91% | 80% | 71% | 2.8 | 0.14 |
| EX | 80% | 91% | 89% | 84% | 5.3 | 0.005 |
| REST/EX | 100% | 91% | 91% | 100% | >10 | <0.005 |

+PV: POSITIVE PREDICTIVE VALUE;    −PV: NEGATIVE PREDICTIVE VALUE;
RR: RELATIVE RISK;    p: SIGNIFICANCE LEVEL.

# FIG. 41

EP 0 746 229 B1